(19) Europäisches Patentamt
European Patent Office
Office européen des brevets

(11) EP 2 069 385 B1

(12) # FASCICULE DE BREVET EUROPEEN

(45) Date de publication et mention de la délivrance du brevet:
**05.01.2011 Bulletin 2011/01**

(21) Numéro de dépôt: **07847151.3**

(22) Date de dépôt: **13.11.2007**

(51) Int Cl.:
***C07K 1/107*** (2006.01) ***G01N 33/68*** (2006.01)
***C07K 17/06*** (2006.01)

(86) Numéro de dépôt international:
**PCT/EP2007/062277**

(87) Numéro de publication internationale:
**WO 2008/058963 (22.05.2008 Gazette 2008/21)**

(54) **IMMOBILISATION DE PROTEINES MEMBRANAIRES SUR UN SUPPORT PAR L'INTERMEDIAIRE D'UNE MOLECULE AMPHIPHILE**

MEMBRANPROTEINIMMOBILISIERUNG AUF EINEM TRÄGER MIT EINEM AMPHIPHILEN MOLEKÜL

IMMOBILIZATION OF MEMBRANE PROTEINS ONTO SUPPORTS VIA AN AMPHIPHILE

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **13.11.2006 FR 0609882**

(43) Date de publication de la demande:
**17.06.2009 Bulletin 2009/25**

(73) Titulaires:
- **Centre National de la Recherche Scientifique (CNRS)**
  **75016 Paris (FR)**
- **Université Paris 7 - Denis Diderot**
  **75005 Paris (FR)**

(72) Inventeurs:
- **POPOT, Jean-Luc**
  **75013 Paris (FR)**
- **CHARVOLIN, Delphine**
  **92200 Neuilly sur Seine (FR)**
- **GIUSTI, Fabrice**
  **92330 Sceaux (FR)**

(74) Mandataire: **Warcoin, Jacques et al**
**Cabinet Régimbeau**
**20, rue de Chazelles**
**75847 Paris Cedex 17 (FR)**

(56) Documents cités:
**WO-A-98/27434**

- **DATABASE CA [Online] CHEMICAL ABSTRACTS SERVICE, COLUMBUS, OHIO, US; 1991, PIDGEON, CHARLES ET AL: "Immobilized artificial membrane chromatography: rapid purification of functional membrane proteins" XP002438251 extrait de STN Database accession no. 114:224839 & ANALYTICAL BIOCHEMISTRY , 194(1), 163-73 CODEN: ANBCA2; ISSN: 0003-2697, 1991,**
- **C L POCANSCHI ET AL.: "Amphipatic polymers: tools to fold integral membrane proteins to their active forms" BIOCHEMISTRY., vol. 45, no. 47, avril 2006 (2006-04), pages 13954-13961, XP002438248 US AMERICAN CHEMICAL SOCIETY. EASTON, PA.**
- **Y GOHON ET AL.: "Well-definď nanoparticles formed by hydrophobic assembly of a short and polydisperse random terpolymer, Amphipol A8-35" LANGMUIR., vol. 22, no. 3, juin 2006 (2006-06), pages 1281-1290, XP002470386 US ACS, WASHINGTON, DC.**
- **J.-L. POPOT ET AL.: "Amphipols: polymeric surfactants for membrane biology research" CMLS CELLULAR AND MOLECULAR LIFE SCIENCES., vol. 60, 2003, pages 1559-1574, XP002470387 DE BIRKHAUSER VERLAG, HEIDELBERG.**
- **H LIU ET AL: "Single-step purification of rat liver aldolase using immobilized artificial membrane chromatography" JOURNAL OF CHROMATOGRAPHY, BIOMEDICAL APPLICATIONS., vol. 703, 1997, pages 53-62, XP004100020 NL ELSEVIER SCIENCE PUBLISHERS B.V. AMSTERDAM. 1431977**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 069 385 B1

- **S ONG ET AL.: "Immobilized artificial-membrane chromatography ...." JOURNAL OF CHROMATOGRAPHY, CHROMATOGRAPHIC REVIEWS., vol. 728, 1996, pages 113-128, XP004039444 NL ELSEVIER SCIENCE PUBLISHERS B.V. AMSTERDAM.**
- **C E SCHAFMEISTER ET AL.: "SCIENCE" , vol. 262, 29 October 1993 (1993-10-29), pages 734-738, AMERICAN ASSOCIATION FOR THE ADVANCEMENT OF SCIENCE ISSN: 0036-8075**
- **MC GREGOR ET AL.: "BIO/TECHNOLOGY." , vol. 212, no. 2, February 2003 (2003-02), pages 171-176, NATURE PUBLISHING CO. NEW YORK. ISSN: 0733-222X**
- **E DIAMANDIS & T K CHRISTOPOULOS: CLINICAL CHEMISTRY., vol. 37, no. 5, 1991, pages 625-636, AMERICAN ASSOCIATION FOR CLINICAL CHEMISTRY ISSN: 0009-9147**
- **C E SCHAFMEISTER ET AL.: "SCIENCE" , vol. 262, 29 October 1993 (1993-10-29), pages 734-738, AMERICAN ASSOCIATION FOR THE ADVANCEMENT OF SCIENCE ISSN: 0036-8075**
- **MC GREGOR ET AL.: "BIO/TECHNOLOGY." , vol. 212, no. 2, February 2003 (2003-02), pages 171-176, NATURE PUBLISHING CO. NEW YORK. ISSN: 0733-222X**

## Description

### DOMAINE DE L'INVENTION

L'invention se situe dans le domaine de l'immobilisation de protéines membranaires sur un support. Elle concerne un produit comprenant un support et au moins une protéine membranaire fixée à sa surface, **caractérisé en ce que** ladite protéine membranaire est fixée sur ledit support par l'intermédiaire d'une molécule amphiphile avec laquelle ladite protéine membranaire est complexée la molécule amphiphile étant choisie parmi un polymère vinylique amphiphile ou un polypeptide amphiphile, et ladite au moins une protéine membranaire étant avantageusement choisie parmi un antigène, un anticorps, une enzyme, un récepteur cellulaire, un canal ionique, ou une protéine membranaire d'origine virale, bactérienne, ou eucaryote. Elle concerne également un procédé de préparation d'un tel produit, ainsi que différentes applications dans les domaines du diagnostic, du drug design et des biotechnologies. Elle concerne enfin un kit, ainsi qu'une molécule amphiphile fonctionnalisée, pour préparer un produit selon l'invention comprenant un support et une molécule amphiphile, où la molécule amphiphile et le support interagissent par une liaison hydrophobe, une liaison ionique, une liaison spécifique ou une liaison covalente.

### ART ANTERIEUR

Les protéines membranaires intégrales représentent environ 30% des séquences codantes des génomes eucaryotes. L'importance de leurs fonctions, et notamment celles des protéines qui sont insérées dans la membrane plasmique des cellules et exposées au milieu extérieur, en fait des cibles privilégiées dans les domaines du biomédical ou de la pharmacologie. On estime en effet qu'elles sont la cible d'au moins 60% des agents thérapeutiques actuellement sur le marché. Les protéines membranaires constituent notamment des sites de liaisons, des points d'attaches ou des cibles privilégiées pour de multiples interactions. Celles-ci vont de la reconnaissance de petits ligands, tels que des neuromédiateurs ou des hormones, à l'association de cellules entre elles pour constituer des tissus. Les protéines membranaires sont aussi le plus souvent la première cible des virus, bactéries pathogènes ou parasites, ou encore des anticorps lors de la défense immunitaire ou des maladies auto-immunes. Elles peuvent également être impliquées dans les associations membranes/ADN ou membrane/cytosquelette, dans la régulation ou dérégulation de la division cellulaire (cancers), ou encore sont reconnues par des effecteurs macromoléculaires comme les protéines G ou les kinésines.

Du fait de leur importance dans les domaines du biomédical et de la pharmacologie, il est essentiel de disposer d'outils d'étude des protéines membranaires et de leurs ligands. Notamment, il est important d'être capable de détecter la liaison d'un ligand à une protéine membranaire d'intérêt. En effet, un procédé de détection de la liaison d'un ligand a plusieurs applications très importantes :

- dans le cas où la protéine membranaire est issue de la membrane d'un agent pathogène, un tel procédé peut permettre de détecter dans un échantillon biologique obtenu d'un sujet la présence ou l'absence d'anticorps dirigés contre cette protéine membranaire, et donc la présence ou l'absence d'une exposition du sujet à l'agent pathogène ;

- dans le cas d'un récepteur membranaire humain ou animal démontré comme impliqué dans la pathogenèse d'une maladie et constituant donc une cible thérapeutique pour le traitement de cette maladie, un tel procédé peut permettre de réaliser un criblage de banques de composés de façon à identifier des composés agonistes ou antagonistes de ce récepteur membranaire.

Pour mettre en oeuvre un procédé de détection de liaison d'un ligand à une protéine membranaire, il est très utile de disposer de cette protéine membranaire sous une forme immobilisée sur un support. On sait depuis longtemps réaliser la fixation ou immobilisation de protéines sur un support. Toutefois, le problème est plus complexe s'agissant de protéines membranaires. En effet, les protéines membranaires possèdent nécessairement une région fortement hydrophobe leur permettant d'interagir avec la membrane, ce qui rend leur manipulation difficile, en particulier du fait qu'il est généralement nécessaire d'utiliser des doses importantes de détergents pour les isoler de la membrane et pour les solubiliser. Par rapport aux protéines cytosoliques, leur manipulation et donc leur immobilisation sur un support est rendue beaucoup plus ardue du fait de leur hydrophobie.

Différentes techniques d'immobilisation ont été mises au point pour déposer des protéines membranaires à la surface d'un support. Par exemple, on peut, par modification génétique ou chimique de la protéine, insérer un groupement fonctionnel à l'une des extrémités de la protéine pour favoriser l'adhésion de la protéine à la surface d'un support fonctionnalisé (par exemple, en introduisant une extrémité His-Tag sur la protéine, elle interagira avec un support greffé de groupements NTA ligandant des ions $Ni^{2+}$ ou $Co^{2+}$) (1,2).

Toutefois, une telle technique repose sur la possibilité de manipuler génétiquement ou chimiquement la protéine d'intérêt, et est donc difficile ou impossible à mettre en oeuvre pour une protéine membranaire sur laquelle on ne dispose

que de peu d'information. De plus, elle peut être lourde et fastidieuse à mettre en oeuvre, la modification génétique ou chimique d'une protéine impliquant de nombreuses étapes. Enfin, sa mise au point doit être renouvelée pour chaque protéine d'intérêt et cette technique ne peut donc en aucun cas être utilisée de façon routinière, ou bien simultanément pour un grand nombre de protéines membranaires.

Dans une autre technique, on peut, en maintenant la protéine dans sa membrane cellulaire d'origine, ou en l'insérant dans une membrane artificielle (vésicule par exemple), favoriser l'adhésion de la protéine sur un support greffé de chaînes hydrophobes par le biais d'interactions entre les chaînes et la membrane (3-5). Encore une autre technique consiste à utiliser les propriétés de charge des domaines extra-membranaires de la protéine, ou des têtes hydrophiles des lipides de la membrane dans laquelle la protéine est insérée, pour favoriser de simples interactions électrostatiques avec un support chargé.

Toutefois, pour ces deux techniques, soit la protéine est extraite de la membrane, et il est généralement indispensable de travailler en présence de détergent, ce qui rend les protocoles d'immobilisation beaucoup plus complexes, tant parce que les propriétés des solutions sont modifiées (par exemple par diminution de la tension de surface des solutions, qui peut rendre le *spotting* des protéines à la surface des puces peu précis) que parce que la présence de détergent affecte la stabilité de bon nombre de protéines membranaires (en particulier des complexes membranaires), soit des protéines insérées dans des membranes, naturelles ou synthétiques (vésicules lipidiques) sont utilisées, et la technique est alors complexe et pose un problème de sensibilité : la faible densité de protéine d'intérêt peut diminuer le rapport signal/bruit de l'expérience et la présence plus ou moins importante de composants indésirables dans le cas des membranes naturelles (autres protéines, grande variété de lipides naturels, cofacteurs divers) peut introduire un parasitage du signal expérimental avec des réactions indésirables et du bruit de fond.

Au total, les protocoles existants d'immobilisation (ou fixation) de protéines membranaires sur des supports sont clairement insatisfaisants. Il existe donc un besoin pour un procédé de fixation de protéines membranaires sur des supports dans lequel les inconvénients précédemment cités seraient éliminés, c'est-à-dire pour un procédé possédant les caractéristiques suivantes :

- procédé permettant de maintenir les protéines membranaires sous forme hydrosoluble et biochimiquement stabilisée en l'absence totale de détergent, simplifiant ainsi le protocole et évitant une déstabilisation des protéines membranaires,
- procédé universel, pouvant s'appliquer à n'importe quelle protéine membranaire, sans adaptation particulière du protocole expérimental à chaque protéine et sans nécessité d'aucune modification de la protéine, pouvant donc être utilisé y compris lorsque la protéine est mal connue, ou que sa biochimie et sa génétique ne sont pas contrôlées, et
- procédé permettant de fixer les protéines membranaires à la surface d'un support avec une haute densité, favorisant de meilleurs rendements et une meilleure sensibilité des résultats expérimentaux et donc une meilleure interprétation des données.

Les amphipols sont des amphiphiles polymériques présentant une bonne solubilité, de nombreuses chaînes latérales hydrophobes, et des dimensions moléculaires et une flexibilité leur permettant de s'associer en de multiples points avec la surface transmembranaire hydrophobe des protéines (Figure 1, références 6, 7). Le principe de l'attachement multi-point est d'assurer une cinétique de désorption très lente, rendant l'association entre la protéine membranaire et l'amphipol quasi-irréversible.

De plus, il a été montré que l'approche est universelle, en ce sens que toutes les protéines membranaires testées à ce jour, soit plus d'une vingtaine, peuvent être maintenues en solution sous forme de complexes avec les amphipols (7, 8). En outre, les protéines ainsi piégées conservent leur structure native, restent solubles en l'absence de tensioactif libre dans la solution (7-9) et voient leur stabilité au pire égalée mais le plus souvent améliorée par rapport à un maintien en solution en détergent (7, 8, 10). Enfin, lorsque le piégeage d'un mélange est effectué dans des conditions appropriées, toutes les protéines membranaires du mélange sont piégées séparément (8).

Ces molécules représentent donc un outil permettant de stabiliser en solution n'importe quelle protéine membranaire, quelles que soient sa structure, sa fonction et/ou son origine. Toutefois, elles n'ont été utilisées à ce jour que pour solubiliser les protéines membranaires et les manipuler en solution ou pour les stabiliser de façon temporaire, et aucune étude n'a jamais décrit ni suggéré la possibilité de les utiliser en outre comme intermédiaires médiant la fixation de protéines membranaires sur un support. Au contraire, jusqu'à présent, les amphipols n'ont été utilisés que dans le but d'obtenir des complexes hydrosolubles, pouvant se mouvoir librement en solution, à partir de protéines membranaires naturellement insolubles en milieux aqueux, pour stabiliser temporairement de telles protéines membranaires dans des expériences de reconstitution de membrane lipidique (11), ou pour stabiliser de telles protéines membranaires biotinylées fixées par l'interaction usuelle biotine/avidine sur un support solide, l'amphipol ne servant qu'à stabiliser la protéine et n'étant absolument pas impliqué dans l'association de la protéine membranaire au support solide (8).

## DESCRIPTION DE L'INVENTION

Les inventeurs ont trouvé qu'il était en outre possible d'utiliser un amphipol complexé à une protéine membranaire comme intermédiaire pour fixer cette protéine membranaire sur un support, la liaison au support étant réalisée au niveau de l'amphipol.

L'utilisation de l'amphipol comme intermédiaire pour la liaison au support présente de nombreux avantages par rapport aux techniques connues. Le premier avantage majeur est que ce procédé est applicable sans modification du protocole à n'importe quelle protéine membranaire, quelle que soit son origine ou les informations disponibles sur cette protéine. En particulier, le procédé est applicable même à une protéine membranaire dont on ignore l'identité. De plus, le procédé est simple à mettre en oeuvre, notamment du fait de l'absence de nécessité de détergent à l'étape de la fixation du complexe protéine membranaire/amphipol sur le support. Ce procédé permet également de fixer les protéines membranaires à la surface d'un support avec une haute densité, favorisant ainsi de meilleurs rendements et surtout une meilleure sensibilité des résultats d'analyse de liaison de ligands sur la protéine membranaire immobilisée.

Enfin, la présence des amphipols complexés aux protéines membranaires assure leur stabilisation biochimique, permettant ainsi également une plus grande précision et reproductibilité des résultats d'analyse de liaison de ligands sur la protéine membranaire immobilisée.

Le procédé mis au point par les inventeurs permet donc de réaliser facilement, avec une haute densité, des produits comprenant un support et une ou plusieurs protéines membranaires quelconques, connues ou non, fixées à sa surface, les protéines membranaires étant de plus biochimiquement stabilisées en milieu aqueux, facilitant ainsi grandement les expériences d'analyse de liaison de ligands sur les protéines membranaires immobilisées qui peuvent être mises en oeuvre grâce au produit réalisé.

Ainsi, l'invention concerne un produit comprenant un support et au moins une protéine membranaire fixée à sa surface, **caractérisé en ce que** ladite protéine membranaire est fixée sur ledit support par l'intermédiaire d'une molécule amphiphile avec laquelle ladite protéine membranaire est complexée, la molécule amphiphile étant choisie parmi un polymère vinylique amphiphile ou un polypeptide amphiphile, et ladite au moins une protéine membranaire étant avantageusement choisie parmi un antigène, un anticorps, une enzyme, un récepteur cellulaire, un canal ionique, ou une protéine membranaire d'origine virale, bactérienne, ou eucaryote.

Par « amphiphile », on entend une molécule organique qui présente à la fois des propriétés hydrophiles et hydrophobes médiées par des parties différentes de la molécule. Ainsi, une molécule « amphiphile » contient généralement un ou plusieurs groupements hydrophiles et un ou plusieurs groupements hydrophobes.

Dans le cas présent, les groupements hydrophobes de la molécule amphiphile la lient à la protéine membranaire, dont la surface transmembranaire est elle-même hydrophobe, et les groupements hydrophiles permettent de solubiliser l'ensemble du complexe molécule amphiphile/protéine membranaire. De préférence, la molécule amphiphile comprend de nombreuses chaînes latérales hydrophobes, de façon à s'associer en de multiples points avec la surface transmembranaire hydrophobe des protéines membranaires, cet attachement multiple conduisant à une cinétique de désorption très lente, rendant l'association quasi-irréversible. Avantageusement, le nombre de chaînes latérales hydrophobes est supérieur à 10.

De plus, la molécule amphiphile comprend de préférence également de nombreux groupements hydrophiles permettant de solubiliser le complexe molécule amphiphile/protéine membranaire. Avantageusement, le nombre de groupements hydrophiles est supérieur à 20.

De préférence, le rapport du nombre de chaînes latérales hydrophobes au nombre de groupement hydrophiles est compris entre 0,25 et 2,5 ; de préférence entre 0,25 et 2 ; entre 0,25 et 1,5 ; entre 0,25 et 1 ; voire entre 0,25 et 0,5.

Par « polymère vinylique », on entend un polymère composé de motifs de type - $(CH_2-)_n-CR_aR_b-$, où $R_a$ et $R_b$ sont des substituants variés et n est un nombre entier ajustable supérieur ou égal à 1. Avantageusement, n est compris entre 1 et 3, plus avantageusement, n = 1.

Dans un mode de réalisation préféré, la molécule amphiphile est un polymère vinylique amphiphile, c'est-à-dire un polymère vinylique dont un ou plusieurs des substituants ont un caractère hydrophile et un ou plusieurs autres un caractère hydrophobe.

De préférence, la molécule amphiphile est un polymère vinylique de formule (I) :

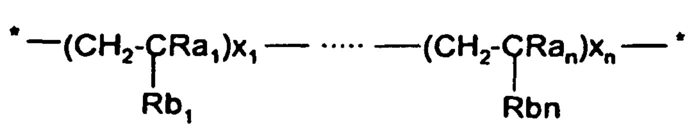
(I)

dans laquelle :

$Ra_1$ à $Ra_n$ identiques ou différents sont l'atome d'hydrogène, ou le radical méthyle :

$Rb_1$ à $Rb_n$ sont différents et choisis parmi :

- un groupe hydrophile choisi parmi
    - un radical carboxylate $-COO^- M^+$, un radical sulfonate $-SO_3^-M^+$, ou un radical phosphonate $-PO_3^-M^+$, $M^+$ étant un contre-ion cationique,
    - un radical ($C_1$-$C_5$) alkylcarboxylate, un radical ($C_1$-$C_5$) alkylsulfonate, ou un radical ($C_1$-$C_5$) alkylphosphonate
    - un phényl sulfonate
    - CONRc1Rc2, Rc1 et Rc2, identiques ou différents étant un radical ($-C(CH_2ORd1)(CH_2ORd2)(CH_2ORd3)$) avec Rd1, Rd2, Rd3 représentant indépendamment l'atome d'hydrogène, un reste sucre, polyoxyalkylène, notamment polyoxyéthylène, ayant de 4 à 10 unités d'oxyde d'alkylène, un radical zwitterionique, un hydroxyalkyl - $(CH_2)$mOH primaire, secondaire ou tertiaire, avec m variant de 1 à 4, un radical ($C_1$-$C_5$) alkylcarboxylate, un radical ($C_1$-$C_5$) alkylsulfonate ou un radical ($C_1$-$C_5$) alkylphosphonate, un reste sucre
    - COORe, Re étant un reste sucre, un hydroxyalkyl -$(CH_2)$mOH primaire, secondaire ou tertiaire, avec m variant de 1 à 4, un polyoxyalkylène, notamment polyoxyéthylène, ayant 4 à 10 unités d'oxyde d'alkylène, un radical $(CH_2)$t-NRf1Rf2, t nombre entier de 1 à 5, Rf1, Rf2 identiques ou différents, étant l'atome d'hydrogène ou un radical ($C_1$-$C_4$) alkyle,
    - un hydroxyle
    - un hydroxyalkyl -$(CH_2)$mOH primaire, secondaire ou tertiaire, avec m variant de 1 à 4,
    - une amine primaire, secondaire, tertiaire
    - un ammonium quaternaire
    - N-formamide, N-alkylformamide,
    - N-acétamide, N-alkylacétamide,
    - N-pyrrolidonyle,
    - CONRg1Rg2, Rg1 et Rg2, identiques ou différents étant l'atome d'hydrogène, un reste sucre, polyoxyalkylène, notamment polyoxyéthylène, ayant de 4 à 10 unités d'oxyde d'alkylène, un radical zwitterionique, un hydroxyalkyl -$(CH_2)$mOH primaire, secondaire ou tertiaire, avec m variant de 1 à 4,
    - COORh ou CONRkR1, Rh étant un radical ($C_1$-$C_5$) alkyle, un alkylsulfonate, ou ayant l'une des significations de Re ou de Rg1 à l'exception d'un atome d'hydrogène, et Rk et Rl ayant indépendamment l'une des significations de Rh et en plus l'un des deux pouvant correspondre à l'atome d'hydrogène ;

- un groupe hydrophobe choisi parmi :
    - un atome d'hydrogène,
    - un atome d'halogène,
    - un radical $-CONH(-C(CH_2ORm1)(CH_2ORm2)(CH_2ORm3))$ avec Rm1, Rm2, Rm3 étant indépendamment un alkyle, un alcényle ou alcynyle linéaire ou ramifié de 3 à 50 atomes de carbone, un carbamoyle d'alkyle (O=C-NH-Rn) ou un acyle (O=C-Ro) avec Rn et Ro étant des radicaux alkyle, alcényle ou alcynyle linéaire ou ramifié de 3 à 50 atomes de carbone,
    - COORp, CORp, COSRp, C-NH-Rp ou CONRq1Rq2, où Rp est un radical alkyle, alcynyle ou alcényle linéaire ou ramifié et/ou cyclique comprenant de 3 à 50 atomes de carbone, et Rq1 et Rq2 identiques ou différents ont l'une des significations de Rp, et de plus l'un des deux peut correspondre à l'atome d'hydrogène,
    - un radical -Rr, -ORr, ou -SRr où Rr représente un groupe alkyle, alcényle ou alcynyle linéaire ou ramifié et/ou cyclique comprenant de 3 à 50 atomes de carbone ; ou

- un groupe amphiphile choisi parmi :
    - un radical alkyl -$(CH_2)$m-Rs avec m compris entre 6 et 20, Rs signifiant un groupement hydrophile de type carboxylate, sulfonate, phosphonate, sulfate, phosphate, zwitterion, ammonium, poly(oxyéthylène), sucre,
    - un radical poly(oxyethylene)-O-alkyl ($-(CH_2CH_2O)$m-Rt) avec Rt un radical alkyle, alcényle, alcynyle linéaire, ramifié ou cyclique de 6 à 20 atomes de carbone,
    - un radical COORu, CORu, COSRu, CONRvRw, Ru signifiant un radical poly(oxyethylene)-O-alkyl

(-($CH_2CH_2O$)m-Rt) avec Rt un radical alkyle, alcényle, alcynyle linéaire, ramifié ou cyclique de 6 à 20 atomes de carbone, un radical glycosylalkyl, Rv pouvant signifier l'atome d'hydrogène ou ayant une des significations de Ru, Rw ayant une des significations de Ru,

- un radical -CONH(-C($CH_2ORx1$)($CH_2OPx2$)($CH_2OPx3$)), avec Rx1, Rx2, Rx3 étant tels qu'un ou deux de ces groupements aient une des significations de Rm1 Rm2, Rm3 et qu'un ou deux de ces groupements soient différents de l'atome d'hydrogène et aient une des significations de Rd1 Rd2, Rd3, Ru, ou bien Rx1, Rx2, Rx3 différents ou identiques et tels qu'au moins l'un des groupement soit différents de l'atome d'hydrogène et ayant une des significations de Ru,

n est un nombre entier supérieur ou égal à 2, de préférence compris entre 2 et 10, entre 2 et 8, entre 2 et 6, entre 2 et 4, avantageusement n égal 3 ;

$x_1$ à $x_n$ correspondent aux pourcentages respectifs des motifs $(\sum_{i=1}^{n} x_i = 100\%)$, où le rapport du pourcentage total de groupes où $Rb_i$ est un groupe hydrophobe ou amphiphile au pourcentage total de groupes où $Rb_i$ est un groupe hydrophile $\sum_{Rb_i hydrophobe} x_i + \sum_{Rb_j amphiphile} x_j \Big/ \sum_{Rb_k hydrophile} x_k$ est compris entre 0,25 et 2,5 ; de préférence entre 0,25 et 2 ; entre 0,25 et 1,5 ; entre 0,25 et 1 ; voire entre 0,25 et 0,5 ; et

la masse molaire moyenne est comprise entre 500 et 100000, avantageusement entre 1000 et 50000, entre 2000 et 25000, plus avantageusement entre 4000 et 15000, entre 6000 et 12000, et de préférence entre 8000 et 10000, voire entre 9000 et 10000 $g.mol^{-1}$.

Par « contre ion cationique », on entend selon l'invention, un cation apte à neutraliser la charge négative portée par l'atome d'oxygène chargé négativement du groupe COO- de R1 quand R1 est $COO\text{-}M^+$. Ce contre-ion peut notamment être un cation alcalin.

Par « reste sucre », on entend selon l'invention un radical mono ou hétéro ou homopolysaccharide, notamment mais pas seulement de formule $(C_nH_{2n-2}O_{n-1})_m$. Si m = 1 le monosaccharide peut être un radical glucosyle, galactosyle, mannosyle, etc. Si m = 2 le disaccharide peut-être un radical maltosyle, lactosyle, saccharosyle, etc.

Par « polyoxyalkylène », on entend selon l'invention un radical de formule $(C_nH_{2n}O)_m$, où n est un nombre entier entre 1 et 6 et m est un nombre entier supérieur ou égal à 2. Cela inclut notamment le polyoxyéthylène où n=2.

Par « radical zwittérionique », on entend selon l'invention on entend selon l'invention un groupement porteur d'une charge positive et d'une charge négative tel qu'un groupement carboxybétaïne de formule générale $-(CH_2)_n-N^+Ry1Ry2-(CH_2)_m-CO_2^-$, un groupement sulfobétaïne de formule générale $-(CH_2)_n-N^+Rz1Rz2-(CH_2)_m-SO_3^-$ où Ry1, Ry2 Rz1 et Rz2 sont des radicaux alkyle, alcényle ou alcyle linéaires, ramifiés ou cycliques et n un entier supérieur ou égal à 1 et m un entier variant de 2 à 4.

Par « alkylsulfonate », on entend selon l'invention un radical de formule générale $-(CH_2)_n-SO_3^-M^+$ où $M^+$ est tel que défini précédemment

Dans tous les cas ci-dessus et ci-dessous où il est fait référence à un alkyle, un alcényle ou un alcynyle linéaire ou ramifié de 3 à 50 atomes de carbone, le radical alkyle, alcényle ou alcynyle linéaire ou ramifié possède avantageusement de 3 à 40, de 3 à 30, de 3 à 25, de 3 à 20, voire de 3 à 18 atomes de carbone.

Avantageusement, le polymère vinylique est plus précisément de formule (II) :

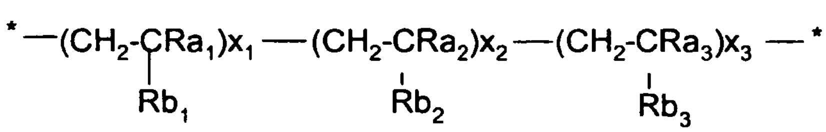

(II)

,

dans laquelle :

$Ra_1$, $Ra_2$ et $Ra_3$ identiques ou différents sont l'atome d'hydrogène, ou le radical méthyle

$Rb_1$ est un groupe hydrophile choisi parmi:

- un radical carboxylate -COO- $M^+$, un radical sulfonate $-SO_3^-M^+$, ou un radical phosphonate $-PP_3^-M^+$, $M^+$ étant

un contre-ion cationique,

- un radical ($C_1$-$C_5$) alkylcarboxylate, radical ($C_1$-$C_5$) alkylsulfonate, ou un radical ($C_1$-$C_5$) alkylphosphonate
- un phényl sulfonate
- CONRc1Rc2, Rc1 et Rc2, identiques ou différents étant un radical (-C($CH_2$ORd1)($CH_2$ORd2)($CH_2$ORd3)) avec Rd1, Rd2, Rd3 représentant indépendamment l'atome d'hydrogène, un reste sucre, polyoxyalkylène, notamment polyoxyéthylène, ayant de 4 à 10 unités d'oxyde d'alkylène, un radical zwitterionique, un hydroxyalkyl-($CH_2$) mOH primaire, secondaire ou tertiaire, avec m variant de 1 à 4, un radical ($C_1$-$C_5$) alkylcarboxylate, un radical ($C_1$-$C_5$) alkylsulfonate ou un radical ($C_1$-$C_5$) alkylphosphonate, un reste sucre

$Rb_2$ est

- un groupe hydrophobe choisi parmi:

  - un atome d'hydrogène,
  - un atome d'halogène,
  - un radical -CONH(-C($CH_2$ORm1)($CH_2$ORm2)($CH_2$ORm3)) avec Rm1, Rm2, Rm3 étant indépendamment un alkyle, un alcényle ou alcynyle linéaire ou ramifié de 3 à 50 atomes de carbone, un carbamoyle d'alkyle (O=C-NH-Rn) ou un acyle (O=C-Ro) avec Rn et Ro étant des radicaux alkyle, alcényle ou alcynyle linéaire ou ramifié de 3 à 50 atomes de carbone,
  - COORp, CORp, CSRp, C-NH-Rp ou CONRq1Rq2, où Rp est un radical alkyle, alcynyle ou alcényle linéaire ou ramifié et/ou cyclique comprenant de 3 à 50 atomes de carbone, et Rq1 et Rq2 identiques ou différents ont l'une des significations de Rp, et de plus l'un des deux peut correspondre à l'atome d'hydrogène,
  - un radical -Rr, -ORr, ou -SRr où Rr représente un groupe alkyle, alcényle ou alcynyle linéaire ou ramifié et/ou cyclique comprenant de 3 à 50 atomes de carbone ; ou

- un groupe amphiphile choisi parmi:

  - un radical alkyl -($CH_2$)m-Rs avec m compris entre 6 et 20, Rs signifiant un groupement hydrophile de type carboxylate, sulfonate, phosphonate, sulfate, phosphate, zwitterion, ammonium, poly(oxyéthylène), sucre,
  - un radical poly(oxyethylene)-O-alkyl (-($CH_2CH_2$O)m-Rt) avec Rt un radical alkyle, alcényle, alcynyle linéaire, ramifié ou cyclique de 6 à 20 atomes de carbone,
  - un radical COORu, CORu, COSRu, CONRvRw, Ru signifiant un radical poly(oxyethylene)-O-alkyl (-($CH_2CH_2$O)m-Rt) avec Rt un radical alkyle, alcényle, alcynyle linéaire, ramifié ou cyclique de 6 à 20 atomes de carbone, un radical glycosylalkyl, Rv pouvant signifier l'atome d'hydrogène ou ayant une des significations de Ru, Rw ayant une des significations de Ru,
  - un radical -CONH(-C($CH_2$ORx1)($CH_2$ORx2)($CH_2$ORx3)), avec Rx1, Rx2, Rx3 étant tels qu'un ou deux de ces groupements aient une des significations de Rm1, Rm2, Rm3 et qu'un ou deux de ces groupements soient différents de l'atome d'hydrogène et aient une des significations de Rd1, Rd2, Rd3, Ru,

ou bien Rx1, Rx2, Rx3 différents ou identiques et tels qu'au moins l'un des groupement soit différents de l'atome d'hydrogène et ayant une des significations de Ru,

$Rb_3$ est un groupe hydrophile choisi parmi:

- COORe, Re étant un reste sucre, un hydroxyalkyl -($CH_2$)mOH primaire, secondaire ou tertiaire, avec m variant de 1 à 4, un polyoxyalkylène, notamment polyoxyéthylène, ayant 4 à 10 unités d'oxyde d'alkylène, un radical ($CH_2$)t-NRf1Rf2, t nombre entier de 1 à 5, Rf1, Rf2 identiques ou différents, étant l'atome d'hydrogène ou un radical ($C_1$-$C_4$) alkyle,
- un hydroxyle
- un hydroxyalkyl -($CH_2$)mOH primaire, secondaire ou tertiaire, avec m variant de 1 à 4,
- une amine primaire, secondaire, tertiaire
- un ammonium quaternaire
- N-formamide, N-akylformamide,
- N-acétamide, N-alkylacétamide,
- N-pyrrolidonyle,
- CONRglRg2, Rg1 et Rg2, identiques ou différents étant l'atome d'hydrogène, un reste sucre, polyoxyalkylène, notamment polyoxyéthylène, ayant de 4 à 10 unités d'oxyde d'alkylène, un radical zwitterionique, un hydroxyalkyl -($CH_2$)mOH primaire, secondaire ou tertiaire, avec m variant de 1 à 4 (R3),

- COORh ou CONRkR1, Rh étant un radical ($C_1$-$C_5$) alkyle, un alkylsulfonate, ou ayant l'une des significations de Re ou de Rg1 à l'exception d'un atome d'hydrogène, et Rk, Rl ayant indépendamment l'une des significations de Rh et en plus l'un des deux pouvant correspondre à l'atome d'hydrogène ;

x1, x2, x3 correspondent aux pourcentages respectifs des motifs, avec

- x 1 compris entre 20 et 90 %
- x2 compris entre 10 et 80 %
- x3 compris entre 0 et 60 %, et
- $x_2/x_1 + x_3$ compris entre 0,25 et 2,5 ; de préférence entre 0,25 et 2 ; entre 0,25 et 1,5 ; entre 0,25 et 1 ; voire entre 0,25 et 0,5 ; et

la masse molaire moyenne étant comprise entre 500 et 100000, avantageusement entre 1000 et 50000, entre 2000 et 25000, plus avantageusement entre 4000 et 15000, entre 6000 et 12000, et de préférence entre 8000 et 10000, voire entre 9000 et 10000 $g.mol^{-1}$.

Avantageusement, dans un polymère vinylique amphiphile de formule (II) :

$Ra_1$, $Ra_2$ et $Ra_3$ identiques ou différents sont l'atome d'hydrogène, ou le radical méthyle ;

$Rb_1$ représente $COO^- M^+$, $M^+$ étant un contre-ion cationique ;

$Rb_2$ représente CONRq1Rq2, où Rq1 et Rq2 représentent indépendamment un radical alkyle, alcynyle ou alcényle linéaire ou ramifié et/ou cyclique comprenant de 3 à 50 atomes de carbone, et de plus l'un des deux peut correspondre à l'atome d'hydrogène ;

$Rb_3$ représente CONRkRl, où Rk et Rl représentent indépendamment un radical ($C_1$-$C_5$) alkyle, un alkylsulfonate, un reste sucre, un hydroxyalkyl -($CH_2$)mOH primaire, secondaire ou tertiaire, avec m variant de 1 à 4, un polyoxyalkylène, notamment polyoxyéthylène, ayant 4 à 10 unités d'oxyde d'alkylène, un radical zwitterionique, un radical ($CH_2$)t-NRf1Rf2, t nombre entier de 1 à 5, Rf1, Rf2 identiques ou différents étant l'atome d'hydrogène ou un radical ($C_1$-$C_4$) alkyl, et de plus l'un des deux de Rk et Rl peut correspondre à l'atome d'hydrogène.

Plus précisément, dans un mode de réalisation particulier avantageux d'un polymère vinylique de formule (II) :

$Ra_1$, $Ra_2$ et $Ra_3$ identiques ou différents sont l'atome d'hydrogène, ou le radical méthyle ;

$Rb_1$ représente $COO^- M^+$, $M^+$ étant $Na^+$ ou $K^+$;

$Rb_2$ représente CONRq 1 Rq2, où Rq 1 est un n-octyle, et Rq2 est H ;

x1 est compris entre 70 et 80 %, x2 est compris entre 20 et 30 %, et x3 est 0 % ; et la masse molaire moyenne est comprise entre 2000 et 50 000 $g.mol^{-1}$.

Dans un autre mode de réalisation particulier avantageux d'un polymère vinylique de formule (II) :

$Ra_1$, $Ra_2$ et $Ra_3$ identiques ou différents sont l'atome d'hydrogène, ou le radical méthyle ;

$Rb_1$ représente $COO^- M^+$, $M^+$étant un contre-ion cationique ;

$Rb_2$ représente CONRq1Rq2, où Rq 1 est un n-octyle, et Rq2 est H ;

$Rb_3$ est tel que défini précédemment dans la formule (II), c'est-à-dire représente CONRkRl, où Rk et Rl représentent indépendamment un radical ($C_1$-$C_5$) alkyle, un alkylsulfonate, un reste sucre, un hydroxyalkyl -($CH_2$)mOH primaire, secondaire ou tertiaire, avec m variant de 1 à 4, un polyoxyalkylène, notamment polyoxyéthylène, ayant 4 à 10 unités d'oxyde d'alkylène, un radical zwitterionique, un radical ($CH_2$)t-NRf1Rf2, t nombre entier de 1 à 5, Rf1, Rf2 identiques ou différents étant l'atome d'hydrogène ou un radical ($C_1$-$C_4$) alkyle, et de plus l'un des deux de Rk et

Rl peut correspondre à l'atome d'hydrogène ;

x1 est compris entre 30 et 40 %, x2 est compris entre 20 et 30 %, et x3 est compris entre 30 et 50 % ; et

la masse molaire moyenne est comprise entre 2000 et 50 000 g.mol$^{-1}$.

Dans un autre mode de réalisation, la molécule amphiphile est un polymère amphiphile de type polypeptide amphiphile, c'est-à-dire un polymère amphiphile d'acide aminés. Pour être amphiphile, un polypeptide devrait comporter un mélange d'acides aminés hydrophobes et d'acides aminés hydrophiles dans les proportions indiquées précédemment. Alternativement, un peptide hydrophile peut être modifié par greffage de chaînes latérales hydrophobes et ainsi gagner un caractère amphiphile.

Dans un mode de réalisation avantageux d'un quelconque produit selon l'invention tel que décrit précédemment, le support est un support solide. En effet, ce mode de réalisation particulier est avantageux pour un certain nombre d'applications, notamment pour la réalisation de puces porteuses de protéines membranaires, de billes revêtues de protéines membranaires, de membranes revêtues de protéines membranaires, de fibres ou de nanotubes revêtus de protéines membranaires. Ainsi, de préférence, le support solide est choisi parmi une puce, une bille, une membrane poreuse ou non poreuse, une fibre, ou un nanotube.

Par « puce », on entend une petite plaque ou lame d'un matériau solide à la surface de laquelle on peut greffer des biomolécules telles que des acides nucléiques ou des protéines.

Par « bille », on entend une particule globulaire, par exemple sphérique. Une telle bille peut de plus posséder toute caractéristique utile pour l'application envisagée, par exemple elle peut être magnétique de façon à pouvoir être aisément séparée du milieu dans lequel elle se trouve.

Par « membrane poreuse », on entend une mince couche de matériau poreux et flexible, la taille des pores du matériau permettant le passage des molécules de dimension inférieure à une certaine valeur, et interdisant le passage des molécules de dimension supérieure.

Par « membrane non poreuse », on entend une mince couche de matériau flexible, éventuellement façonnée en faisceaux de tubes minces, mille-feuilles, ou autres dispositifs permettant d'en multiplier la surface.

Par « fibre », on entend une formation élémentaire d'aspect filamenteux, se présentant généralement sous forme de faisceaux. Les fibres peuvent être « naturelles », c'est-à-dire préexistant dans la nature, ou « chimiques », c'est-à-dire préparées par l'homme. Des exemples de fibres naturelles incluent les fibres végétales telles que coton, lin, chanvre, ramie, jute, abaca, alfa, kapok, coco, genet, henequen, kenaf, maguey, sisal, bambou, les fibres animales telles que laine, alpaga, chameau, cachemire, guanaco, lapin angora, mohair, vigogne, yack, soie, et les fibres d'origine minérale tel que l'amiante. Des exemples de fibres chimiques incluent les fibres artificielles d'origine végétale cellulosiques telles que viscose, cupro, modal, non cellulosiques comme l'alginate, les fibres d'origine animale comme la chitine, les fibres synthétiques d'origine organique telles que polyamides, polyesters, chlorofibres, acryliques, modacryliques, polypropylène, élastodiène, élasthanne, polyuréthane, vinylal, et les fibres synthétiques d'origine minérale telles que aramide, verre, et textile.

Par « nanotube », on entend une structure cristalline particulière, de forme tubulaire, creuse, composée d'atomes disposés régulièrement en forme de polygones, notamment de pentagones, hexagones et/ou heptagones, obtenue à partir de certains matériaux, en particulier le carbone et nitrure de bore. Différents types de nanotubes, en particulier de nanotubes de carbone, sont bien connus de l'homme du métier.

Dans un autre mode de réalisation d'un quelconque produit selon l'invention telle que décrit précédemment, le support est une macromolécule ou particule soluble choisie parmi les polymères, les dendrimères, les vésicules, ou les micelles.

Par « vésicule », on entend un assemblage de molécules amphiphiles constitué d'une (ou plusieurs) bicouche(s) ou membrane(s) refermée(s) sur elle(s)-même(s) et délimitant une (ou plusieurs) cavité(s) interne(s) aqueuse(s) isolée(s) du milieu extérieur.

Par « micelle », on entend un agrégat globulaire, discoïdal ou linéaire de molécules possédant une tête polaire hydrophile dirigée vers le solvant et une chaîne hydrophobe dirigée vers l'intérieur.

Quel que soit le support, il faut que celui-ci puisse interagir avec la molécule amphiphile telle que décrit précédemment pour former une liaison entre le support et la protéine membranaire complexée à la molécule amphiphile. Différents types de liaisons peuvent être formées entre le support et la molécule amphiphile.

Ainsi, dans un mode de réalisation d'un produit quelconque selon l'invention tel que décrit précédemment, la fixation de la molécule amphiphile sur le support est médiée par une liaison hydrophobe, une liaison ionique, une liaison spécifique de type récepteur-ligand entre au moins un groupement fonctionnel de la molécule amphiphile et au moins un groupement fonctionnel à la surface du support, ou une liaison covalente entre au moins un groupement fonctionnel réactif de la molécule amphiphile et au moins un groupement fonctionnel réactif à la surface du support.

En effet, la molécule amphiphile possédant de nombreuses chaînes latérales hydrophobes, certaines de ces

chaînes peuvent interagir avec un support lui-même hydrophobe, que celui-ci soit fait d'un matériau hydrophobe ou soit revêtu de groupements hydrophobes, tels par exemple que des groupements alkyles, par exemple octyles ou stéaryles (silice, verre, quartz, or, résine ou autre support, greffé en $C_n$, où n varie entre 4 et 30).

Alternativement, la liaison entre le support et la molécule amphiphile peut être une liaison ionique entre des groupements chargés de la molécule amphiphile et des groupements chargés du support. Par exemple, dans le cas où la molécule amphiphile est un polymère vinylique de formule (II) quelconque tel que décrit précédemment, les groupements $CO_2^-$ du polymère vinylique peuvent former une liaison ionique avec des groupements chargés positivement présents sur le support, soit naturellement, soit après traitement du support ou après greffage de groupements chargés positivement sur le support (par exemple le groupement ammonium quaternaire utilisé pour la synthèse de résines anioniques telles que QAE polyoside).

Encore une autre possibilité de liaison entre le support et la molécule amphiphile consiste en une liaison spécifique de type récepteur-ligand entre au moins un groupement de la molécule amphiphile et au moins une molécule à la surface du support, ou vice-versa. En effet, il existe de nombreux couples de molécules de type récepteur-ligand, c'est-à-dire capables d'interagir spécifiquement l'une avec l'autre. Ainsi, si un ou plusieurs groupements fonctionnels représentant la première moitié d'un couple de molécules récepteur-ligand sont greffés sur la molécule amphiphile, et qu'un ou plusieurs groupements fonctionnels représentant la deuxième moitié du couple de molécules récepteur-ligand sont greffés ou adsorbés ou fixés d'une façon quelconque sur le support, alors une liaison spécifique peut être formée entre le support et la molécule amphiphile.

Ainsi, dans un mode de réalisation avantageux d'un produit quelconque selon l'invention tel que décrit précédemment, le produit est **caractérisé en ce que** :

a) la molécule amphiphile comprend en outre au moins un groupement fonctionnel constituant la première moitié d'un couple de molécules récepteur-ligand,

b) le support comprend en outre au moins un groupement fonctionnel fixé à sa surface constituant la deuxième moitié dudit couple de molécules récepteur-ligand, et

c) la fixation de la molécule amphiphile sur le support est médiée par une liaison spécifique de type récepteur-ligand entre le(s) groupement(s) fonctionnel(s) de la molécule amphiphile et le(s) groupement(s) fonctionnel(s) fixé(s) sur le support.

Comme indiqué précédemment, il existe de nombreux couples de molécules de type récepteur-ligand capables d'interagir spécifiquement l'une avec l'autre. Par exemple, des couples (groupement fonctionnel de la molécule amphiphile/groupement fonctionnel fixé sur le support) appropriés pour un produit selon l'invention où l'interaction entre le support et la molécule amphiphile est médiée par une liaison spécifique de type récepteur-ligand peuvent être choisis parmi les couples utilisés classiquement en chromatographie d'affinité, et notamment parmi :

- les couples ayant une interaction de type enzyme-substrat constitués d'une petite molécule reconnue par une protéine ayant une forte affinité pour ce substrat, tels que les couples (biotine/avidine), (glutathion/glutathion S-transférase, protéines fixant le glutathion, ou protéines de fusion incluant la glutathion S-transférase), (calmoduline/ATPase, protéine kinase, phosphodiestérase, ou neurotransmetteur), (L-arginine ou *p*-aminobenzamidine/protéase à sérine), (L-lysine/plasminogène (et activateur) ou ARNr), (AMP, ADP, ou ATP/enzyme à cofacteurs), (lectine/protéine glucannée, glucolipide, ou polysaccharide), (héparine/facteur de croissance et de coagulation, récepteur stéroïdien, endonucléase, lipoprotéine, ou lipase), ou (Cibacron blue® /enzymes à cofacteurs NAD ou NADP, albumine, facteur de coagulation, ou interféron), ainsi que les couples inversés correspondants

- les couples de type (antigène/anticorps) ou (haptène/anticorps), ou inversement de type (anticorps/antigène) ou (anticorps/haptène),

- les couples ayant une interaction de type chélation, constitués d'un groupement impliquant des chélations avec des métaux de transition, tels que (acide nitrilotriacétique (NTA)/métal de transition), (EDTA/ métal de transition),

- les couples de type (acide nucléique/acide nucléique complémentaire) et notamment (oligonucléotide/oligonucléotide complémentaire), et

- les couples d'affinité de type (acide phénylboronique (APB)/acide salicylhydroxamique (ASH)).

Avantageusement, le couple (groupement fonctionnel de la molécule amphiphile/groupement fonctionnel fixé

sur le support) est choisi parmi (biotine/avidine), (avidine/biotine), (glutathion/glutathion S-transférase), (glutathion/S-transférase glutathion), (antigène/anticorps), (haptène/anticorps), (anticorps/antigène), (anticorps/haptène), (oligonucléotide/oligonucléotide complémentaire).

Ainsi, dans les produits selon la présente invention où la molécule amphiphile et le support sont liés par une liaison spécifique de type récepteur-ligand entre le(s) groupement(s) fonctionnel(s) de la molécule amphiphile et le(s) groupement(s) fonctionnel(s) fixé(s) sur le support, il est particulièrement avantageux d'utiliser comme molécule amphiphile un polymère vinylique de formule (I) ou (II) telles que définies précédemment, de façon générale ou tel que dans les modes de réalisations avantageux des polymères de formule (I) ou (II) précédemment décrits, qui comprenne en outre un pourcentage compris entre 0 et 4% d'un monomère de formule

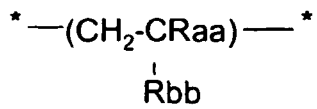

(III)

,

dans laquelle :

Raa est l'atome d'hydrogène ou le radical méthyle ;

Rbb représente un groupe COORcc, ou -COSRcc ou -CORcc ou CONRccRdd, où

Rcc représente le groupement fonctionnel de la molécule amphiphile constituant la première moitié du couple récepteur-ligand, et

Rdd représente un radical ($C_1$-$C_5$) alkyle, un alkylsulfonate, l'atome d'hydrogène, un reste sucre, un hydroxyalkyl -($CH_2$)mOH primaire, secondaire ou tertiaire, avec m variant de 1 à 4, un polyoxyalkylène, notamment polyoxyéthylène, ayant 4 à 10 unités d'oxyde d'alkylène, un radical zwitterionique, un radical ($CH_2$)t-NRf1Rf2, t nombre entier de 1 à 5, Rf1, Rf2 identiques ou différents étant l'atome d'hydrogène ou un radical ($C_1$-$C_4$) alkyle.

Dans ces produits particulièrement avantageux, le couple (groupement fonctionnel de la molécule amphiphile/groupement fonctionnel fixé sur le support) est avantageusement choisi parmi ceux décrits précédemment.

Enfin, on peut envisager de fixer la molécule amphiphile sur le support par le biais d'une liaison covalente entre au moins un groupement fonctionnel réactif de la molécule amphiphile et au moins un groupement fonctionnel réactif du support (12-15). Ainsi, si un ou plusieurs groupements fonctionnels sont greffés sur la molécule amphiphile et qu'un ou plusieurs groupements fonctionnels pouvant réagir chimiquement avec le ou les groupement de la molécule amphiphile sont adsorbés ou greffés d'une façon quelconque sur le support, on peut envisager de faire réagir chimiquement entre eux les différents groupements dans des conditions favorables à la formations d'une liaison covalente entre le support et la molécule amphiphile. Concernant le groupement fonctionnel réactif de la molécule amphiphile et le groupement fonctionnel réactif du support capables de former dans des conditions favorables une liaison covalente, on parlera alors de « couple réactif chimique ».

Ainsi, dans un mode de réalisation avantageux d'un produit quelconque selon l'invention tel que décrit précédemment, le produit est **caractérisé en ce que** :

a) la molécule amphiphile comprend en outre au moins un groupement fonctionnel constituant la première moitié d'un couple réactif chimique,

b) le support comprend en outre au moins un groupement fonctionnel fixé à sa surface constituant la deuxième moitié dudit couple réactif chimique, et

c) la fixation de la molécule amphiphile sur le support est effectuée par réaction chimique entre le(s) groupement(s) fonctionnel(s) de la molécule amphiphile et le(s) groupement(s) fonctionnel(s) fixé(s) sur le support.

A titre d'exemple, il serait possible d'envisager l'utilisation d'un procédé connu pour sa grande sélectivité qui consiste à adsorber à la surface un dérivé quinonique qui, dans certaines conditions compatibles avec nos expériences, pourra réagir suivant un mécanisme type Diels-Aldert de manière spontanée avec un groupement cyclopentadienyle fixé sur la molécule amphiphile. Les liaisons formées sont covalentes.

De même, il est possible d'envisager le même type de réaction sélective entre un support comportant à sa surface des groupements azotures ($N_3$) et un polymère amphiphile comportant un groupement alkynyle (triple liaison).
De même, on peut envisager de fonctionnaliser la molécule amphiphile par un groupement alkoxylamine (ou un groupement carbonyle) pouvant se condenser très sélectivement sur une fonction carbonyle (ou un groupement alkoxylamine) fixé à la surface du support. La liaison formée (alkoxylimine) est covalente.
Ainsi, dans les produits selon la présente invention où la molécule amphiphile et le support sont liés par une liaison covalente entre le(s) groupement(s) fonctionnel(s) réactif(s) de la molécule amphiphile et le(s) groupement(s) fonctionnel(s) réactif(s) fixé(s) sur le support, il est particulièrement avantageux d'utiliser comme molécule amphiphile un polymère vinylique de formule (I) ou (II) telles que définies précédemment, de façon générale ou tel que dans les modes de réalisations avantageux des polymères de formule (I) ou (II) précédemment décrits, qui comprenne en outre un pourcentage compris entre 0 et 4% d'un monomère de formule :

$$^{*}—(CH_2\text{-}\underset{\text{Rbbb}}{\underset{|}{C}}\text{Raaa})—^{*} \quad \text{(IV)}$$

,

dans laquelle :

Raaa est l'atome d'hydrogène ou le radical méthyle ;

Rbbb représente un groupe COORccc, ou -COSRccc ou -CORccc ou CONRcccRddd, où

Rccc représente le groupement fonctionnel réactif de la molécule amphiphile constituant la première moitié d'un couple réactif chimique, et

Rdd représente un radical ($C_1$-$C_5$) alkyle, un alkylsulfonate, l'atome d'hydrogène, un reste sucre, un hydroxyalkyl -$(CH_2)$mOH primaire, secondaire ou tertiaire, avec m variant de 1 à 4, un polyoxyalkylène, notamment polyoxyéthylène, ayant 4 à 10 unités d'oxyde d'alkylène, un radical zwitterionique, un radical $(CH_2)$t-NRf1Rf2, t nombre entier de 1 à 5, Rf1, Rf2 identiques ou différents étant l'atome d'hydrogène ou un radical ($C_1$-$C_4$) alkyle.

Dans ces produits particulièrement avantageux, le couple (groupement fonctionnel réactif de la molécule amphiphile/groupement fonctionnel réactif fixé sur le support) est avantageusement choisi parmi ceux décrits précédemment.
Les produits selon l'invention décrits précédemment peuvent comprendre, fixée à leur surface par l'intermédiaire de la molécule amphiphile, n'importe quelle protéine membranaire. C'est un des grands avantages des produits selon l'invention, puisque n'importe quelle protéine membranaire, quelle que soit sa structure, sa fonction, et qu'elle soit connue ou non, peut être immobilisée sur un support sous la forme d'un produit selon l'invention. Dans certains modes de réalisation, la (ou les) protéine(s) membranaire(s) immobilisée(s) à la surface du support est (sont) choisie(s) parmi un antigène, un anticorps, une enzyme, un récepteur cellulaire, un canal ionique, ou une protéine membranaire d'origine virale, bactérienne, ou eucaryote. La ou les protéine(s) membranaire(s) immobilisée(s) à la surface du support peut (peuvent) être choisie(s) en fonction de l'application envisagée pour le produit selon l'invention.
L'invention concerne également un procédé de préparation d'un produit selon l'invention tel que décrit précédemment, comprenant :

a) la fourniture d'au moins une protéine membranaire, d'un support et d'une molécule amphiphile telle que décrite précédemment dans un quelconque mode de réalisation,

b) la formation d'un complexe entre la molécule amphiphile et la protéine membranaire, et

c) la fixation de la molécule amphiphile du complexe sur le support par une liaison hydrophobe, une liaison ionique, une liaison spécifique de type récepteur-ligand entre au moins un groupement fonctionnel de la molécule amphiphile et au moins un groupement fonctionnel à la surface du support, ou une liaison covalente entre au moins un groupement fonctionnel réactif de la molécule amphiphile et au moins un groupement fonctionnel réactif à la surface du support.

A titre d'exemple, la formation du complexe entre la molécule amphiphile et la protéine membranaire (étape

b) peut être réalisée de la façon suivante (7-10) :

- à une solution de protéine(s) membranaire(s) maintenue(s) soluble(s) par l'utilisation de détergent à une concentration supérieure à sa concentration micellaire critique, on ajoute une masse de molécule amphiphile égale à 0,5 à 10 fois la masse de protéine.

- Après 15 min d'incubation à 4°C, on élimine le détergent soit par adsorption sur des Bio-beads®, soit par dialyse, soit en diluant sous la concentration micellaire critique du détergent puis en concentrant la préparation sur un système filtrant laissant passer le détergent mais non la protéine (système Centricon® ou Amicon®, par exemple), soit en précipitant le détergent, etc. On peut aussi utiliser successivement plusieurs de ces procédures pour s'assurer d'une totale élimination du détergent.

- A ce stade et selon la nature et la taille de la protéine utilisée, on peut ensuite laver les complexes de l'excès de molécule amphiphile soit en centrifugeant la préparation sur un gradient de sucrose, soit en séparant les complexes des molécules amphiphiles sur tamis moléculaire ou colonne d'affinité.

- Alternativement, la formation des complexes protéine(s) membranaire(s)/molécule amphiphile peut être réalisée par diverses autres voies telles que l'extraction directe, aidée ou non par la présence de détergent, des protéines des préparations de départ (membranes biologiques, corps d'inclusion etc.), le piégeage lors d'un protocole de renaturation, la synthèse acellulaire etc.

La fixation de la molécule amphiphile du complexe sur le support (étape c) peut être réalisée de différentes façons : ainsi, à titre d'exemple, dans le cas de supports solides, on peut déposer des solutions de complexe entre la (les) protéine(s) membranaire(s) et la molécule amphiphile sur le support, en respectant les conditions optimales de formation de l'interaction entre la molécule amphiphile et le support (par exemple, pour une interaction de type avidine/biotine, en tampon NaCl et pH = 7,4) : le temps d'incubation dépendra et de la nature du support, et du type d'interaction la molécule amphiphile/support choisi.

Les produits selon l'invention, obtenus par les procédés décrits ci-dessus, ont de nombreuses applications possibles, notamment en terme de diagnostic, de drug design, ou de biotechnologies.

Ainsi, l'invention concerne notamment l'utilisation d'un produit selon l'invention comprenant un support et au moins une protéine membranaire fixée à sa surface tel que décrit précédemment pour détecter la présence ou l'absence dans un échantillon biologique d'un ligand de ladite au moins une protéine membranaire.

Par « échantillon biologique », on entend tout type d'échantillon comprenant de la matière biologique. En particulier, un tel échantillon biologique peut être choisi parmi un échantillon sanguin, un échantillon de lymphe, de sérum, d'urine, de selles, de salive, un échantillon de tissu, une biopsie. L'échantillon peut provenir de tout type d'organisme, notamment d'un être humain, d'un animal, d'un microorganisme, d'un virus ou d'une plante. L'échantillon brut, prélevé à partir de l'organisme d'intérêt, peut ensuite avoir été transformé pour le rendre acceptable pour l'analyse à suivre. Ce ne sera pas le cas lorsque la molécule à détecter est directement accessible dans l'échantillon. En revanche, si la molécule à détecter n'est pas directement accessible dans l'échantillon, il conviendra de transformer l'échantillon pour qu'elle le devienne. Par exemple, si l'échantillon comprend des cellules mais que celles-ci ne sont pas en solution et que le ligand à détecter est lui-même membranaire, l'échantillon peut être transformé pour donner une solution de cellules, à la surface desquelles le ligand est directement détectable. Si le ligand à détecter est une molécule intracellulaire, l'échantillon devrait être transformé pour que le ligand soit directement accessible. Par exemple si le ligand à détecter est une protéine intracellulaire, de nombreuses techniques bien connues de l'homme du métier existent pour extraire les protéines à partir d'un échantillon biologique. Si le ligand à détecter est un acide nucléique, de nombreuses techniques bien connues de l'homme du métier existent également pour extraire les acides nucléiques à partir d'un échantillon biologique. Même chose pour d'autres types de ligands comme des lipides ou des sucres.

La détection de la présence ou de l'absence du ligand de la protéine membranaire dans l'échantillon biologique peut être réalisée par différentes techniques connues de l'homme du métier. Par exemple, la détection de la liaison du ligand sur la protéine membranaire peut être réalisée en utilisant la technique de résonance de plasmon de surface, qui permet la détection et le suivi en temps réel des interactions entre des molécules circulantes et une ou des molécule(s) immobilisée(s), grâce à l'observation en continu d'une modification de résonance de plasmon de surface induite par l'interaction des molécules circulantes avec la puce servant de support d'analyse. Mais la liaison du ligand à la protéine membranaire immobilisée peut aussi être détectée par d'autres techniques. En particulier, si le ligand dont la présence est testée est connu, il est possible d'utiliser des techniques bien connues de type ELISA, où le ligand fixé sur la protéine membranaire est détecté grâce à une troisième molécule se liant spécifiquement à ce ligand (par exemple un anticorps spécifique, ou si le ligand peut lier plusieurs protéines membranaires à la fois, une seconde protéine membranaire sous forme soluble), cette troisième molécule étant détectable par une émission de fluorescence, une réaction enzymatique,

sa radioactivité, ou tout autre moyen conventionnellement utilisé dans ce type de technique.

Plus précisément, une telle utilisation d'un produit selon l'invention pour détecter la présence ou l'absence dans un échantillon biologique d'un ligand de ladite au moins une protéine membranaire peut être appliquée pour le diagnostic de la présence ou de l'absence du ligand d'une protéine membranaire dans un échantillon biologique. Notamment, dans le cas où ladite au moins une protéine membranaire est un antigène membranaire d'un agent pathogène, un produit selon l'invention comprenant un support et cet antigène membranaire d'un agent pathogène fixé à sa surface tel que décrit précédemment peut être utilisé pour diagnostiquer chez un sujet la présence ou l'absence d'une exposition à cet agent pathogène, en détectant la présence ou l'absence d'anticorps dirigés contre ledit antigène dans le sérum de ce sujet. Ainsi, dans un mode de réalisation avantageux d'une telle utilisation, ladite au moins une protéine membranaire est un antigène membranaire d'un agent pathogène, ledit échantillon biologique est un échantillon de sérum, et ledit ligand à détecter est un anticorps dirigé contre ledit antigène.

L'invention concerne également l'utilisation d'un produit selon l'invention comprenant un support et au moins une protéine membranaire fixée à sa surface tel que décrit précédemment pour cribler une banque de composés à la recherche de ligands de ladite au moins une protéine membranaire.

En effet, une telle utilisation est très utile en pharmacologie pour des protéines membranaires constituant des cibles thérapeutiques. Pour chaque nouvelle cible membranaire identifiée, on réalise généralement un criblage de banques de composés pour identifier différents ligands candidats médicaments potentiels, agonistes ou antagonistes, de la cible en question, ces candidats étant ensuite optimisés en terme d'efficacité et d'absence de toxicité.

Comme indiqué précédemment, l'avantage des produits selon l'invention pour une telle application est que les protéines membranaires immobilisées sur le support, du fait qu'elles sont complexées avec la molécule amphiphile, sont complètement solubles en solution aqueuse, et stabilisées biochimiquement dans leur conformation naturelle. Ainsi, la détection peut être réalisée en milieu aqueux tout en conservant la structure native de la protéine membranaire, permettant ainsi non seulement de simplifier le protocole de détection de la liaison, mais aussi de s'assurer que la protéine membranaire étudiée est bien dans son état natif, et donc susceptible de lier, avec la même affinité, les mêmes composés qu'au sein de l'organisme vivant.

Dans ce cas, outre les techniques de détection de la liaison d'un ligand à la protéine membranaire décrites précédemment, il est possible d'utiliser des composés tests directement détectables par fluorescence, colorimétrie ou tout autre type de détection.

L'invention concerne en outre l'utilisation d'un produit selon l'invention comprenant un support et au moins une protéine membranaire fixée à sa surface tel que décrit précédemment, dans lequel ladite au moins une protéine est une enzyme, pour la transformation du substrat de ladite enzyme dans des conditions contrôlées. En effet, un produit selon l'invention peut notamment être une membrane fonctionnalisée, c'est-à-dire une membrane sur laquelle est immobilisé un « tapis » d'enzyme membranaire, cette répartition plus ou moins régulière d'enzyme sur la membrane ayant été réalisée grâce à un procédé selon l'invention. En effet, cela permet notamment de transformer une solution d'un substrat de cette enzyme membranaire en une solution du produit généré après action de l'enzyme sur ce substrat par simple filtration de la solution au travers de la membrane ainsi fonctionnalisée, ou passage de la solution au travers d'un faisceau de tubes à la surface interne desquels l'enzyme membranaire est fixée selon l'invention.

L'invention repose d'une manière générale sur le principe d'utiliser des molécules amphiphiles pour immobiliser des protéines membranaires quelconques sur un support, en absence de détergent ou à des concentrations de détergent inférieures à la concentration micellaire critique (CMC), et dans des conditions où la protéine membranaire est biochimiquement stabilisée.

L'invention concerne donc également un kit pour la mise en oeuvre d'un procédé de préparation d'un produit selon l'invention tel que décrit précédemment, comprenant un support et une molécule amphiphile, **caractérisé en ce que** ladite molécule amphiphile et ledit support interagissent par une liaison hydrophobe, une liaison ionique, une liaison spécifique de type récepteur-ligand entre au moins un groupement fonctionnel de la molécule amphiphile et au moins un groupement fonctionnel à la surface du support, ou une liaison covalente entre au moins un groupement fonctionnel réactif de la molécule amphiphile et au moins un groupement fonctionnel réactif à la surface du support. Le support et la molécule amphiphile peuvent être tout type de support ou de molécule amphiphile tels que décrit précédemment, sous réserve que les deux entités du kit, support et molécule amphiphile, interagissent par une liaison hydrophobe, une liaison ionique, une liaison spécifique de type récepteur-ligand entre au moins un groupement fonctionnel de la molécule amphiphile et au moins un groupement fonctionnel à la surface du support, ou une liaison covalente entre au moins un groupement fonctionnel réactif de la molécule amphiphile et au moins un groupement fonctionnel réactif à la surface du support.

Dans un mode de réalisation avantageux d'un tel kit selon l'invention, le support et la molécule amphiphile interagissent par une liaison spécifique de type récepteur-ligand entre au moins un groupement fonctionnel de la molécule amphiphile et au moins un groupement fonctionnel à la surface du support. Plus précisément, de préférence, le kit est **caractérisé en ce que** :

a) la molécule amphiphile comprend en outre au moins un groupement fonctionnel constituant la première moitié d'un couple de molécules récepteur-ligand,

b) le support comprend en outre au moins un groupement fonctionnel fixé à sa surface constituant la deuxième moitié dudit couple de molécules récepteur-ligand, et

c) la fixation de la molécule amphiphile sur le support est médiée par une liaison spécifique de type récepteur-ligand entre le(s) groupement(s) fonctionnel(s) de la molécule amphiphile et le(s) groupement(s) fonctionnel(s) fixé(s) sur le support.

Avantageusement, le couple (groupement fonctionnel de la molécule amphiphile/groupement fonctionnel fixé sur le support) est choisi parmi les couples (biotine/avidine), (glutathion/glutathion S-transférase, protéines fixant le glutathion, ou protéines de fusion incluant la glutathion S-transférase), (calmoduline/ATPase, protéine kinase, phosphodiestérase, ou neurotransmetteur), (L-arginine ou *p*-aminobenzamidine/protéase à sérine), (L-lysine/plasminogène (et activateur) ou ARNr), (AMP, ADP, ou ATP/enzyme à cofacteurs), (lectine/protéine glucannée, glucolipide, ou polysaccharide), (héparine/facteur de croissance et de coagulation, récepteur stéroïdien, endonucléase, lipoprotéine, ou lipase), ou (Cibacron blue® /enzymes à cofacteurs NAD ou NADP, albumine, facteur de coagulation, ou interféron), ou les couples inversés correspondants, (antigène/anticorps), (haptène/anticorps), (anticorps/antigène), (anticorps/haptène), (acide nitrilotriacétique (NTA)/métal de transition), (EDTA/ métal de transition), (acide phénylboronique (APB)/acide salicylhydroxamique (ASH)), ou (oligonucléotide/oligonucléotide complémentaire).

Dans un kit selon la présente invention où la molécule amphiphile et le support sont liés par une liaison spécifique de type récepteur-ligand entre le(s) groupement(s) fonctionnel(s) de la molécule amphiphile et le(s) groupement(s) fonctionnel(s) fixé(s) sur le support, il est particulièrement avantageux d'utiliser comme molécule amphiphile un polymère vinylique de formule (I) ou (II) telles que définies précédemment, de façon générale ou tel que dans les modes de réalisations avantageux des polymères de formule (I) ou (II) précédemment décrits, qui comprenne en outre un pourcentage compris entre 0 et 4% d'un monomère de formule :

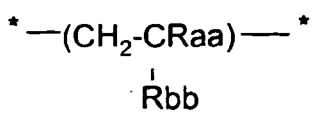

(III)

,

dans laquelle :

Raa est l'atome d'hydrogène ou le radical méthyle ;

Rbb représente un groupe COORcc, ou -COSRcc ou -CORcc ou CONRccRdd, où

Rcc représente le groupement fonctionnel de la molécule amphiphile constituant la première moitié du couple récepteur-ligand, et

Rdd représente un radical ($C_1$-$C_5$) alkyle, un alkylsulfonate, l'atome d'hydrogène, un reste sucre, un hydroxyalkyl -$(CH_2)$mOH primaire, secondaire ou tertiaire, avec m variant de 1 à 4, un polyoxyalkylène, notamment polyoxyéthylène, ayant 4 à 10 unités d'oxyde d'alkylène, un radical zwitterionique, un radical $(CH_2)$t-NRf1Rf2, t nombre entier de 1 à 5, Rf1, Rf2 identiques ou différents étant l'atome d'hydrogène ou un radical ($C_1$-$C_4$) alkyle.

Dans ce cas, le couple (groupement fonctionnel de la molécule amphiphile/groupement fonctionnel fixé sur le support) est avantageusement choisi parmi ceux décrits précédemment.

Dans un autre mode de réalisation avantageux d'un kit selon l'invention, le support et la molécule amphiphile interagissent par le biais d'une liaison covalente entre au moins un groupement fonctionnel réactif de la molécule amphiphile et au moins un groupement fonctionnel réactif du support. Plus précisément, de préférence, le kit est **caractérisé en ce que** :

a) la molécule amphiphile comprend en outre au moins un groupement fonctionnel constituant la première moitié d'un couple réactif chimique,

b) le support comprend en outre au moins un groupement fonctionnel fixé à sa surface constituant la deuxième moitié dudit couple réactif chimique, et

c) la fixation de la molécule amphiphile sur le support est effectuée par réaction chimique entre le(s) groupement (s) fonctionnel(s) de la molécule amphiphile et le(s) groupement(s) fonctionnel(s) fixé(s) sur le support.

Dans un kit selon la présente invention où la molécule amphiphile et le support sont liés par une liaison covalente entre le(s) groupement(s) fonetionnel(s) réactif(s) de la molécule amphiphile et le(s) groupement(s) fonctionnel(s) réactif (s) fixé(s) sur le support, il est particulièrement avantageux d'utiliser comme molécule amphiphile un polymère vinylique de formule (1) ou (II) telles que définies précédemment, de façon générale ou tel que dans les modes de réalisations avantageux des polymères de formule (I) ou (II) précédemment décrits, qui comprenne en outre un pourcentage compris entre 0 et 4% d'un monomère de formule :

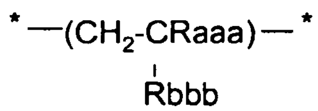

(IV)

,

dans laquelle :

Raaa est l'atome d'hydrogène ou le radical méthyle ;

Rbbb représente un groupe COORccc, ou -COSRccc ou -CORccc ou CONRcccRddd, où

Rccc représente le groupement fonctionnel réactif de la molécule amphiphile constituant la première moitié d'un couple réactif chimique, et

Rdd représente un radical ($C_1$-$C_5$) alkyle, un alkylsulfonate, l'atome d'hydrogène, un reste sucre, un hydroxyalkyl -$(CH_2)$mOH primaire, secondaire ou tertiaire, avec m variant de 1 à 4, un polyoxyalkylène, notamment polyoxyéthylène, ayant 4 à 10 unités d'oxyde d'alkylène, un radical zwitterionique, un radical $(CH_2)$t-NRf1Rf2, t nombre entier de 1 à 5, Rf1 Rf2 identiques ou différents étant l'atome d'hydrogène ou un radical ($C_1$-$C_4$) alkyle.

Dans ces kits, le couple (groupement fonctionnel réactif de la molécule amphiphile/groupement fonctionnel réactif fixé sur le support) est avantageusement choisi parmi ceux décrits précédemment.

L'invention concerne en outre l'utilisation d'une molécule amphiphile pour complexer une protéine membranaire et la fixer sur un support. Ladite molécule amphiphile, ladite protéine membranaire et ledit support peuvent être quelconques parmi l'ensemble des molécules amphiphiles, protéines membranaires et supports décrits précédemment.

Enfin, l'invention concerne également une molécule amphiphile quelconque telle que définie précédemment, comprenant en outre au moins un groupement fonctionnel constituant la première moitié d'un couple de molécules récepteur-ligand ou la première moitié d'un couple réactif chimique.

Dans un premier mode de réalisation, la molécule amphiphile comprend en outre au moins un groupement fonctionnel constituant la première moitié d'un couple de molécules récepteur-ligand. Une telle molécule amphiphile fonctionnalisée permet de fixer une protéine membranaire quelconque sur un support comprenant au moins un groupement fonctionnel fixé d'une façon quelconque à sa surface constituant la deuxième moitié dudit couple de molécules récepteur-ligand par une liaison spécifique de type récepteur-ligand entre le(s) groupement(s) fonctionnel(s) de la molécule amphiphile et le(s) groupement(s) fonctionnel(s) fixé(s) sur le support. Avantageusement, le(s) groupement (s) fonctionnel(s) de la molécule amphiphile sont choisis parmi la biotine, l'avidine, le glutathion, la glutathion S-transférase, la calmoduline, une ATPase, une protéine kinase, une phosphodiestérase, un neurotransmetteur, la L-arginine, la *p*-aminobenzamidine, une protéase à sérine, la L-lysine, le plasminogène (et activateur), un ARNr, l'AMP, l'ADP, l'ATP, une enzyme à cofacteurs, une lectine, une protéine glucannée, un glucolipide, un polysaccharide, l'héparine, un facteur de croissance et de coagulation, un récepteur stéroïdien, une endonucléase, une lipoprotéine, une lipase, du Cibacron blue®, une enzyme à cofacteurs NAD ou NADP, l'albumine, un facteur de coagulation, un interféron, un antigène, un haptène, un anticorps, l'acide nitrilotriacétique (NTA), l'EDTA, l'acide phénylboronique (APB), l'acide salicylhydroxamique (ASH), un oligonucléotide, un groupement cyclopentadienyle, un groupement alkynyle ou un groupement alkoxylamine. De préférence, le(s) groupement(s) fonctionnel(s) de la molécule amphiphile sont choisis parmi la biotine, l'avidine, le glutathion, la glutathion S-transférase, un antigène, un haptène, un anticorps, l'acide nitrilotriacétique (NTA), l'EDTA ou un oligonucléotide.

En outre, la molécule amphiphile est avantageusement un polymère de formule (I) ou (II) comprenant en outre un pourcentage compris entre 0 et 4% d'un monomère de formule

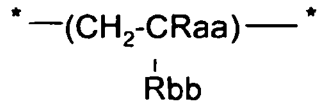 (III)

,

dans laquelle Raa et Rbb sont tels que définis précédemment.

Dans un deuxième mode de réalisation, la molécule amphiphile comprend en outre au moins un groupement fonctionnel constituant la première moitié d'un couple réactif chimique. Une telle molécule amphiphile fonctionnalisée permet de fixer une protéine membranaire quelconque sur un support comprenant au moins un groupement fonctionnel réactif fixé d'une façon quelconque à sa surface constituant la deuxième moitié dudit couple réactif chimique par une liaison covalente entre le(s) groupement(s) fonctionnel(s) réactif(s) de la molécule amphiphile et le(s) groupement(s) fonctionnel(s) réactif(s) fixé(s) sur le support. Avantageusement, le(s) groupement(s) fonctionnel(s) réactif(s) de la molécule amphiphile sont choisis parmi ceux décrits précédemment.

En outre, la molécule amphiphile est avantageusement un polymère de formule (I) ou (II) comprenant en outre un pourcentage compris entre 0 et 4% d'un monomère de formule

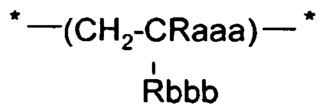 (IV)

,

dans laquelle Raaa et Rbbb sont tels que définis précédemment.

Une autre application des produits selon l'invention comprenant un support et au moins une protéine membranaire fixée à sa surface par l'intermédiaire d'une molécule amphiphile avec laquelle ladite protéine membranaire est complexée tels que décrit précédemment est d'utiliser un tel produit comme étape transitoire dans un procédé d'analyse des protéines membranaires présentes dans un échantillon par spectrométrie de masse.

En effet, la spectrométrie de masse, technique très importante en protéomique pour l'identification des protéines, est rendue beaucoup plus difficile, dans le cas des protéines membranaires, par la présence de détergents, qui interfèrent avec la détection des protéines ou des peptides qui en dérivent après protéolyse ménagée. L'immobilisation par des molécules amphiphiles tel que décrit précédemment fournit un moyen très élégant de contourner ce problème en supprimant les détergents tout en permettant une protéolyse partielle des protéines membranaires immobilisées sur un support par l'intermédiaire d'une molécule amphiphile (voir Figure 8).

La présente invention a donc également pour objet un procédé d'analyse des protéines membranaires présentes dans un échantillon, comprenant la préparation d'un produit selon l'invention comprenant un support et au moins une protéine membranaire fixée à sa surface par l'intermédiaire d'une molécule amphiphile avec laquelle ladite protéine membranaire est complexée tel que décrit précédemment. Dans un mode de réalisation préféré, le procédé comprend les étapes consistant à :

a) solubiliser les protéines membranaires en détergent,

b) complexer les protéines membranaires avec une molécule amphiphile telle que décrite précédemment et éliminer le détergent,

c) immobiliser les protéines membranaires sur un support tel que décrit précédemment par l'intermédiaire de la molécule amphiphile,

d) laver extensivement et ajouter une protéase pour générer des fragments protéiques des protéines membranaires, le domaine transmembranaire restant complexé la molécule amphiphile fixée au support,

e) éliminer le support auquel reste attaché le domaine transmembranaire restant complexé la molécule amphiphile et analyser les fragments protéiques par spectrométrie de masse.

Les différents supports, molécules amphiphiles et types de liaisons entre la molécule amphiphile et le support

décrits précédemment sont utilisables dans cette application de l'invention.

Dans un exemple avantageux, le support peut notamment être constitué de billes magnétiques, qui peuvent être facilement séparées à l'étape e) à l'aide d'un aimant.

Une molécule amphiphile avantageuse peut notamment être un polymère vinylique quelconque tel que décrit précédemment.

La molécule amphiphile peut par exemple être biotinylée et le support revêtu d'avidine pour la liaison entre la molécule amphiphile et le support.

Ces différents modes de réalisation préférés peuvent bien entendu être combinés.

## DESCRIPTION DES FIGURES

**Figure 1. Amphipols et complexes protéine membranaire/amphipols. A,** structure chimique d'un amphipol (APol). Une molécule d'amphipol A8-35 a un poids moléculaire moyen d'environ 10 kDa. Elle contient environ 18 chaînes octyles, qui lui confèrent son hydrophobie et son affinité très élevée pour la surface de la protéine. **B,** modélisation du complexe formé par l'association d'amphipols avec le cytochrome $bc_1$, complexe protéique membranaire de 500 kDa. La couronne d'amphipol qui s'est associée à la protéine contient environ 8 molécules d'A8-35 (8).

**Figure 2. Principe de la méthode développée.** Etape *a*. La protéine (rectangle gris clair) est solubilisée en détergent. Etape *b*. La protéine est piégée par un amphipol biotinylé (BAPol), et le détergent est éliminé. Etape *c.* Le complexe protéine/BAPol interagit par le biais d'un couplage biotine/avidine avec un support solide greffé d'avidine. Etape *d*. Un ligand (losange gris sombre) reconnaît la protéine cible.

**Figure 3. structures chimiques de l'amphipol universel (UAPol) et de l'amphipol biotinylé (BAPol).** Détail de la réaction de biotinylation.

**Figure 4. Comparaison de l'adsorption d'amphipols - l'un biotinylé (BAPol), l'autre non (HApol) sur une puce porteuse d'avidine, suivie par résonance de plasmon de surface (RPS).** Enregistrements obtenus au cours d'injections sur deux canaux distincts de 50 μl d'HAPol (gris pointillé) ou de BAPol (noir) dilué à 10 μM dans un tampon NaCl-HEPES (NaCl 150 mM, HEPES 10 mM, pH = 7,4). Ce même tampon circule en continu sur la puce en dehors des périodes d'injection, à 10 μl/min. La différence d'amplitude des plateaux observés après injection, matérialisés par les flèches verticales, reflète le fait que l'adhésion des polymères sur la puce est médiée par la biotine.

**Figure 5. Adhésion de complexes protéine membranaire/amphipol sur puce porteuse d'avidine suivie par RPS.** Enregistrements obtenus au cours d'injection de 100 μl de solutions de BAPol, tOmpA/BAPol, BR/BAPol, $b_6f$/BApol et $bc_1$/BAPol à une concentration fixe en BAPol de 30 μM sur différents canaux d'une puce porteuse d'avidine. Tampon circulant : NaCl-HEPES.

**Figure 6. Détection par résonance plasmonique de surface (RPS) de la liaison d'anticorps sur des protéines membranaires immobilisées à la surface d'une puce porteuse d'avidine par l'intermédiaire d'un amphipol biotinylé.** On injecte successivement 10 μl de sérums purifiés pré-immun (pré-i.), puis post-immuns dirigés contre tOmpA (Post-i.-OA), contre la BR (Post-i.-BR), contre le $b_6f$ (Post-i.-b6f), puis contre le $bc_1$ (Post-i.-bc1), sur des canaux où ont été préalablement déposés du BAPol ou des protéines piégées en BAPol. Les injections sont indiquées par des flèches. Chaque enregistrement est obtenu sur un canal où a été déposé un échantillon différent : l'enregistrement en trait noir épais pour le canal où sont immobilisés les complexes tOmpA/BAPol, noir fin petit pointillé pour le canal BR/BAPol, noir fin grand pointillé pour le canal $b_6f$/BAPol, noir fin pour le canal $bc_1$/BAPol, et gris épais pour le canal de l'échantillon témoin BAPol seul. Tampon circulant : NaCl-HEPES.

**Figure 7. Détection par RPS de la liaison d'anticorps sur une protéine déposée à la surface d'une puce porteuse d'avidine après piégeage en HAPol ou en BAPol.** Enregistrements obtenus lors d'une injection de sérum post-immun dirigé contre la BR sur des canaux sur lesquels a été déposée la BR après piégeage soit par de l'HAPol (gris pointillé), soit par du BAPol (noir). Tampon circulant : NaCl-HEPES. L'expérience met en évidence l'accroissement du signal lorsque l'adsorption est médiée par un amphipol fonctionnalisé.

**Figure 8. Utilisation d'un produit selon l'invention pour analyser les protéine membranaires d'un échantillon par spectrométrie de masse.** Etape (*a)*. La protéine (rectangle gris clair) est solubilisée en détergent. Etape (*b)*.

La protéine est piégée (ou complexée) par un amphipol biotinylé (BAPol), et le détergent est éliminé. Etape (*c)*. Le complexe protéine membranaire/BAPol interagit par le biais d'un couplage biotine/avidine avec un support solide greffé d'avidine. Etape (*d)*. Après un lavage extensif, une protéase est ajoutée pour réaliser une protéolyse ménagée et générer des fragments de protéine membranaire, la partie transmembranaire restant protégée par l'amphipol avec lequel elle est complexée. Etape (*e)*. Après séparation des fragments protéiques du support sur lequel sont encore liés les parties transmembranaires complexées avec l'amphipol biotinylé BAPol (par exemple à l'aide d'un aimant en cas de billes magnétiques), les fragments sont analysés par spectrométrie de masse.

**Figure 9. Immobilisation de la protéine membranaire bactériorhodopsine (BR) sur des billes magnétiques porteuses de streptavidine.** L'absorbance (ou densité optique) de la solution de protéine BR complexée avec l'amphipol biotinylé (BAPol) à temps 0 (avant mélange avec les billes) et après incubation pendant 1h45 avec les billes porteuses de streptavidine est mesurée en fonction de la longueur d'onde. La présence d'un pic caractéristique de la protéine BR à 560 nm est analysée.

**Figure 10. Schéma de synthèse d'un amphipol porteur d'une étiquette polyhistidine.** DCC : dicyclohexyle carbodiimide, NMP : N-méthylpyrrolidinone, HOBT : N-hydroxybenzotriazole, iPr : isopropyle, FMOC : 9-fluorénylméthoxycarbonyle, HIS : histidine, TFA : acide trifluoroacétique, HBTU : O-Benzotriazole-N,N,N',N'-tetramethyluronium-hexafluoro-phosphate, Tr : trityle.

## EXEMPLES

EXEMPLE 1. ***Immobilisation de protéines membranaires à la surface de puces porteuses de streptavidine à l'aide d'un amphipol biotinylé***

### 1.1 Principe

Le principe expérimental développé pour immobiliser les protéines membranaires à la surface de supports solides est le suivant (Figure 2) : la protéine membranaire est solubilisée en détergent (étape *a*) puis piégée par un amphipol biotinylé et débarrassée du détergent (étape *b*) ; le complexe formé est mis en contact avec un support greffé de groupements streptavidine auquel il peut s'associer par le biais d'une interaction spécifique biotine/streptavidine (étape c). On peut alors faire circuler un ligand de la protéine sur le support et observer l'interaction ligand/protéine (étape *d*).

Le suivi expérimental de l'immobilisation a été effectué par la technique de résonance plasmonique de surface (RPS). Cette technique permet la détection et le suivi en temps réel des interactions entre des molécules circulantes et une ou des molécule(s) immobilisée(s), grâce à l'observation en continu d'une modification de résonance de plasmon de surface induite par l'interaction des molécules circulantes avec le support d'analyse. Le résultat d'une expérience de RPS permet de suivre au cours du temps la quantité de matériel interagissant avec le support.

### 1.2 Synthèse d'un amphipol biotinylé (BApol) et comportement en solution

1.2.1 Synthèse d'un amphipol biotinylé (BApol)

La structure du BAPol est décrite dans la Figure 3.

Le protocole de synthèse du BAPol est dérivé de celui des amphipols non fonctionnalisés (7, 16-17). Il consiste à former des liaison amides avec les fonctions acide carboxylique d'un acide polyacrylique de bas poids moléculaire et des alkylamines judicieusement choisies, l'isopropylamine et l'octylamine, ainsi que, dans ce cas précis, une faible proportion d'éthylènediamine sélectivement monoprotégée. Après déprotection de la fonction amine non liée au polymère, on obtient un amphipol fonctionnalisé (amphipol "universel, ou "UAPol" ; Fig. 2), que l'on peut ensuite faire réagir sur n'importe quel substrat suffisamment activé. Ainsi, le BAPol est obtenu par réaction de la fonction amine de l'UAPol sur le succinimidyl ester de la D-biotine (Fig. 3).

1.2.2 Analyse

Les BAPols présentent des caractéristiques en solution identiques à celle des APols non biotinylés. Ils forment en particulier des particules de même taille et de même dispersité.

De plus, les protéines membranaires piégées par les BAPols sont correctement maintenues en solution (elles n'agrègent pas en l'absence de détergent), de la même façon qu'avec les amphipols non biotinylés.

En résumé, malgré le greffage d'une molécule de biotine, les BAPols présentent les mêmes caractéristiques

physico-chimiques et biochimiques que les APols dépourvus de biotine, excepté pour leur capacité à se lier spécifiquement à l'avidine ou streptavidine. Ils permettent donc également de complexer les protéines membranaires et de les rendre hydrosolubles en absence de détergent, puis de les associer à un support.

**1.3 Interaction des amphipols avec le support greffé de groupements streptavidine**

Des solutions d'amphipol non-biotinylé (HAPol) et d'amphipol biotinylé (BAPol) ont été injectées séparément sur deux canaux distincts du support greffé de groupements streptavidine (support SA).

Les résultats obtenus montrent que les deux polymères adhèrent au support SA après injection (Figure 4), mais que la masse de BAPol liée est environ trois fois plus élevée que celle de HAPol.

Seul le greffage de la biotine différenciant les deux polymères, cette expérience indique que c'est bien la présence de cette dernière qui permet une adsorption importante d'amphipol sur la puce.

**1.4 Dépôt de protéines membranaires piégées en BAPol et test de leur reconnaissance par des ligands**

Quatre protéines membranaires modèles dont la biochimie est bien connue et dont le comportement en APol a déjà été étudié ont été utilisées pour tester le procédé d'immobilisation de protéines membranaires selon l'invention : le domaine transmembranaire de la protéine OmpA (tOmpA), la bactériorhodopsine (BR), le cytochrome $b_6f$($b_6f$) et le cytochrome $bc_1$ ($bc_1$).

De plus, des sérums de lapin contenant des anticorps dirigés contre chacune de ces quatre protéines ont été préparés pour servir de ligands dans le suivi des interactions protéines membranaires/ligands par RPS.

1.4.1 Protocole de fixation des protéines membranaires sur le support SA

Les quatre protéines ont été piégées en BAPol :

- tOmpA est disponible en solution à une concentration de 1,2 g/l en présence de détergent octyltetraoxyéthylène ($C_8E_4$) à 6 g/l. Le piégeage est effectué en ajoutant 18 µl d'une solution de BAPol à 100 g/l dans l'eau à 500 µl de solution de protéine (soit 4 g de BAPol par g de tOmpA). Après une incubation de 15 min, 30 mg de Bio-beads sont ajoutés et on laisse incuber sous agitation pendant 3 h à 4°C avant de récupérer la solution et enlever les Bio-beads.

- La solution de BR en détergent octyl thio-glucoside (OTG) est à 1 g/l de protéine, 20 mM d'OTG. On ajoute 23 µl de solution à 100 g/l de BAPol dans l'eau à 450 µl de solution de protéine (soit 5 g de BAPol par g de BR). Après une incubation de 15 min, 30 mg de Bio-beads sont ajoutés et on laisse incuber sous agitation pendant 3 h à 4°C avant de récupérer la solution et enlever les Bio-beads.

- La solution de $b_6f$ en détergent lauryl maltoside (LM) est à 0,27 g/l de protéine et 0,1 g/l LM. On ajoute 1,2 µl de solution à 100 g/l de BAPol dans l'eau à 150 µl de solution de protéine (soit 3 g de BAPol par g de $b_6f$). Après une incubation de 15 min, 140 mg de Bio-beads sont ajoutés et on laisse incuber sous agitation pendant 3 h à 4°C avant de récupérer la solution et enlever les Bio-beads.

- La solution de $bc_1$ en LM est à 35 g/l de protéine et 0.1 g/l de LM. On ajoute 119 µl de solution de BAPol à 100 g/l dans l'eau à 225 µl de solution de protéine (soit 1,5 g de BAPol par g de $bc_1$). Après une incubation de 15 min, 90 mg de Bio-beads sont ajoutés et on laisse incuber sous agitation pendant 3 h à 4°C avant de récupérer la solution et enlever les Bio-beads.

Ensuite, les complexes protéines/BAPol ainsi formés et le BAPol seul ont été déposés sur des canaux distincts d'un support SA : toutes les solutions sont ajustées à une concentration de 30 µM de BAPol en les diluant avec du tampon NaCl-HEPES

(NaCl 150 mM, HEPES 10 mM à pH 7,4), puis sont injectées à raison de 100 µl chacune sur un appareil de type Biacore 2000 de manière à faire passer chaque solution sur des canaux distincts de supports SA. Entre les injections, on fait circuler le tampon NaCl-HEPES. Le flux de circulation est fixé à 10 µl/min pendant et hors des injections.

L'expérience montre qu'il y a fixation effective et irréversible de matériel sur les supports (Figure 5).

1.4.2 Analyse de la liaison d'un lipand sur les protéines membranaires immobilisées sur le support SA par le BApol

Les expériences de reconnaissance anticorps/matériel immobilisé ont ensuite été conduites avec des sérums purifiés dont seuls les anticorps, quel qu'ils soient, ont été conservés, dilué 100 fois dans le tampon NaCl-HEPES.

Brièvement, on injecte successivement 10 μl de sérums purifiés, pré-immun (pré-i.) puis post-immun dirigés contre tOmpA (Post-i.-OA), contre la BR (Post-i.-BR), contre le $b_6f$ (Post-i.-b6f) puis contre le $bc_1$ (Post-i.-bc1) sur des canaux où ont été préalablement déposés du BAPol ou des protéines piégées en BAPol. Les injections durent 60 s et leur temps zéro est indiqué par une flèche. En dehors des injections, le tampon NaCl-HEPES (NaCl 150mM, HEPES 10mM pH=7,4) circule sur le support. Chaque enregistrement est obtenu sur un canal où a été déposé un échantillon différent.

Toutes les réponses observées sont spécifiques (voir Figure 6) : globalement, les résultats montrent que le signal RPS reste élevé après injection uniquement lorsque ce sont les sérums post-immuns qui sont testés et à condition qu'ils circulent sur le canal porteur de la protéine ayant servi à l'immunisation (contre laquelle des anticorps du sérum ont été produits).

Ceci signifie que les protéines membranaires ont bien été immobilisées sur les canaux des supports. Cela montre aussi que l'on peut parfaitement utiliser le procédé mis au point pour suivre des interactions entre des protéines membranaires et des ligands.

En outre, la même expérience a été effectuée avec des séra non purifiés et a mené à la même conclusion, montrant ainsi qu'un ligand spécifique peut être détecté même au sein du milieu extrêmement complexe que constitue un sérum non purifié (résultats non montrés).

**1.5 Détermination de l'agent de médiation de l'immobilisation de la protéine sur le support.**

Lors d'une expérience de contrôle, la réponse obtenue selon que la protéine-test a été déposée sur la puce après piégeage soit en HAPol (non-biotinylé), soit en BAPol (biotinylé) a été comparée, afin de vérifier dans quelle mesure la fixation de la protéine sur le support SA est bien médiée par la biotine fixée de façon covalente sur le polymère, par le biais de la liaison spécifique biotine/streptavidine.

Les résultats (voir Figure 7) montrent que l'anticorps ne se lie de manière significative, donc ne reconnaît la protéine déposée sur le support SA, que si celle-ci a été piégée en BAPol.

**1.6 Conclusion.**

Ces résultats démontrent la validité et la puissance du procédé selon l'invention pour immobiliser des protéines membranaires sur un support. En effet, ils montrent que, dérivés chimiquement de façon appropriée (ici, par un greffage de biotine, mais de multiples autres modes de dérivation sont concevables), les amphipols peuvent être utilisés facilement, sans mise au point significative, pour immobiliser une protéine membranaire quelconque à la surface d'un support solide.

En outre, les résultats obtenus indiquent que les protéines ainsi immobilisées peuvent être utilisées pour étudier des interactions avec des ligands (ici, pour détecter des anticorps circulants). Les produits selon l'invention comprenant un support et une ou plusieurs protéines membranaires fixées à sa surface peuvent donc être utilisés pour détecter la présence ou l'absence dans un échantillon biologique d'un ligand d'une protéine membranaire immobilisée à la surface du support, différents types d'applications étant alors possibles, comme par exemple :

- détection d'anticorps circulants dirigés contre un antigène membranaire, comme présenté ici, ou
- criblage d'une banque de composés à la recherche de ligands d'un récepteur membranaire d'intérêt pharmacologique, en vue d'identifier des agonistes ou antagonistes de ce récepteur.

En outre, les résultats obtenus indiquent que les protéines ainsi immobilisées peuvent être utilisées pour la réalisation de réacteurs enzymatiques : les produits selon l'invention comprenant un support (billes, membranes, fibres, nanotubes etc.) et une ou plusieurs protéines membranaires fixées à sa surface peuvent en effet être utilisés pour exposer aux dites protéines des produits circulant dans la solution, sur lesquels les dites protéines agiront enzymatiquemcnt

***EXEMPLE 2. Immobilisation de protéines membranaires à la surface de billes magnétiques porteuses de streptavidine à l'aide d'un amphipol biotinylé***

Les inventeurs ont testé l'immobilisation de la bactériorhodopsine (BR) sur des billes magnétiques fonctionnalisées par liaison de streptavidine (billes SA).

La BR a été complexée à l'amphipol biotinylé (BAPol) selon le protocole suivant : une solution de BR en détergent octyl thio-glucoside (OTG) est à 1,1 g/l de protéine, 18 mM d'OTG. On ajoute 17 μl de solution à 100 g/l de BAPol dans l'eau à 300 μl de solution de protéine (soit 5 g de BAPol par g de BR). Après une incubation de 15 min, 80 mg de Bio-beads sont ajoutés et on laisse incuber sous agitation pendant 3 h à 4°C (adsorption de l'OTG) avant de

récupérer la solution et enlever les Bio-beads.

100 mg de billes SA ont été lavées par 3 fois dans le tampon NaCl-HEPES (NaCl 150 mM, HEPES 10 mM à pH 7,4), puis le liquide en excès retiré ; la solution de protéine a été diluée à 13 mg/L dans du tampon NaCl-HEPES.

Au temps zéro, la solution de protéine est ajoutée aux billes et l'ensemble mis à agiter sur un Vortex à 4˚C. L'échantillon est récupéré 1 h 45 plus tard. Les billes sont séparées grâce à un aimant, et le surnageant est analysé. Le suivi de l'immobilisation de la BR est fait par mesure de la densité optique de la solution avant et après incubation en présence des billes.

Les résultats sont présentés sur la Figure 9 et montrent que le pic caractéristique de la BR à 560 nm a disparu après l'incubation, indiquant la fixation de la BR sur les billes magnétiques SA.

Ces résultats démontrent qu'il est également possible d'immobiliser des protéines membranaires sur des billes.

### *EXEMPLE 3. Synthèse d'un amphipol porteur d'une étiquette polyhistidine (HISTAPol)*

La structure de l'amphipol biotinylé (BAPol) est décrite dans la Figure 3.

Le protocole de synthèse de l'amphipol porteur d'une étiquette polyhistidine (HISTAPol) est dérivé de celui des amphipols non fonctionnalisés (7, 16-17) et comporte trois étapes.

La première étape consiste à former des liaisons amides avec les fonctions acide carboxylique d'un acide polyacrylique de bas poids moléculaire et des alkylamines judicieusement choisies, l'isopropylamine et l'octylamine.

La deuxième étape consiste en l'élaboration d'un hexamère de l'histidine. Cette synthèse est effectuée sur support solide en utilisant une technique automatisée de double couplage du monomère sélectivement protégé.

La dernière étape est la condensation du N-terminus du peptide sur les fonctions acide de l'amphipol obtenu lors de la première étape de la synthèse. Le peptide est utilisé encore protégé sur ses chaînes latérales et fixé au support solide. Les proportions d'amphipols et de peptide utilisées sont calculées pour ne pas excéder 2 % de greffage d'étiquette poly(histidine). Après déprotection et décrochage du peptide du support solide, l'HISTAPol est purifié par voie classique. La synthèse est décrite dans la Figure 10.

Une telle synthèse peut être réalisée avec un autre type d'amphipol ou de molécule amphiphile. De plus, d'autres procédés de synthèse peuvent également être employés pour synthétiser des molécules amphiphiles, et notamment des amphipols, porteurs d'une étiquette poly(histidine).

De tels amphipols fonctionnalisés permettent de fixer des protéines membranaires complexées avec ces amphipols sur des supports porteurs de groupes Ni-NTA.

### BIBLIOGRAPHIE

1. Giess F. et coll. (2004). The protein-tethered lipid bilayer: a novel mimic of the biological membrane. Biophys. J. 87(5), 3213-20.

2. Pal P. et coll. (2005). A novel immobilisation method for single protein spFRER studies. Biophys. J. 89(2), L11-3

3. Hoffman T.L. et coll. (2000) A biosensor assay for studying ligand-membrane receptor interactions : binding of antibodies and HIV-1 Env to chemokine receptors. PNAS, 97, 11215-11220.

4. Minic J. et coll. (2005). Immobilization of native membrane-bound rhodopsin on biosensor surfaces. Biochim. Biophys. Acta, 1724(3), 324-32 5. Cooper M.A (2002) Optical biosensor in drug discovery. Nat. rev. Drug Discov. 1,515-528

6. EP0946875

7. Tribet, C., Audebert, R. & Popot, J.-L. (1996). Amphipols: polymers that keep membrane proteins soluble in aqueous solutions. Proc. Natl. Acad. Sci. USA 93, 15047-15050.

8. Popot, J.-L., Berry, E. A., Charvolin, D., Creuzenet, C., Ebel, C., Engelman, D. M., Flötenmeyer, M., Giusti, F., Gohon, Y., Hervé, P., Hong, Q., Lakey, J. H., Leonard, K., Shuman, H. A., Timmins, P., Warschawski, D. E., Zito, F., Zoonens, M., Pucci, B. & Tribet, C. (2003). Amphipols: polymeric surfactants for membrane biology research. Cell. Mol. Life Sci. 60, 1559-1574.

9. Zoonens, M., Catoire, L. J., Giusti, F. & Popot, J.-L. (2005). NMR study of a membrane protein in detergent-free

aqueous solution. Proc. Natl. Acad. Sci. USA 102, 8893-8898.

10. Picard, M., Dahmane, T., Garrigos, M., Gauron, C., Giusti, F., le Maire, M., Popot, J.-L. & Champeil, P. (2006). Protective and inhibitory effects of various types of amphipols on the Ca2+-ATPase from sarcoplasmic reticulum: a comparative study. Biochemistry 45, 1861-1869.

11. Nagy, J. K., Kuhn Hoffmann, A., Keyes, M. H., Gray, D. N., Oxenoid, K. & Sanders, C. R. (2001). Use of amphipathic polymers to deliver a membrane protein to lipid bilayers. FEBS Lett. 501, 115-120.

12. Q. Xu, K. S. Lam, Protein and Chemical Microarrays_Powerful Tools for Proteomics, J. Biomed. Biotechnol., 2003, 257, 2003.

13. M. N. Yousaf, M. Mrksich, Diels-Aldert Reaction for the Selective Imobilization of Proteine to Electroactive Self-Assembled Monolayers, J. Am. Chem. Soc., 121,4286,1999.

14. N. K. Devaraj, G. P. Miller, W. Ebina, B. Kakaradov, J. P. Collman, E. T. Kool, C. E. D. Chidsey, Chemoselective Covalent Coupling of Oligonucleotide Probes to Self-Assembled Monolayers, J. Am. Chem. Soc., 127, 6800, 2005.

15. I. Taniguchi, A. M. Mayes, E. W. L. Chan, L. G. Griffith, A Chemoselective Approach to Grafting Biodegradable Polyesters, Macromolecules, 38, 216-219, 2005.

16. Gohon, Y., Pavlov, G., Timmins, P., Tribet, C., Popot, J.-L. & Ebel, C. (2004). Partial specific volume and solvent interactions of amphipol A8-35. Anal. Biochem. 334, 318-334.

17. Gohon, Y., Giusti, F., Prata, C., Charvolin, D., Timmins, P., Ebel, C., Tribet, C. & Popot, J.-L. (2006). Well-defined nanoparticles formed by hydrophobic assembly of a short and polydisperse random terpolymer, amphipol A8-35. Langmuir 22, 1281-1290.

18. Chemical Abstract 114:224839 (June 15, 1991)

19. J. Chrom. B. 703 (1997), pages 53-62.

## Revendications

**1.** Produit comprenant un support et au moins une protéine membranaire fixée à sa surface, **caractérisé en ce que** ladite protéine membranaire est fixée sur ledit support par l'intermédiaire d'une molécule amphiphile avec laquelle ladite protéine membranaire est complexée, la molécule amphiphile étant choisie parmi un polymère vinylique amphiphile ou un polypeptide amphiphile, et ladite au moins une protéine membranaire étant avantageusement choisie parmi un antigène, un anticorps, une enzyme, un récepteur cellulaire, un canal ionique, ou une protéine membranaire d'origine virale, bactérienne, ou eucaryote.

**2.** Produit selon la revendication 1, **caractérisé en ce que** la molécule amphiphile est un polymère vinylique de formule (I) :

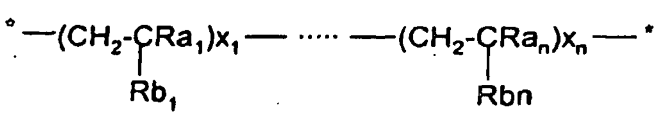 (I)

dans laquelle :

$Ra_1$ à $Ra_n$ identiques ou différents sont l'atome d'hydrogène, ou le radical méthyle :
$Rb_1$ à $Rb_n$ sont différents et choisis parmi :

- un groupe hydrophile choisi parmi

○ un radical carboxylate $-COO^- M^+$, un radical sulfonate $-SO_3^-M^+$, ou un radical phosphonate $-PO_3^-M^+$, $M^+$ étant un contre-ion cationique,
○ un radical ($C_1$-$C_5$) alkylcarboxylate, un radical ($C_1$-$C_5$) alkylsulfonate, ou un radical ($C_1$-$C_5$) alkylphosphonate
○ un phényl sulfonate

- CONRc1Rc2, Rc1 et Rc2, identiques ou différents étant un radical (-C($CH_2$ORd1)($CH_2$ORd2)($CH_2$ORd3)) avec Rd1, Rd2, Rd3 représentant indépendamment l'atome d'hydrogène, un reste sucre, polyoxyalkylène, notamment polyoxyéthylène, ayant de 4 à 10 unités d'oxyde d'alkylène, un radical zwitterionique, un hydroxyalkyl $-(CH_2)mOH$ primaire, secondaire ou tertiaire, avec m variant de 1 à 4, un radical ($C_1$-$C_5$) alkylcarboxylate, un radical ($C_1$-$C_5$) alkylsulfonate ou un radical ($C_1$-$C_5$) alkylphosphonate, un reste sucre
- COORe, Re étant un reste sucre, un hydroxyalkyl $-(CH_2)mOH$ primaire, secondaire ou tertiaire, avec m variant de 1 à 4, un polyoxyalkylène, notamment polyoxyéthylène, ayant 4 à 10 unités d'oxyde d'alkylène, un radical $(CH_2)$t-NRf1Rf2, t nombre entier de 1 à 5, Rf1, Rf2 identiques ou différents, étant l'atome d'hydrogène ou un radical ($C_1$-$C_4$) alkyle,
- un hydroxyle
- un hydroxyalkyl $-(CH_2)mOH$ primaire, secondaire ou tertiaire, avec m variant de 1 à 4,
- une amine primaire, secondaire, tertiaire
- un ammonium quaternaire
- N-formamide, N-alkylformamide,
- N-acétamide, N-alkylacétamide,
- N-pyrrolidonyle,
- CONRg1Rg2, Rg1 et Rg2, identiques ou différents étant l'atome d'hydrogène, un reste sucre, polyoxyalkylène, notamment polyoxyéthylène, ayant de 4 à 10 unités d'oxyde d'alkylène, un radical zwitterionique, un hydroxyalkyl $-(CH_2)mOH$ primaire, secondaire ou tertiaire, avec m variant de 1 à 4,
- COORh ou CONRkRl, Rh étant un radical ($C_1$-$C_5$) alkyle, un alkylsulfonate, ou ayant l'une des significations de Re ou de Rg1 à l'exception d'un atome d'hydrogène, et Rk et Rl ayant indépendamment l'une des significations de Rh et en plus l'un des deux pouvant correspondre à l'atome d'hydrogène ;

- un groupe hydrophobe choisi parmi :

- un atome d'hydrogène,
- un atome d'halogène,
- un radical -CONH(-C($CH_2$ORm1)($CH_2$ORm2)($CH_2$ORm3)) avec Rm1, Rm2, Rm3 étant indépendamment un alkyle, un alcényle ou alcynyle linéaire ou ramifié de 3 à 50 atomes de carbone, un carbamoyle d'alkyle (O=C-NH-Rn) ou un acyle (O=C-Ro) avec Rn et Ro étant des radicaux alkyle, alcényle ou alcynyle linéaire ou ramifié de 3 à 50 atomes de carbone,
- COORp, CORp, COSRp, C-NH-Rp ou CONRq1Rq2, où Rp est un radical alkyle, alcynyle ou alcényle linéaire ou ramifié et/ou cyclique comprenant de 3 à 50 atomes de carbone, et Rq1 et Rq2 identiques ou différents ont l'une des significations de Rp, et de plus l'un des deux peut correspondre à l'atome d'hydrogène,
- un radical -Rr, -ORr, ou -SRr où Rr représente un groupe alkyle, alcényle ou alcynyle linéaire ou ramifié et/ou cyclique comprenant de 3 à 50 atomes de carbone ; ou

- un groupe amphiphile choisi parmi :

- un radical alkyl $-(CH_2)$m-Rs avec m compris entre 6 et 20, Rs signifiant un groupement hydrophile de type carboxylate, sulfonate, phosphonate, sulfate, phosphate, zwitterion, ammonium, poly(oxyéthylène), sucre,
- un radical poly(oxyethylene)-O-alkyl ($-(CH_2CH_2O)$m-Rt) avec Rt un radical alkyle, alcényle, alcynyle linéaire, ramifié ou cyclique de 6 à 20 atomes de carbone,
- un radical COORu, CORu, COSRu, CONRvRw, Ru signifiant un radical poly(oxyethylene)-O-alkyl ($-(CH_2CH_2O)$m-Rt) avec Rt un radical alkyle, alcényle, alcynyle linéaire, ramifié ou cyclique de 6 à 20 atomes de carbone, un radical glycosylalkyl, Rv pouvant signifier l'atome d'hydrogène ou ayant une des significations de Ru, Rw ayant une des significations de Ru,

• un radical $-CONH(-C(CH_2ORx1)(CH_2ORx2)(CH_2ORx3))$,
avec Rx1, Rx2, Rx3 étant tels qu'un ou deux de ces groupements aient une des significations de Rm1, Rm2, Rm3 et qu'un ou deux de ces groupements soient différents de l'atome d'hydrogène et aient une des significations de Rd1, Rd2, Rd3, Ru,
ou bien Rx1, Rx2, Rx3 différents ou identiques et tels qu'au moins l'un des groupement soit différents de l'atome d'hydrogène et ayant une des significations de Ru,

n est un nombre entier supérieur ou égal à 2, de préférence compris entre 2 et 10, entre 2 et 8, entre 2 et 6, entre 2 et 4, avantageusement n égal 3 ;

$x_1$ à $x_n$ correspondent aux pourcentages respectifs des motifs $(\sum_{i=1}^{n} x_i = 100\%)$, où le rapport du pourcentage total de groupes où $Rb_i$ est un groupe hydrophobe ou amphiphile au pourcentage total de groupes où $Rb_i$ est un groupe hydrophile $\sum_{Rb_i hydrophobe} x_i + \sum_{Rb_j amphiphile} x_j \Big/ \sum_{Rb_k hydrophile} x_k$ est compris entre 0,25 et 2,5 ; et

la masse molaire moyenne est comprise entre 500 et 100000, avantageusement entre 500 et 50 000.

**3.** Produit selon la revendication 2, **caractérisé en ce que** n égal 3 et le polymère vinylique est de formule (II) :

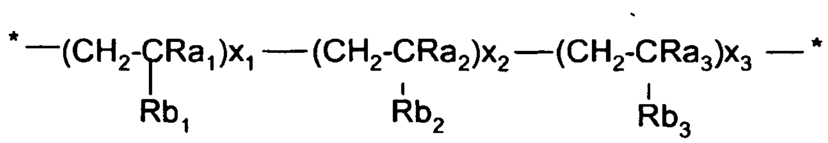 (II)

,

dans laquelle :

$Ra_1$, $Ra_2$ et $Ra_3$ identiques ou différents sont l'atome d'hydrogène, ou le radical méthyle ;
$Rb_1$ est un groupe hydrophile choisi parmi:

• un radical carboxylate $-COO^- M^+$, un radical sulfonate $-SO_3^-M^+$, ou un radical phosphonate $-PO_3^-M^+$, $M^+$ étant un contre-ion cationique,
• un radical ($C_1$-$C_5$) alkylcarboxylate, radical ($C_1$-$C_5$) alkylsulfonate, ou un radical ($C_1$-$C_5$) alkylphosphonate
• un phényl sulfonate
• CONRc1Rc2, Rc1 et Rc2, identiques ou différents étant un radical $(-C(CH_2ORd1)(CH_2ORd2)(CH_2ORd3))$ avec Rd1, Rd2, Rd3 représentant indépendamment l'atome d'hydrogène, un reste sucre, polyoxyalkylène, notamment polyoxyéthylène, ayant de 4 à 10 unités d'oxyde d'alkylène, un radical zwitterionique, un hydroxyalkyl $-(CH_2)mOH$ primaire, secondaire ou tertiaire, avec m variant de 1 à 4, un radical ($C_1$-$C_5$) alkylcarboxylate, un radical ($C_1$-$C_5$) alkylsulfonate ou un radical ($C_1$-$C_5$) alkylphosphonate, un reste sucre

Rb2 est :

- un groupe hydrophobe choisi parmi:

• un atome d'hydrogène,
• un atome d'halogène,
• un radical $-CONH(-C(CH_2ORm1)(CH_2ORm2)(CH_2ORm3))$ avec Rm1, Rm2, Rm3 étant indépendamment un alkyle, un alcényle ou alcynyle linéaire ou ramifié de 3 à 50 atomes de carbone, un carbamoyle d'alkyle (O=C-NH-Rn) ou un acyle (O=C-Ro) avec Rn et Ro étant des radicaux alkyle, alcényle ou alcynyle linéaire ou ramifié de 3 à 50 atomes de carbone,
• COORp, CORp, CSRp, C-NH-Rp ou CONRq1Rq2, où Rp est un radical alkyle, alcynyle ou alcényle linéaire ou ramifié et/ou cyclique comprenant de 3 à 50 atomes de carbone, et Rq1 et Rq2 identiques ou différents ont l'une des significations de Rp, et de plus l'un des deux peut correspondre à l'atome d'hydrogène,

• un radical -Rr, -ORr, ou -SRr où Rr représente un groupe alkyle, alcényle ou alcynyle linéaire ou ramifié et/ou cyclique comprenant de 3 à 50 atomes de carbone ; ou

- un groupe amphiphile choisi parmi:

• un radical alkyl $-(CH_2)m-Rs$ avec m compris entre 6 et 20, Rs signifiant un groupement hydrophile de type carboxylate, sulfonate, phosphonate, sulfate, phosphate, zwitterion, ammonium, poly(oxyéthylène), sucre,
• un radical poly(oxyethylene)-O-alkyl $(-(CH_2CH_2O)m-Rt)$ avec Rt un radical alkyle, alcényle, alcynyle linéaire, ramifié ou cyclique de 6 à 20 atomes de carbone,
• un radical COORu, CORu, COSRu, CONRvRw, Ru signifiant un radical poly(oxyethylene)-O-alkyl $(-(CH_2CH_2O)m-Rt)$ avec Rt un radical alkyle, alcényle, alcynyle linéaire, ramifié ou cyclique de 6 à 20 atomes de carbone, un radical glycosylalkyl, Rv pouvant signifier l'atome d'hydrogène ou ayant une des significations de Ru, Rw ayant une des significations de Ru,
• un radical $-CONH(-C(CH_2ORx1)(CH_2ORx2)(CH_2ORx3))$, avec Rx1, Rx2, Rx3 étant tels qu'un ou deux de ces groupements aient une des significations de Rm1, Rm2, Rm3 et qu'un ou deux de ces groupements soient différents de l'atome d'hydrogène et aient une des significations de Rd1, Rd2, Rd3, Ru,
ou bien Rx1, Rx2, Rx3 différents ou identiques et tels qu'au moins l'un des groupement soit différents de l'atome d'hydrogène et ayant une des significations de Ru,

$Rb_3$ est un groupe hydrophile choisi parmi:

• COORe, Re étant un reste sucre, un hydroxyalkyl $-(CH_2)mOH$ primaire, secondaire ou tertiaire, avec m variant de 1 à 4, un polyoxyalkylène, notamment polyoxyéthylène, ayant 4 à 10 unités d'oxyde d'alkylène, un radical $(CH_2)t-NRf1Rf2$, t nombre entier de 1 à 5, Rf1, Rf2 identiques ou différents, étant l'atome d'hydrogène ou un radical $(C_1-C_4)$ alkyle,
• un hydroxyle
• un hydroxyalkyl $-(CH_2)mOH$ primaire, secondaire ou tertiaire, avec m variant de 1 à 4,
• une amine primaire, secondaire, tertiaire
• un ammonium quaternaire
• N-formamide, N-alkylformamide,
• N-acétamide, N-alkylacétamide,
• N-pyrrolidonyle,
• CONRg1Rg2, Rg1 et Rg2, identiques ou différents étant l'atome d'hydrogène, un reste sucre, polyoxyalkylène, notamment polyoxyéthylène, ayant de 4 à 10 unités d'oxyde d'alkylène, un radical zwitterionique, un hydroxyalkyl $-(CH_2)mOH$ primaire, secondaire ou tertiaire, avec m variant de 1 à 4 (R3),
• COORh ou CONRkRl, Rh étant un radical $(C_1-C_5)$ alkyle, un alkylsulfonate, ou ayant l'une des significations de Re ou de Rg1 à l'exception d'un atome d'hydrogène, et Rk, Rl ayant indépendamment l'une des significations de Rh et en plus l'un des deux pouvant correspondre à l'atome d'hydrogène ;

x1, x2, x3 correspondent aux pourcentages respectifs des motifs, avec

- x1 compris entre 20 et 90 %
- x2 compris entre 10 et 80 %
- x3 compris entre 0 et 60 %, et
- $x_2/x_1 + x_3$ compris entre 0,25 et 2,5 ; et

la masse molaire moyenne étant comprise entre 500 et 100000, avantageusement entre 500 et 50 000.

**4.** Produit selon la revendication 3, **caractérisé en ce que** :

$Ra_1$, $Ra_2$ et $Ra_3$ identiques ou différents sont l'atome d'hydrogène, ou le radical méthyle ;
$Rb_1$ représente COO- $M^+$, $M^+$ étant contre-ion cationique ;
$Rb_2$ représente CONRq1Rq2, où Rq 1 et Rq2 représentent indépendamment un radical alkyle, alcynyle ou alcényle linéaire ou ramifié et/ou cyclique comprenant de 3 à 50 atomes de carbone, et de plus l'un des deux peut correspondre à l'atome d'hydrogène ;
$Rb_3$ représente CONRkRl, où Rk et Rl représentent indépendamment un radical $(C_1-C_5)$ alkyle, un alkylsulfo-

nate, un reste sucre, un hydroxyalkyl -$(CH_2)$mOH primaire, secondaire ou tertiaire, avec m variant de 1 à 4, un polyoxyalkylène, notamment polyoxyéthylène, ayant 4 à 10 unités d'oxyde d'alkylène, un radical zwitterionique, un radical $(CH_2)$t-NRf1Rf2, t nombre entier de 1 à 5, Rf1, Rf2 identiques ou différents étant l'atome d'hydrogène ou un radical $(C_1\text{-}C_4)$ alkyle, et de plus l'un des deux de Rk et Rl peut correspondre à l'atome d'hydrogène.

5. Produit selon l'une quelconque des revendications 1 à 4, **caractérisé en ce que** le support est un support solide, avantageusement le support est choisi parmi une puce, une bille, une membrane, une fibre, ou un nanotube ou le support est une macromolécule ou particule soluble choisie parmi les polymères, dendrimères, vésicules, ou micelles.

6. Produit selon l'une quelconque des revendications 1 à 5, **caractérisé en ce que** la fixation de la molécule amphiphile sur le support est médiée par une liaison hydrophobe, une liaison ionique, une liaison spécifique de type récepteur-ligand entre au moins un groupement fonctionnel de la molécule amphiphile et au moins un groupement fonctionnel à la surface du support, ou une liaison covalente entre au moins un groupement fonctionnel réactif de la molécule amphiphile et au moins un groupement fonctionnel réactif à la surface du support.

7. Produit selon la revendication 6, **caractérisé en ce que** :

a. la molécule amphiphile comprend en outre au moins un groupement fonctionnel constituant la première moitié d'un couple de molécules récepteur-ligand,
b. le support comprend en outre au moins un groupement fonctionnel fixé à sa surface constituant la deuxième moitié dudit couple de molécules récepteur-ligand, et
c. la fixation de la molécule amphiphile sur le support est médiée par une liaison spécifique de type récepteur-ligand entre le(s) groupement(s) fonctionnel(s) de la molécule amphiphile et le(s) groupement(s) fonctionnel(s) fixé(s) sur le support,

le couple récepteur-ligand (groupement fonctionnel de la molécule amphiphile / un groupement fonctionnel fixé sur le support) étant choisi parmi les couples (biotine/avidine), (glutathion/glutathion S-transférase, protéines fixant le glutathion, ou protéines de fusion incluant la glutathion S-transférase), (calmoduline/ ATPase, protéine kinase, phosphodiestérase, ou neurotransmetteur), (L-arginine ou p-aminobenzamidine/protéase à sérine), (L-lysine/plasminogène (et activateur) ou ARNr), (AMP, ADP, ou ATP/ Enzyme à cofacteurs), (lectine/protéine glucannée, glucolipide, ou polysaccharide), (héparine/facteur de croissance et de coagulation, récepteur stéroïdien, endonucléase, lipoprotéine, ou lipase), ou (Cibacron blue®/ enzymes à cofacteurs NAD ou NADP, albumine, facteur de coagulation, ou interféron), (antigène/anticorps), (haptène/anticorps), (anticorps/antigène), (anticorps/haptène), (acide nitrilotriacétique (NTA)/métal de transition), (EDTA/
métal de transition), (acide phénylboronique (APB)/acide salicylhydroxamique (ASH)), ou (oligonucléotide/oligonucléotide complémentaire) ou les couples inversés correspondants.

8. Produit selon la revendication 7, **caractérisé en ce que** la molécule amphiphile est un polymère de formule (I) ou (II) comprenant en outre un pourcentage compris
entre 0 et 4% d'un monomère de formule

$$^{*}\!-\!(CH_2\text{-}\underset{\displaystyle Rbb}{\underset{|}{C}}Raa)\!-\!^{*} \quad (III)$$

,

dans laquelle :

Raa est l'atome d'hydrogène ou le radical méthyle ;
Rbb représente un groupe COORcc, ou -COSRcc ou -CORcc ou CONRccRdd, où

Rcc représente le groupement fonctionnel de la molécule amphiphile constituant la première moitié du couple récepteur-ligand, et
Rdd représente un radical $(C_1\text{-}C_5)$ alkyle, un alkylsulfonate, l'atome d'hydrogène, un reste sucre, un hydroxyalkyl -$(CH_2)$mOH primaire, secondaire ou tertiaire, avec m variant de 1 à 4, un polyoxyalkylène,

notamment polyoxyéthylène, ayant 4 à 10 unités d'oxyde d'alkylène, un radical zwitterionique, un radical $(CH_2)$t-NRf1Rf2, t nombre entier de 1 à 5, Rf1, Rf2 identiques ou différents étant l'atome d'hydrogène ou un radical $(C_1-C_4)$ alkyle.

**9.** Produit selon la revendication 6, **caractérisé en ce que :**

a. la molécule amphiphile comprend en outre au moins un groupement fonctionnel constituant la première moitié d'un couple réactif chimique,
b. le support comprend en outre au moins un groupement fonctionnel fixé à sa surface constituant la deuxième moitié dudit couple réactif chimique, et
c. la fixation de la molécule amphiphile sur le support est effectuée par réaction chimique entre le(s) groupement (s) fonctionnel(s) de la molécule amphiphile et le(s) groupement(s) fonctionnel(s) fixé(s) sur le support.

**10.** Procédé de préparation d'un produit selon l'une quelconque des revendications 1 à 9, comprenant :

a) la fourniture d'au moins une protéine membranaire, d'un support et d'une molécule amphiphile telle que définie dans l'une quelconque des revendications 1 à 4, et 7 à 9,
b) la formation d'un complexe entre la molécule amphiphile et la protéine membranaire, et
c) la fixation de la molécule amphiphile du complexe sur le support par une liaison hydrophobe, une liaison ionique, une liaison spécifique de type récepteur-ligand entre au moins un groupement fonctionnel de la molécule amphiphile et au moins un groupement fonctionnel à la surface du support, ou une liaison covalente entre au moins un groupement fonctionnel réactif de la molécule amphiphile et au moins un groupement fonctionnel réactif à la surface du support.

**11.** Utilisation d'un produit selon l'une quelconque des revendications 1 à 9 pour détecter la présence ou l'absence dans un échantillon biologique d'un ligand de ladite au moins une protéine membranaire, ladite au moins une protéine membranaire étant avantageusement un antigène membranaire d'un agent pathogène, ledit échantillon biologique un échantillon de sérum, et ledit ligand à détecter un anticorps dirigé contre ledit antigène.

**12.** Utilisation d'un produit selon l'une quelconque des revendications 1 à 9 pour cribler une banque de composés à la recherche de ligands de ladite au moins une protéine membranaire.

**13.** Utilisation in vitro d'un produit selon l'une quelconque des revendications 1 à 9, dans lequel ladite au moins une protéine est une enzyme, pour la transformation du substrat de ladite enzyme dans des conditions contrôlées.

**14.** Kit pour la mise en oeuvre du procédé selon la revendication 10, comprenant un support et une molécule amphiphile, **caractérisé en ce que** la fixation entre ladite molécule amphiphile et ledit support a lieu par une liaison hydrophobe, une liaison ionique, une liaison spécifique de type récepteur-ligand entre au moins un groupement fonctionnel de la molécule amphiphile et au moins un groupement fonctionnel à la surface du support, ou une liaison covalente entre au moins un groupement fonctionnel réactif de la molécule amphiphile et au moins un groupement fonctionnel réactif à la surface du support.

**15.** Kit selon la revendication 14, **caractérisé en ce que :**

a) la molécule amphiphile comprend en outre au moins un groupement fonctionnel constituant la première moitié d'un couple de molécules récepteur-ligand,
b) le support comprend en outre au moins un groupement fonctionnel fixé à sa surface constituant la deuxième moitié dudit couple de molécules récepteur-ligand, et
c) la fixation de la molécule amphiphile sur le support est médiée par une liaison spécifique de type récepteur-ligand entre le(s) groupement(s) fonctionnel(s) de la molécule amphiphile et le(s) groupement(s) fonctionnel(s) fixé(s) sur le support,

le couple récepteur-ligand (groupement fonctionnel du polymère vinylique amphiphile / groupement fonctionnel fixé sur le support) étant choisi parmi les couples (biotine/avidine), (glutathion/glutathion S-transférase, protéines fixant le glutathion, ou protéines de fusion incluant la glutathion S-transférase), (calmoduline/ ATPase, protéine kinase, phosphodiestérase, ou neurotransmetteur), (L-arginine ou p-aminobenzamidine/protéase à sérine), (L-lysine/plasminogène (et activateur) ou ARNr), (AMP, ADP, ou ATP/ Enzyme à cofacteurs), (lectine/protéine glucannée, glucolipide, ou polysaccharide), (héparine/facteur de croissance et de coagulation, récepteur stéroïdien, endonucléase,

lipoprotéine, ou lipase), ou (Cibacron blue®/enzymes à cofacteurs NAD ou NADP, albumine, facteur de coagulation, ou interféron), ou les couples inversés correspondants, (antigène/anticorps), (haptène/anticorps), (anticorps/antigène), (anticorps/haptène), (acide nitrilotriacétique (NTA)/métal de transition), (EDTA/métal de transition), (acide phénylboronique (APB)/acide salicylhydroxamique (ASH)), ou (oligonucléotide/oligonucléotide complémentaire).

**16.** Kit selon la revendication 15, **caractérisé en ce que** la molécule amphiphile est un polymère de formule (I) ou (II) comprenant en outre un pourcentage compris entre 0 et 4% d'un monomère de formule

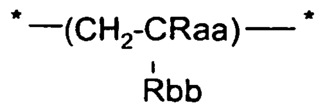
(III)

,

dans laquelle Raa et Rbb sont tels que définis dans la revendication 8.

**17.** Kit selon la revendication 14, **caractérisé en ce que** :

a) la molécule amphiphile comprend en outre au moins un groupement fonctionnel constituant la première moitié d'un couple réactif chimique,
b) le support comprend en outre au moins un groupement fonctionnel fixé à sa surface constituant la deuxième moitié dudit couple réactif chimique, et
c) la fixation de la molécule amphiphile sur le support est effectuée par réaction chimique entre le(s) groupement(s) fonctionnel(s) de la molécule amphiphile et le(s) groupement(s) fonctionnel(s) fixé(s) sur le support.

**18.** Utilisation d'une molécule amphiphile tel que définie dans l'une quelconque des revendications 1 à 4, et 7 à 9 pour complexer une protéine membranaire et la fixer sur un support.

**19.** Molécule amphiphile telle que définie dans l'une quelconque des revendications 1 à 4, comprenant en outre un groupement fonctionnel constituant la première moitié d'un couple de molécules récepteur-ligand, le groupement fonctionnel étant choisi parmi la biotine, l'avidine, le glutathion, la glutathion S-transférase, la calmoduline, une ATPase, une protéine kinase, une phosphodiestérase, un neurotransmetteur, la L-arginine, la p-aminobenzamidine, une protéase à sérine, la L-lysine, le plasminogène (et activateur), un ARNr, l'AMP, l'ADP, l'ATP, une enzyme à cofacteurs, une lectine, une protéine glucannée, un glucolipide, un polysaccharide, l'héparine, un facteur de croissance et de coagulation, un récepteur stéroïdien, une endonucléase, une lipoprotéine, une lipase, du Cibacron blue®, une enzyme à cofacteurs NAD ou NADP, l'albumine, un facteur de coagulation, un interféron, un antigène, un haptène, un anticorps, l'acide nitrilotriacétique (NTA), l'EDTA ou un oligonucléotide.

**20.** Molécule amphiphile selon la revendication 19, **caractérisée en ce que** la molécule amphiphile est un polymère de formule (I) ou (II) comprenant en outre un pourcentage compris entre 0 et 4% d'un monomère de formule

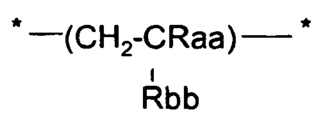
(III)

,

dans laquelle Raa et Rbb sont tels que définis dans la revendication 8.

**21.** Molécule amphiphile telle que définie dans l'une quelconque des revendications 1 à 4, comprenant en outre un groupement fonctionnel constituant la première moitié d'un couple réactif chimique choisi parmi des groupements cyclopentadienyle capables de réagir de manière spontanée suivant un mécanisme Diels-Alder avec des dérivés quinoniques fixés à la surface du support solide, des groupements alkynyle (triple liaison) capables de réagir avec des groupements azoture fixés à la surface du support solide, ou des groupements alkoxylamine ou carbonyle pouvant se condenser d'une façon très sélective sur une fonction carbonyle ou alkoxylamine fixé à la surface du support solide.

**22.** Procédé d'analyse des protéines membranaires présentes dans un échantillon, comprenant la préparation d'un produit selon l'une quelconque des revendications 1-9, le procédé comprenant avantageusement les étapes consistant à :

a) solubiliser les protéines membranaires en détergent,
b) complexer les protéines membranaires avec une molécule amphiphile telle que définie dans les revendications 1-5 et éliminer le détergent,
c) immobiliser les protéines membranaires sur un support tel que défini dans les revendications 1, et 6 à 8 par l'intermédiaire de la molécule amphiphile,
d) laver extensivement et ajouter une protéase pour générer des fragments protéiques des protéines membranaires, le domaine transmembranaire restant complexé la molécule amphiphile fixée au support,
e) éliminer le support auquel reste attaché le domaine transmembranaire restant complexé la molécule amphiphile et analyser les fragments protéiques par spectrométrie de masse.

## Claims

**1.** A product comprising a support and at least one membrane protein attached to the surface thereof, wherein said membrane protein is attached to said support using an amphiphilic molecule with which said membrane protein is complexed, and wherein the amphiphilic molecule is chosen from an amphiphilic vinyl polymer or an amphiphilic polypeptide, and said at least one membrane protein is chosen advantageously from an antigen, an antibody, an enzyme, a cell receptor, an ion channel, and a membrane protein of viral, bacterial, or eukaryotic origin.

**2.** The product according to claim 1 wherein the amphiphilic molecule is a vinyl polymer of formula (I):

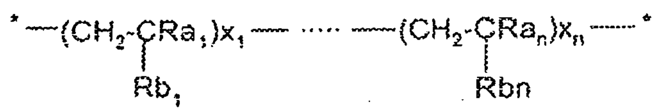
(I)

wherein:

$Ra_1$ to $Ra_n$ are the same or different, and represent a hydrogen atom, or a methyl radical;
$Rb_1$ to $Rb_n$ are different and selected from:

- a hydrophilic group selected from:

• a carboxylate radical $-COO^-M^+$, a sulfonate radical $-SO_3^-M^+$, or a phosphonate radical $-PO_3^-M^+$, wherein $M^+$ is a cationic counter-ion;
• a ($C_1$-$C_5$) alkylcarboxylate radical, a ($C_1$-$C_5$) alkylsulfonate radical, or a ($C_1$-$C_5$) alkylphosphonate radical;
• a phenylsulfonate;
• CONRc1Rc2, wherein Rc1 and Rc2, which may be the same or different, represent a $(-C(CH_2ORd1)(CH_2ORd2)(CH_2ORd3))$ radical, wherein Rd1, Rd2 and Rd3 represent independently a hydrogen atom, a sugar moiety, a polyoxyalkylene containing from 4 to 10 alkylene oxide units, a zwitterionic radical, a primary, secondary or tertiary hydroxyalkyl $-(CH_2)mOH$, wherein m is within the range of 1 to 4, a ($C_1$-$C_5$) alkylcarboxylate radical, a ($C_1$-$C_5$) alkylsulfonate radical, a ($C_1$-$C_5$) alkylphosphonate radical, or a sugar moiety;
• COORe, wherein Re represents a sugar moiety, a primary, secondary or tertiary hydroxyalkyl $-(CH_2)mOH$, wherein m is within the range of 1 to 4, a polyoxyalkylene, notably polyoxyethylene, having 4 to 10 alkylene oxide units, a $(CH_2)t-NRf1Rf2$ radical, wherein t is an integer from 1 to 5, and Rf1, Rf2, which may be the same or different, represent a hydrogen atom or a ($C_1$-$C_4$) alkyl radical;
• a hydroxyl group;
• a primary, secondary or tertiary hydroxyalkyl $-(CH_2)mOH$, wherein m is within the range of 1 to 4;
• a primary, secondary, tertiary amine;
• a quaternary ammonium;
• N-formamide or N-alkylformamide;

- N-acetamide or N-alkylacetamide;
- N-pyrrolidonyl;
- CONRg1Rg2, wherein Rg1 and Rg2, which may be the same or different, are a hydrogen atom, a sugar moiety, a polyoxyalkylene, notably polyoxyethylene, containing from 4 to 10 alkylene oxide units, a zwitterionic radical, a primary, secondary or tertiary hydroxyalkyl $-(CH_2)mOH$, wherein m is within the range of 1 to 4;
- COORh or CONRkRl, wherein Rh represents a ($C_1$-$C_5$) alkyl radical, an alkylsulfonate, or has one of the meanings given for Re or Rg1, provided that it is not a hydrogen atom, and Rk and Rl have independently one of the meanings given for Rh, and additionally one of them can represent a hydrogen atom;

- a hydrophobic group selected from:

  - a hydrogen atom;
  - a halogen atom;
  - a $-CONH(-C(CH_2ORm1)\ (CH_2ORm2)\ (CH_2ORm3))$ radical, wherein Rm1, Rm2, Rm3 are independently a linear or branched alkyl, alkenyl or alkynyl having from 3 to 50 carbon atoms, an alkylcarbamoyl (O=C-NH-Rn) or an acyl (O=C-Ro), where Rn and Ro are linear or branched alkyl, alkenyl or alkynyl radicals having from 3 to 50 carbon atoms;
  - COORp, CORp, COSRp, C-NH-Rp or CONRq1Rq2, wherein Rp is a linear or branched and/or cyclic alkyl, alkynyl or alkenyl radical containing from 3 to 50 carbon atoms, and Rq1 and Rq2, which may be the same or different, have one of the meanings given for Rp, and further either one of them can represent a hydrogen atom;
  - a -Rr, -ORr, or -SRr radical wherein Rr represents a linear or branched and/or cyclic alkyl, alkenyl or alkynyl group containing from 3 to 50 carbon atoms; or

- an amphiphilic group selected from:

  - an alkyl radical $-(CH_2)m$-Rs, wherein m is between 6 and 20, and Rs is a hydrophilic group chosen from carboxylate, sulfonate, phosphonate, sulfate, phosphate, zwitterion, ammonium, poly(oxyethylene), and sugar;
  - a poly(oxyethylene)-O-alkyl radical $(-(CH_2CH_2O)m$-Rt) wherein Rt is a linear, branched or cyclic alkyl, alkenyl, alkynyl radical with 6 to 20 carbon atoms;
  - a COORu, CORu, COSRu, CONRvRw radical, wherein Ru is a poly(oxyethylene)-O-alkyl radical $(-(CH_2CH_2O)m$-Rt) and wherein Rt is a linear, branched or cyclic alkyl, alkenyl, alkynyl radical with 6 to 20 carbon atoms, a glycosylalkyl radical, wherein Rv may be a hydrogen atom or has one of the meanings given for Ru, wherein Rw has one of the meanings given for Ru;
  - a $-CONH\ (-C(CH_2ORx1)\ (CH_2ORx2)\ (CH_2ORx3))$ radical, wherein Rx1, Rx2, Rx3 are such that one or two of these groups have one of the meanings given for Rm1, Rm2, Rm3 and one or two of these groups are different from a hydrogen atom and have one of the meanings given for Rd1, Rd2, Rd3, and Ru; or Rx1, Rx2, Rx3 are the same or different, and are such that at least one of the groups is different from a hydrogen atom and has one of the meanings given for Ru;

n is an integer equal to or greater than 2, preferably between 2 and 10, between 2 and 8, between 2 and 6, between 2 and 4, advantageously n equals 3;

$x_1$ to $x_n$ represent, respectively, the percentages of the units $(\sum_{i=1}^{n} x_i = 100\%)$, wherein the ratio of the total percentage of groups wherein $Rb_i$ is a hydrophobic or amphiphilic group to the total percentage of groups wherein $Rb_i$ is a hydrophilic group $\sum_{hydrophobic\ Rb_i} x_i + \sum_{amphiphilic\ Rb_j} x_j \Big/ \sum_{hydrophilic\ Rb_k} x_k$ is between 0.25 and 2.5; and

the average molecular weight is between 500 and 100,000, advantageously between 500 and 50,000.

**3.** The product according to claim 2 wherein n is 3 and the vinyl polymer is of formula (II):

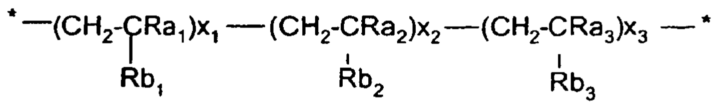 (II)

,

wherein:

$Ra_1$, $Ra_2$ and $Ra_3$ are the same or different, and represent a hydrogen atom, or a methyl radical;
$Rb_1$ is a hydrophilic group chosen from:

- a carboxylate radical $-COO^-M^+$, a sulfonate radical $-SO_3^-M^+$, or a phosphonate radical $-PO_3^-M^+$, wherein $M^+$ is a cationic counter-ion;
- a ($C_1$-$C_5$) alkylcarboxylate radical, a ($C_1$-$C_5$) alkylsulfonate radical, or a ($C_1$-$C_5$) alkylphosphonate radical;
- a phenylsulfonate;
- CONRc1Rc2, wherein Rc1 and Rc2, which may be the same or different, represent a ($-C(CH_2ORd1)(CH_2ORd2)(CH_2ORd3)$) radical, wherein Rd1, Rd2 and Rd3 represent independently a hydrogen atom, a sugar moiety, a polyoxyalkylene, notably polyoxyethylene, containing from 4 to 10 alkylene oxide units, a zwitterionic radical, a primary secondary or tertiary hydroxyalkyl $-(CH_2)mOH$, wherein m is within the range of 1 to 4, a ($C_1$-$C_5$) alkylcarboxylate radical, a ($C_1$-$C_5$) alkylsulfonate radical, a ($C_1$-$C_5$) alkylphosphonate radical, or a sugar moiety;

$Rb_2$ is chosen from:

- a hydrophobic group chosen from:

  - a hydrogen atom;
  - a halogen atom;
  - a $-CONH(-C(CH_2ORm1)(CH_2ORm2)(CH_2ORm3))$ radical, wherein Rm1, Rm2, Rm3 are independently a linear or branched alkyl, alkenyl or alkynyl comprising from 3 to 50 carbon atoms, an alkylcarbamoyl (O=C-NH-Rn) or an acyl (O=C-Ro), wherein Rn and Ro are linear or branched alkyl, alkenyl or alkynyl radicals having from 3 to 50 carbon atoms;
  - COORp, CORp, CSRp, C-NH-Rp or CONRq1Rq2, wherein Rp is a linear or branched and/or cyclic alkyl, alkynyl or alkenyl radical containing from 3 to 50 carbon atoms, and Rq1 and Rq2, which may be the same or different, have one of the meanings given for Rp, and further either one of them can represent a hydrogen atom;
  - a -Rr, -ORr, or -SRr radical wherein Rr represents a linear or branched and/or cyclic alkyl, alkenyl or alkynyl group containing from 3 to 50 carbon atoms; or

- an amphiphilic group chosen from:

  - an alkyl radical $-(CH_2)m-Rs$, wherein m is between 6 and 20, Rs is a hydrophilic chosen from carboxylate, sulfonate, phosphonate, sulfate, phosphate, zwitterion, ammonium, poly(oxyethylene), and sugar;
  - a poly(oxyethylene)-O-alkyl radical ($-(CH_2CH_2O)m-Rt$) wherein Rt is a linear, branched or cyclic alkyl, alkenyl, alkynyl radical with 6 to 20 carbon atoms;
  - a COORu, CORu, COSRu, CONRvRw radical, wherein Ru is a poly(oxyethylene)-O-alkyl radical ($-(CH_2CH_2O)m-Rt$) and wherein Rt is a linear, branched or cyclic alkyl, alkenyl, alkynyl radical with 6 to 20 carbon atoms, a glycosylalkyl radical, wherein Rv may be a hydrogen atom or has one of the meanings given for Ru, wherein Rw has one of the meanings given for Ru;
  - a $-CONH(-C(CH_2ORx1)(CH_2ORx2)(CH_2ORx3))$ radical, wherein Rx1, Rx2, Rx3 are such that one or two of these groups have one of the meanings given for Rm1, Rm2, Rm3, and one or two of these groups are different from a hydrogen atom and have one of the meanings given for Rd1, Rd2, Rd3, Ru; or Rx1, Rx2, Rx3 are the same or different, and are such that at least one of the groups is different from a hydrogen atom and has one of the meanings given for Ru;

$Rb_3$ is a hydrophilic group chosen from:

• COORe, wherein Re represents a sugar moiety, a primary, secondary or tertiary hydroxyalkyl -$(CH_2)$mOH, wherein m is within the range of 1 to 4, a polyoxyalkylene, notably polyoxyethylene, having 4 to 10 alkylene oxide units, a $(CH_2)$t-NRf1Rf2 radical, where t is an integer from 1 to 5, and Rf1, Rf2, which may be the same or different, represent a hydrogen atom or a $(C_1-C_4)$ alkyl radical;
• a hydroxyl group;
• a primary, secondary or tertiary hydroxyalkyl -$(CH_2)$mOH, wherein m is within the range of 1 to 4;
• a primary, secondary, tertiary amine;
• a quaternary ammonium;
• N-formamide or N-alkylformamide;
• N-acetamide or N-alkylacetamide;
• N-pyrrolidonyl;
• CONRg1Rg2, wherein Rg1 and Rg2, which may be the same or different, are a hydrogen atom, a sugar moiety, a polyoxyalkylene, notably polyoxyethylene, containing from 4 to 10 alkylene oxide units, a zwitterionic radical, a primary, secondary or tertiary hydroxyalkyl -$(CH_2)$mOH, wherein m is within the range of 1 to 4 (R3),
• COORh or CONRkRl, wherein Rh represents a $(C_1-C_5)$ alkyl radical, an alkylsulfonate, or has one of the meanings given for Re or Rg1, provided that it is not a hydrogen atom, and Rk, Rl have independently one of the meanings given for Rh, and additionally one of them can represent a hydrogen atom;

x1, x2, x3 represent the respective percentages of the units, wherein:

- x1 is between 20 and 90%;
- x2 is between 10 and 80%;
- x3 is between 0 and 60%; and
- $x_2/x_1+x_3$ is between 0.25 and 2.5; and

wherein the average molecular weight is between 500 and 100,000, advantageously between 500 and 50,000.

**4.** The product according to claim 3 wherein:

$Ra_1$, $Ra_2$ and $Ra_3$ are the same or different, and represent a hydrogen atom or a methyl radical;
$Rb_1$ represents $COO^-M^+$, wherein $M^+$ is a cationic counter-ion;
$Rb_2$ represents CONRq1Rq2, wherein Rq1 and Rq2 represent independently a linear or branched and/or cyclic alkyl, alkynyl or alkenyl radical containing from 3 to 50 carbon atoms, and further either one of them can represent a hydrogen atom;
$Rb_3$ represents CONRkRl, wherein Rk and Rl represent independently a $(C_1-C_5)$ alkyl radical, an alkylsulfonate, a sugar moiety, a primary, secondary or tertiary hydroxyalkyl -$(CH_2)$mOH, wherein m is within the range of 1 to 4, a polyoxyalkylene, notably polyoxyethylene, having 4 to 10 alkylene oxide units, a zwitterionic radical, a $(CH_2)$t-NRf1Rf2 radical, wherein t is an integer from 1 to 5, Rf1, Rf2 are the same or different, and represent a hydrogen atom or a $(C_1-C_4)$ alkyl radical, and further either one of Rk and Rl can represent a hydrogen atom.

**5.** The product according to any of claims 1 to 4 wherein the support is a solid support, advantageously the support is chosen from a chip, a bead, a membrane, a fiber, a nanotube, and a soluble macromolecule or particle chosen from a polymer, a dendrimer, a vesicle, and a micelle.

**6.** The product according to any of claims 1 to 5 wherein the binding of the amphiphilic molecule to the support is mediated by a hydrophobic bond, an ionic bond, a specific receptor-ligand binding between at least one functional group in the amphiphilic molecule and at least one functional group at the support surface, or a covalent bond between at least one reactive functional group in the amphiphilic molecule and at least one reactive functional group at the support surface.

**7.** The product according to claim 6 wherein:

a) the amphiphilic molecule further comprises at least one functional group providing the first half of a receptor-ligand molecule pair;
b) the support further comprises at least one functional group attached to the surface thereof providing the second half of said receptor-ligand molecule pair; and
c) the binding of the amphiphilic molecule to the support is mediated through a specific receptor-ligand binding

between the functional group(s) in the amphiphilic molecule and the functional group(s) attached to the support,

the receptor-ligand pair (functional group in the amphiphilic molecule/functional group attached to the support) being chosen from the following pairs: (biotin/avidin), (glutathion/glutathion S-transferase, glutathion-binding proteins, or fusion proteins including glutathion S-transferase), (calmoduline/ATPase, protein kinase, phosphodiesterase, or neurotransmitter), (L-arginine or p-aminobenzamidine/serine protease), (L-lysine/plasminogen (and activator) or rRNA), (AMP, ADP, or ATP/Cofactor enzyme), (lectin/glucanated protein, glucolipid, or polysaccharide), (heparin/growth and coagulation factor, steroid receptor, endonuclease, lipoprotein, or lipase), or (Cibacron Blue®/NAD or NADP cofactor enzymes, albumin, coagulation factor, or interferon), (antigen/antibody), (hapten/antibody), (antibody/antigen), (antibody/hapten), (nitrilotriacetic acid (NTA)/transition metal), (EDTA/transition metal), (phenylboronic acid (APB)/salicylhydroxamic acid (ASH)), and (oligonucleotide/complementary oligonucleotide), and the corresponding reversed pairs.

**8.** The product according to claim 7 wherein the amphiphilic molecule is a polymer of formula (I) or (II) further comprising a percentage of between 0 and 4% of a monomer of the formula

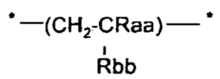 (III)

,

wherein:

Raa is a hydrogen atom or a methyl radical;
Rbb represents a COORcc, or -COSRcc or -CORcc or CONRccRdd group; wherein
Rcc represents the functional group in the amphiphilic molecule providing the first half of the receptor-ligand pair; and
Rdd represents a ($C_1$-$C_5$) alkyl radical, an alkylsulfonate, a hydrogen atom, a sugar moiety, a primary, secondary or tertiary hydroxyalkyl -($CH_2$)mOH, where m is within the range of 1 to 4, a polyoxyalkylene, notably polyoxyethylene, having 4 to 10 alkylene oxide units, a zwitterionic radical, a ($CH_2$)t-NRf1Rf2 radical, where t is an integer from 1 to 5, Rf1, Rf2 are the same or different, and represent a hydrogen atom or a ($C_1$-$C_4$) alkyl radical.

**9.** The product according to claim 6 wherein:

a) the amphiphilic molecule further comprises at least one functional group providing the first half of a chemical reactive pair;
b) the support further comprises at least one functional group attached to the surface thereof providing the second half of said chemical reactive pair; and
c) the binding of the amphiphilic molecule to the support is carried out by chemically reacting the functional group(s) in the amphiphilic molecule with the functional group(s) attached to the support.

**10.** A process for preparing a product according to any of claims 1 to 9 comprising:

a) providing at least one membrane protein, a support, and an amphiphilic molecule according to claims 1 to 4, and 7 to 9;
b) forming a complex between the amphiphilic molecule and the membrane protein; and
c) attaching the amphiphilic molecule of the complex to the support via a hydrophobic bond, an ionic bond, a specific receptor-ligand binding between at least one functional group in the amphiphilic molecule and at least one functional group at the support surface, or a covalent bond between at least one reactive functional group in the amphiphilic molecule and at least one reactive functional group at the support surface.

**11.** Use of a product according to any of claims 1 to 9 for detecting the presence or absence in a biological specimen of a ligand of said at least one membrane protein, said at least one membrane protein being advantageously a membrane antigen of a pathogenic agent, said biological specimen a serum sample, and said ligand to be detected an antibody raised against said antigen.

**12.** Use of a product according to any of claims 1 to 9 for screening a bank of compounds for ligands of said at least one membrane protein.

**13.** Use *in vitro* of a product according to any of claims 1 to 9, wherein said at least one protein is an enzyme, for transforming the substrate of said enzyme under controlled conditions.

**14.** A kit for carrying out the process according to claim 10, comprising a support and an amphiphilic molecule, wherein the binding between said amphiphilic molecule and said support occurs through a hydrophobic bond, an ionic bond, a specific receptor-ligand binding between at least one functional group in the amphiphilic molecule and at least one functional group at the support surface, or a covalent bond between at least one reactive functional group in the amphiphilic molecule and at least one reactive functional group at the support surface.

**15.** The kit according to claim 14 wherein:

a) the amphiphilic molecule further comprises at least one functional group providing the first half of a receptor-ligand molecule pair;
b) the support further comprises at least one functional group attached to the surface thereof providing the second half of said receptor-ligand molecule pair; and
c) the binding of the amphiphilic molecule to the support is mediated through a specific receptor-ligand binding between the functional group(s) in the amphiphilic molecule and the functional group(s) attached to the support,

the receptor-ligand pair (functional group of the amphiphilic vinyl polymer/functional group attached to the support) is chosen from the following pairs: (biotin/avidin), (glutathion/glutathion S-transferase, glutathion-binding proteins, or fusion proteins including glutathion S-transferase), (calmoduline/ATPase, protein kinase, phosphodiesterase, or neurotransmitter), (L-arginine or p-aminobenzamidine/serine protease), (L-lysine/plasminogen (and activator) or rRNA), (AMP, ADP, or ATP/Cofactor enzyme), (lectin/glucanated protein, glucolipid, or polysaccharide), (heparin/growth and coagulation factor, steroid receptor, endonuclease, lipoprotein, or lipase), and (Cibacron Blue®/NAD or NADP cofactor enzymes, albumin, coagulation factor, or interferon), and the corresponding reversed pairs, (antigen/antibody), (hapten/antibody), (antibody/antigen), (antibody/hapten), (nitrilotriacetic acid (NTA)/transition metal), (EDTA/transition metal), (phenylboronic acid (APB)/salicylhydroxamic acid (ASH)), or (oligonucleotide/complementary oligonucleotide).

**16.** The kit according to claim 15 wherein the amphiphilic molecule is a polymer of the formula (I) or (II) further comprising a percentage of between 0 and 4% of a monomer of the formula

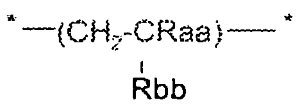

(III)

,

wherein Raa and Rbb are as defined in claim 8.

**17.** The kit according to claim 14 wherein:

a) the amphiphilic molecule further comprises at least one functional group providing the first half of a chemical reactive pair;
b) the support further comprises at least one functional group attached to the surface thereof providing the second half of said chemical reactive pair; and
c) the binding of the amphiphilic molecule to the support is carried out by chemically reacting the functional group(s) in the amphiphilic molecule with the functional group(s) attached to the support.

**18.** Use of an amphiphilic molecule as defined in any of claims 1 to 4, and 7 to 9 to complex a membrane protein and bind said membrane protein on a support.

**19.** An amphiphilic molecule such as defined in any of claims 1 to 4, further comprising a functional group providing the first half of a receptor-ligand molecule pair, wherein the functional group is chosen from biotin, avidin, glutathion, glutathion S-transferase, calmoduline, an ATPase, a protein kinase, a phosphodiesterase, a neurotransmitter, L-arginine, p-aminobenzamidine, a serine protease, L-lysine, plasminogen (and activator), an rRNA, AMP, ADP, ATP, a cofactor enzyme, a lectin, a glucanated protein, a glucolipid, a polysaccharide, heparin, a growth and coagulation factor, a steroid receptor, an endonuclease, a lipoprotein, a lipase, Cibacron Blue®, a NAD or NADP cofactor enzyme,

albumin, a coagulation factor, an interferon, an antigen, a hapten, an antibody, nitrilotriacetic acid (NTA), EDTA, and an oligonucleotide.

**20.** The amphiphilic molecule according to claim 19 wherein the amphiphilic molecule is a polymer of the formula (I) or (II) further comprising a percentage of between 0 and 4% of a monomer of the formula

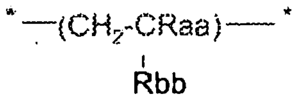 (III)

,

wherein Raa and Rbb are as defined in claim 8.

**21.** An amphiphilic molecule as defined in any of claims 1 to 4 further comprising a functional group providing the first half of a chemical reactive pair chosen from cyclopentadienyl groups able to react spontaneously following a Diels-Alder mechanism with quinone derivatives attached to the surface of the solid support, alkynyl groups (triple bond) able to react with azide groups attached to the surface of the solid support, or alkoxylamine or carbonyl groups that can condense highly selectively on a carbonyl or alkoxylamine function attached to the surface of the solid support.

**22.** A process for assaying membrane proteins in a sample, comprising the preparation of a product according to any of claims 1 to 9, the process advantageously comprising the following steps:

a) solubilizing the membrane proteins in a detergent;
b) complexing the membrane proteins with an amphiphilic molecule as defined in claims 1 to 5 and removing the detergent;
c) immobilizing the membrane proteins onto a support as defined in claims 1, and 6 to 8 through the amphiphilic molecule;
d) extensively washing, and adding a protease for generating protein fragments of the membrane proteins, wherein the transmembrane domain remains complexed with the amphiphilic molecule attached to the support;
e) removing the support to which the transmembrane domain still complexed with the amphiphilic molecule remains bound, and analyzing the protein fragments by mass spectrometry.

## Patentansprüche

**1.** Produkt, welches einen Träger und mindestens ein Membranprotein, das an dessen Oberfläche angeheftet ist, umfasst, **dadurch gekennzeichnet, dass** das Membranprotein an den Träger mittels eines Zwischenglieds aus einem amphiphilen Molekül, mit welchem das Membranprotein komplexiert ist, angeheftet ist, wobei das amphiphile Molekül aus einem amphiphilen vinylischen Polymer oder einem amphiphilen Polypeptid ausgewählt ist und wobei das mindestens eine Membranprotein vorteilhaft ausgewählt ist aus einem Antigen, einem Antikörper, einem Enzym, einem zellulären Rezeptor, einem Ionenkanal oder einem Membranprotein viraler, bakterieller oder eukaryotischer Herkunft.

**2.** Produkt nach Anspruch 1, **dadurch gekennzeichnet, dass** das amphiphile Molekül ein vinylisches Polymer der folgenden Formel (I) ist:

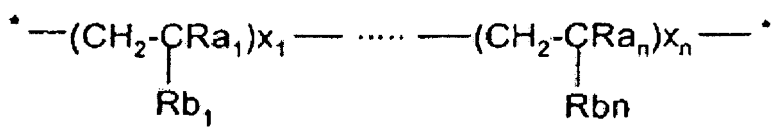 (I)

,

in welcher:

$Ra_1$ bis $Ra_n$, die gleich oder verschieden sind, ein Wasserstoffatom oder ein Methylrest sind;
$Rb_1$ bis $Rb_n$ verschieden und ausgewählt sind aus:

- einer hydrophilen Gruppe, ausgewählt aus:

  • einem Carboxylatrest $-COO^-M^+$, einem Sulfonatrest $-SO_3^-M^+$ oder einem Phosphonatrest $-PO_3^-M^+$, wobei $M^+$ ein kationisches Gegenion ist,
  • einem ($C_1$-$C_5$)-Alkylcarboxylatrest, einem ($C_1$-$C_5$)-Alkylsulfonatrest oder einem ($C_1$-$C_5$)-Alkylphosphonatrest,
  • einem Phenylsulfonat,
  • CONRc1Rc2, wobei Rc1 und Rc2, die gleich oder verschieden sind, ein Rest ($-C(CH_2ORd1)(CH_2ORd2)(CH_2ORd3)$) sind, wobei Rd1, Rd2, Rd3 unabhängig voneinander für ein Wasserstoffatom, einen Zukkerrest, Polyoxyalkylen, insbesondere Polyoxyethylen, mit 4 bis 10 Alkylenoxideinheiten, einen zwitterionischen Rest, ein primäres, sekundäres oder tertiäres Hydroxyalkyl $-(CH_2)mOH$, wobei m von 1 bis 4 variiert, einen ($C_1$-$C_5$)-Alkylcarboxylatrest, einen ($C_1$-$C_5$)-Alkylsulfonatrest oder einen ($C_1$-$C_5$)-Alkylphosphonatrest, einen Zuckerrest stehen;
  • COORe, wobei Re ein Zuckerrest, ein primäres, sekundäres oder tertiäres Hydroxyalkyl $-(CH_2)mOH$, wobei m von 1 bis 4 variiert, ein Polyoxyalkylen, insbesondere Polyoxyethylen, mit 4 bis 10 Alkylenoxideinheiten, ein Rest $(CH_2)t$-NRf1Rf2, wobei t eine ganze Zahl von 1 bis 5 ist, Rf1, Rf2, die gleich oder verschieden sind, ein Wasserstoffatom oder ein ($C_1$-$C_4$)-Alkylrest sind, ist;
  • einem Hydroxyl;
  • einem primären, sekundären oder tertiären Hydroxyalkyl $-(CH_2)mOH$, wobei m von 1 bis 4 variiert;
  • einem primären, sekundären, tertiären Amin;
  • einem quartären Ammonium;
  • N-Formamid, N-Alkylformamid;
  • N-Acetamid, N-Alkylacetamid;
  • N-Pyrrolidonyl;
  • CONRg1Rg2, wobei Rg1 und Rg2, die gleich oder verschieden sind, ein Wasserstoffatom, ein Zukkerrest, Polyoxyalkylen, insbesondere Polyoxyethylen, mit 4 bis 10 Alkylenoxideinheiten, ein zwitterionischer Rest, ein primäres, sekundäres oder tertiäres Hydroxyalkyl $-(CH_2)mOH$, wobei m von 1 bis 4 variiert, sind,
  • COORh oder CONRkRl, wobei Rh ein ($C_1$-$C_5$)-Alkylrest, ein Alkylsulfonat ist oder eine der Bedeutungen von Re oder von Rg1 hat mit der Ausnahme eines Wasserstoffatoms, und Rk und Rl unabhängig von einander eine der Bedeutungen von Rh haben und außerdem einer von den beiden einem Wasserstoffatom entsprechen kann;

- einer hydrophoben Gruppe, ausgewählt unter:

  • einem Wasserstoffatom;
  • einem Halogenatom;
  • einem Rest $-CONH(-C(CH_2ORm1)(CH_2ORm2)(CH_2ORm3))$, wobei Rm1, Rm2, Rm3 unabhängig voneinander ein lineares oder verzweigtes Alkyl, Alkenyl oder Alkinyl mit 3 bis 50 Kohlenstoffatomen, ein Alkylcarbamoyl (O=C-NH-Rn) oder ein Acyl (O=C-Ro) sind, wobei Rn und Ro lineare oder verzweigte Alkyl-, Alkenyl- oder Alkinylreste mit 3 bis 50 Kohlenstoffatomen sind,
  • COORp, CORp, COSRp, C-NH-Rp oder CONRq1Rq2, worin Rp ein linearer oder verzweigter und/oder cyclischer Alkyl-, Alkenyl- oder Alkinylrest, welcher 3 bis 50 Kohlenstoffatome umfasst, ist und Rq1 und Rq2, die gleich oder verschieden sind, eine der Bedeutungen von Rp haben und außerdem einer der beiden einem Wasserstoffatom entsprechen kann,
  • einem Rest -Rr, -ORr oder -SRr, worin Rr für eine lineare oder verzweigte und/oder cyclische Alkyl-, Alkenyl- oder Alkinylgruppe, welche 3 bis 50 Kohlenstoffatome umfasst, steht; oder

- einer amphiphilen Gruppe, ausgewählt aus:

  • einem Alkylrest $-(CH_2)m$-Rs, wobei m zwischen 6 und 20 eingeschlossen liegt, wobei Rs eine hydrophile Gruppe vom Carboxylat-, Sulfonat-, Phosphonat-, Sulfat-, Phosphat-, Zwitterion-, Ammonium-, Poly(oxyethylen)-, Zuckertyp bezeichnet,
  • einem Rest Poly(oxyethylen)-O-alkyl ($-(CH_2CH_2O)m$-Rt), wobei Rt ein linearer, verzweigter oder cyclischer Alkyl-, Alkenyl-, Alkinylrest mit 6 bis 20 Kohlenstoffatomen ist,
  • einem Rest COORu, CORu, COSRu, CONRvRw, wobei Ru einen Poly(oxyethylen)-O-alkyl ($-(CH_2CH_2O)m$-Rt)-Rest, wobei Rt ein linearer, verzweigter oder cyclischer Alkyl-, Alkenyl-, Alkinylrest mit 6 bis 20 Kohlenstoffatomen ist, einen Glycosylalkylrest bezeichnet, wobei Rv ein Wasserstoffatom

bezeichnen kann oder eine der Bedeutungen von Ru hat, und Rw eine der Bedeutungen von Ru hat,
• einem Rest -CONH(-C($CH_2$ORx1)($CH_2$ORx2)($CH_2$ORx3)), wobei Rx1, Rx2, Rx3 derart sind, dass eine oder zwei dieser Gruppierungen eine der Bedeutungen von Rm1, Rm2, Rm3 haben, und dass eine oder zwei dieser Gruppierungen von einem Wasserstoffatom verschieden sind und eine der Bedeutungen von Rd1, Rd2, Rd3, Ru haben, oder ebenso Rx1, Rx2, Rx3 verschieden oder gleich sind und derart sind, dass mindestens eine der Gruppierungen von einem Wasserstoffatom verschieden ist und eine der Bedeutungen von Ru hat,

n eine ganze Zahl größer oder gleich 2 ist, vorzugsweise zwischen 2 und 10 einschließlich, zwischen 2 und 8 einschließlich, zwischen 2 und 6 einschließlich, zwischen 2 und 4 einschließlich liegt, wobei n vorteilhaft gleich 3 ist;

$x_1$ bis $x_n$ den jeweiligen Prozentsätzen der Struktureinheiten $(\sum_{i=1}^{n} x_i = 100\%)$ entsprechen, worin das Verhältnis des Gesamtprozentsatzes von Gruppen, worin $Rb_i$ eine hydrophobe oder amphiphile Gruppe ist, zu dem Gesamtprozentsatz von Gruppen, worin $Rb_i$ eine hydrophile Gruppe ist,

$$\sum_{Rb_i hydrophobe} x_i + \sum_{Rb_j amphiphile} x_j \Big/ \sum_{Rb_k hydrophil} x_k$$ zwischen 0,25 und 2,5 einschließlich liegt; und

die mittlere Molmasse zwischen 500 und 100.000 einschließlich, in vorteilhafter Weise zwischen 500 und 50.000 einschließlich liegt.

3. Produkt nach Anspruch 2, **dadurch gekennzeichnet, dass** n gleich 3 ist und das vinylische Polymer die Formel (II) aufweist:

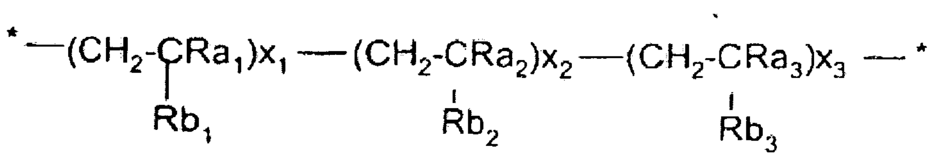 (II)

,

in welcher:

$Ra_1$, $Ra_2$ und $Ra_3$, die gleich oder verschieden sind, ein Wasserstoffatom oder ein Methylrest sind;
$Rb_1$ eine hydrophile Gruppe ist, die ausgewählt ist aus:

• einem Carboxylatrest $-COO^-M^+$, einem Sulfonatrest $-SO_3^-M^+$ oder einem Phosphonatrest $-PO_3^-M^+$, wobei $M^+$ ein kationisches Gegenion ist,
• einem ($C_1$-$C_5$)-Alkylcarboxylatrest, einem ($C_1$-$C_5$)-Alkylsulfonatrest oder einem ($C_1$-$C_5$)-Alkylphosphonatrest,
• einem Phenylsulfonat,
• CONRc1Rc2, wobei Rc1 und Rc2, die gleich oder verschieden sind, ein Rest (-C($CH_2$ORd1)($CH_2$ORd2)($CH_2$ORd3)) sind, wobei Rd1, Rd2, Rd3 unabhängig voneinander für ein Wasserstoffatom, einen Zuckerrest, Polyoxyalkylen, insbesondere Polyoxyethylen, mit 4 bis 10 Alkylenoxideinheiten, einen zwitterionischen Rest, ein primäres, sekundäres oder tertiäres Hydroxyalkyl -($CH_2$)mOH, wobei m von 1 bis 4 variiert, einen ($C_1$-$C_5$)-Alkylcarboxylatrest, einen ($C_1$-$C_5$)-Alkylsulfonatrest oder einen ($C_1$-$C_5$)-Alkylphosphonatrest, einen Zuckerrest stehen;

$Rb_2$:

- eine hydrophobe Gruppe, ausgewählt aus:

• einem Wasserstoffatom;
• einem Halogenatom;

• einem Rest -CONH(-C($CH_2$ORm1)($CH_2$ORm2)($CH_2$ORm3)), wobei Rm1, Rm2, Rm3 unabhängig voneinander ein lineares oder verzweigtes Alkyl, Alkenyl oder Alkinyl mit 3 bis 50 Kohlenstoffatomen, ein Alkylcarbamoyl (O=C-NH-Rn) oder ein Acyl (O=C-Ro) sind, wobei Rn und Ro lineare oder verzweigte Alkyl-, Alkenyl- oder Alkinylreste mit 3 bis 50 Kohlenstoffatomen sind,
• COORp, CORp, CSRp, C-NH-Rp oder CONRq1Rq2, worin Rp ein linearer oder verzweigter und/oder cyclischer Alkyl-, Alkenyl- oder Alkinylrest, welcher 3 bis 50 Kohlenstoffatome umfasst, ist und Rq1 und Rq2, die gleich oder verschieden sind, eine der Bedeutungen von Rp haben und außerdem einer der beiden einem Wasserstoffatom entsprechen kann,
• einem Rest -Rr, -ORr oder -SRr, worin Rr für eine lineare oder verzweigte und/oder cyclische Alkyl-, Alkenyl- oder Alkinylgruppe steht, welche 3 bis 50 Kohlenstoffatome umfasst; oder

- eine amphiphile Gruppe ist, ausgewählt aus:

• einem Alkylrest -($CH_2$)m-Rs, wobei m zwischen 6 und 20 eingeschlossen liegt, wobei Rs eine hydrophile Gruppe vom Carboxylat-, Sulfonat-, Phosphonat-, Sulfat-, Phosphat-, Zwitterion-, Ammonium-, Poly(oxyethylen)-, Zuckertyp bezeichnet,
• einem Rest Poly(oxyethylen)-O-alkyl (-($CH_2CH_2O$)m-Rt), wobei Rt ein linearer, verzweigter oder cyclischer Alkyl-, Alkenyl-, Alkinylrest mit 6 bis 20 Kohlenstoffatomen ist,
• einem Rest COORu, CORu, COSRu, CONRvRw, wobei Ru einen Poly(oxyethylen)-O-alkyl (-($CH_2CH_2O$)m-Rt) Rest, wobei Rt ein linearer, verzweigter oder cyclischer Alkyl-, Alkenyl-, Alkinylrest mit 6 bis 20 Kohlenstoffatomen ist, einen Glycosylalkylrest bezeichnet, wobei Rv ein Wasserstoffatom bezeichnen kann oder eine der Bedeutungen von Ru hat, und Rw eine der Bedeutungen von Ru hat,
• einem Rest -CONH(-C($CH_2$ORx1)($CH_2$ORx2)($CH_2$ORx3)), wobei Rx1, Rx2, Rx3 derart sind, dass eine oder zwei von diesen Gruppierungen eine der Bedeutungen von Rm1, Rm2, Rm3 haben und dass eine oder zwei von diesen Gruppierungen von einem Wasserstoffatom verschieden sind und eine der Bedeutungen von Rd1, Rd2, Rd3, Ru haben, oder ebenso Rx1, Rx2, Rx3 verschieden oder gleich sind und derart sind, dass mindestens eine der Gruppierungen von einem Wasserstoffatom verschieden ist und eine der Bedeutungen von Ru hat,

$Rb_3$ eine hydrophile Gruppe ist, die ausgewählt ist aus:

• COORe, wobei Re ein Zuckerrest, ein primäres, sekundäres oder tertiäres Hydroxyalkyl -($CH_2$)mOH, wobei m von 1 bis 4 variiert, ein Polyoxyalkylen, insbesondere Polyoxyethylen, mit 4 bis 10 Alkylenoxideinheiten, ein Rest ($CH_2$)t-NRf1Rf2 ist, wobei t eine ganze Zahl von 1 bis 5 ist, Rf1, Rf2, die gleich oder verschieden sind, ein Wasserstoffatom oder ein ($C_1$-$C_4$)-Alkylrest sind;
• einem Hydroxyl;
• einem primären, sekundären oder tertiären Hydroxyalkyl -($CH_2$)mOH, wobei m von 1 bis 4 variiert;
• einem primären, sekundären, tertiären Amin;
• einem quartären Ammonium;
• N-Formamid, N-Alkylformamid;
• N-Acetamid, N-Alkylacetamid;
• N-Pyrrolidonyl;
• CONRg1Rg2, wobei Rg1 und Rg2, die gleich oder verschieden sind, ein Wasserstoffatom, ein Zuckerrest, Polyoxyalkylen, insbesondere Polyoxyethylen, mit 4 bis 10 Alkylenoxideinheiten, ein zwitterionischer Rest, ein primäres, sekundäres oder tertiäres Hydroxyalkyl -($CH_2$)mOH sind, wobei m von 1 bis 4 variiert,
• COORh oder CONRkRl, wobei Rh ein ($C_1$-$C_5$)-Alkylrest, ein Alkylsulfonat ist oder eine der Bedeutungen von Re oder von Rg1 hat mit der Ausnahme eines Wasserstoffatoms, und Rk und Rl unabhängig von einander eine der Bedeutungen von Rh haben und außerdem einer von den beiden einem Wasserstoffatom entsprechen kann;

x1, x2, x3 den jeweiligen Prozentsätzen der Motive entsprechen mit

- x1 zwischen 20 und 90% einschließlich,
- x2 zwischen 10 und 80% einschließlich,
- x3 zwischen 0 und 60% einschließlich und
- $X_2/X_1 + X_3$ zwischen 0,25 und 2,5 einschließlich; und

wobei die mittlere Molmasse zwischen 500 und 100.000 einschließlich, in vorteilhafter Weise zwischen 500

und 50.000 einschließlich liegt.

**4.** Produkt nach Anspruch 3, **dadurch gekennzeichnet, dass:**

$Ra_1$, $Ra_2$ und $Ra_3$, die gleich oder verschieden sind, ein Wasserstoffatom oder ein Methylrest sind;
$Rb_1$ für $COO^-M^+$, wobei $M^+$ ein kationisches Gegenion ist, steht;
$Rb_2$ für CONRq1 Rq2 steht, worin Rq1 und Rq2 unabhängig voneinander für einen linearen oder verzweigten und/oder cyclischen Alkyl-, Alkinyl- oder Alkenylrest, welcher 3 bis 50 Kohlenstoffatome umfasst, stehen und außerdem eines der beiden einem Wasserstoffatom entsprechen kann;
$Rb_3$ für CONRkRl steht, worin Rk und Rl unabhängig voneinander für einen ($C_1$-$C_5$)-Alkylrest, ein Alkylsulfonat, einen Zuckerrest, ein primäres, sekundäres oder tertiäres Hydroxyalkyl -$(CH_2)$mOH, wobei m von 1 bis 4 variiert, ein Polyoxyalkylen, insbesondere Polyoxyethylen, mit 4 bis 10 Alkylenoxideinheiten, einen zwitterionischen Rest, einen Rest $(CH_2)$t-NRf1Rf2, wobei t eine ganze Zahl von 1 bis 5 ist, Rf1, Rf2, die gleich oder verschieden sind, ein Wasserstoffatom oder ein ($C_1$-$C_4$)-Alkylrest sind, stehen und außerdem einer der beiden von Rk und Rl einem Wasserstoffatom entsprechen kann.

**5.** Produkt nach einem der Ansprüche 1 bis 4, **dadurch gekennzeichnet, dass** der Träger ein fester Träger ist, der Träger vorteilhaft aus einem Chip, einer Kugel, einer Membran, einer Faser oder einem Nanoröhrchen ausgewählt ist oder der Träger ein Makromolekül oder lösliches Teilchen, ausgewählt aus Polymeren, Dendrimeren, Vesikeln oder Mizellen, ist.

**6.** Produkt nach einem der Ansprüche 1 bis 5, **dadurch gekennzeichnet, dass** die Anheftung des amphiphilen Moleküls an dem Träger durch eine hydrophobe Bindung, eine ionische Bindung, eine spezifische Bindung vom Rezeptor-Ligand-Typ zwischen mindestens einer funktionellen Gruppe des amphiphilen Moleküls und mindestens einer funktionellen Gruppe auf der Oberfläche des Trägers oder eine kovalente Bindung zwischen mindestens einer reaktiven funktionellen Gruppe des amphiphilen Moleküls und mindestens einer reaktiven funktionellen Gruppe auf der Oberfläche des Trägers vermittelt wird.

**7.** Produkt nach Anspruch 6, **dadurch gekennzeichnet, dass:**

a. das amphiphile Molekül außerdem mindestens eine funktionelle Gruppe umfasst, die die erste Hälfte eines Paars von Rezeptor-Ligand-Molekülen bildet;
b. der Träger außerdem mindestens eine an seiner Oberfläche angeheftete funktionelle Gruppe, die die zweite Hälfte des Paars von Rezeptor-Ligand-Molekülen bildet, umfasst und
c. die Anheftung des amphiphilen Moleküls an dem Träger durch eine spezifische Bindung vom Rezeptor-Ligand-Typ zwischen der/den funktionellen Gruppe(n) des amphiphilen Moleküls und der/den an dem Träger angehefteten funktionellen Gruppe(n), vermittelt wird,

wobei das Rezeptor-Ligand-Paar (funktionelle Gruppe des amphiphilen Moleküls/eine an dem Träger angeheftete funktionelle Gruppe) ausgewählt ist unter den Paaren (Biotin/Avidin), (Glutathion/Glutathion-S-transferase, Proteine, welche Glutathion binden, oder Fusionsproteine, welche die Glutathion-S-transferase umfassen), (Calmodulin/ATPase, Proteinkinase, Phosphodiesterase oder Neurotransmitter), (L-Arginin oder p-Aminobenzamidin/Serinprotease), (L-Lysin/Plasminogen (und -aktivator) oder rRNA), (AMP, ADP oder ATP/Enzym mit Cofaktoren), (Lectin/Glucanstrukturen umfassendes Protein, Glucolipid oder Polysaccharid), (Heparin/Wachstums- und Gerinnungsfaktor, Steroidrezeptor, Endonuklease, Lipoprotein oder Lipase) oder (Cibacron blue®/Enzyme mit NAD-oder NADP-Cofaktoren, Albumin, Gerinnungsfaktor oder Interferon), (Antigen/Antikörper), (Hapten/Antikörper), (Antikörper/Antigen), (Antikörper/Hapten), (Nitrilotriessigsäure (NTA)/Übergangsmetall), (EDTA/Übergangsmetall), (Phenylboronsäure (APB)/Salicylhydroxam(in)säure (ASH)) oder (Oligonukleotid/komplementäres Oligonukleotid) oder den entsprechenden umgekehrten Paaren.

**8.** Produkt nach Anspruch 7, **dadurch gekennzeichnet, dass** das amphiphile Molekül ein Polymer der Formel (I) oder (II) ist, welches außerdem einen Prozentsatz zwischen 0 und 4% eines Monomers der Formel

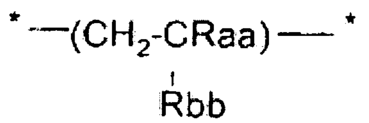

(III)

umfasst, in welcher:

Raa ein Wasserstoffatom oder ein Methylrest ist;
Rbb für eine Gruppe COORcc oder -COSRcc oder -CORcc oder CONRccRdd steht, worin

Rcc für die funktionelle Gruppe des amphiphilen Moleküls, welche die erste Hälfte des Rezeptor-Ligand-Paares bildet, steht und
Rdd für einen ($C_1$-$C_5$)-Alkylrest, ein Alkylsulfonat, ein Wasserstoffatom,

einen Zuckerrest, ein primäres, sekundäres oder tertiäres Hydroxyalkyl - ($CH_2$)mOH, worin m von 1 bis 4 variiert, ein Polyoxyalkylen, insbesondere Polyoxyethylen, mit 4 bis 10 Alkylenoxideinheiten, einen zwitterionischen Rest, einen Rest ($CH_2$)t-NRf1Rf2 steht, wobei t eine ganze Zahl von 1 bis 5 ist, Rf1, Rf2, die gleich oder verschieden sind, ein Wasserstoffatom oder ein ($C_1$-$C_4$)-Alkylrest sind.

**9.** Produkt nach Anspruch 6, **dadurch gekennzeichnet, dass:**

a. das amphiphile Molekül außerdem mindestens eine funktionelle Gruppe umfasst, die die erste Hälfte eines reaktiven chemischen Paares bildet,
b. der Träger außerdem mindestens eine an seiner Oberfläche angeheftete funktionelle Gruppe umfasst, welche die zweite Hälfte des reaktiven chemischen Paares bildet, und
c. die Anheftung des amphiphilen Moleküls an dem Träger bewirkt wird durch chemische Reaktion zwischen der/den funktionellen Gruppe(n) des amphiphilen Moleküls und der/den an dem Träger angehefteten funktionellen Gruppe(n).

**10.** Verfahren zur Herstellung eines Produkts nach einem der Ansprüche 1 bis 9, welches umfasst:

a) das Bereitstellen von mindestens einem Membranprotein, einem Träger und einem amphiphilen Molekül, wie in einem der Ansprüche 1 bis 4 und 7 bis 9 definiert,
b) die Bildung eines Komplexes zwischen dem amphiphilen Molekül und dem Membranprotein und
c) die Anheftung des amphiphilen Moleküls des Komplexes an dem Träger durch eine hydrophobe Bindung, eine ionische Bindung, eine spezifische Bindung vom Rezeptor-Ligand-Typ zwischen mindestens einer funktionellen Gruppe des amphiphilen Moleküls und mindestens einer funktionellen Gruppe auf der Oberfläche des Trägers oder eine kovalente Bindung zwischen mindestens einer reaktiven funktionellen Gruppe des amphiphilen Moleküls und mindestens einer reaktiven funktionellen Gruppe auf der Oberfläche des Trägers.

**11.** Verwendung eines Produkts nach einem der Ansprüche 1 bis 9, um das Vorhandensein oder Fehlen eines Liganden des mindestens einen Membranproteins in einer biologischen Probe nachzuweisen, wobei das mindestens eine Membranprotein in vorteilhafter Weise ein Membranantigen eines pathogenen Agens ist, wobei die biologische Probe eine Serumprobe ist und wobei der nachzuweisende Ligand ein gegen das Antigen gerichteter Antikörper ist.

**12.** Verwendung eines Produkts nach einem der Ansprüche 1 bis 9, um eine Bank von Verbindungen auf der Suche nach Liganden des mindestens einen Membranproteins zu screenen.

**13.** *In vitro*-Verwendung eines Produkts nach einem der Ansprüche 1 bis 9, in welchem das mindestens eine Protein ein Enzym ist, für die Umwandlung des Substrats des Enzyms unter kontrollierten Bedingungen.

**14.** Kit für das Ausführen des Verfahrens nach Anspruch 10, welcher einen Träger und ein amphiphiles Molekül umfasst, **dadurch gekennzeichnet, dass** die Bindung zwischen dem amphiphilen Molekül und dem Träger erfolgt durch eine hydrophobe Bindung, eine ionische Bindung, eine spezifische Bindung vom Rezeptor-Ligand-Typ zwischen mindestens einer funktionellen Gruppe des amphiphilen Moleküls und mindestens einer funktionellen Gruppe auf der Oberfläche des Trägers oder eine kovalente Bindung zwischen mindestens einer reaktiven funktionellen Gruppe

des amphiphilen Moleküls und mindestens einer reaktiven funktionellen Gruppe auf der Oberfläche des Trägers.

**15.** Kit nach Anspruch 14, **dadurch gekennzeichnet, dass:**

a. das amphiphile Molekül außerdem mindestens eine funktionelle Gruppe umfasst, welche die erste Hälfte eines Paares von Rezeptor-Ligand-Molekülen bildet,
b. der Träger außerdem mindestens eine an seiner Oberfläche angeheftete funktionelle Gruppe umfasst, welche die zweite Hälfte des Paares von Rezeptor-Ligand-Molekülen bildet, und
c. die Anheftung des amphiphilen Moleküls an dem Träger vermittelt wird durch spezifische Bindung vom Rezeptor-Ligand-Typ zwischen der/den funktionellen Gruppe(n) des amphiphilen Moleküls und der/den an dem Träger angehefteten funktionellen Gruppe(n),

wobei das Rezeptor-Ligand-Paar (funktionelle Gruppe des amphiphilen vinylischen Polymers/an dem Träger angeheftete funktionelle Gruppe) ausgewählt ist unter den Paaren (Biotin/Avidin), (Glutathion/Glutathion-S-transferase, Proteine, welche Glutathion binden, oder Fusionsproteine, welche die Glutathion-S-transferase umfassen), (Calmodulin/ATPase, Proteinkinase, Phosphodiesterase oder Neurotransmitter), (L-Arginin oder p-Aminobenzamidin/Serinprotease), (L-Lysin/Plasminogen (und -aktivator) oder rRNA), (AMP, ADP oder ATP/Enzym mit Cofaktoren), (Lectin/ Glucanstrukturen umfassendes Protein, Glucolipid oder Polysaccharid), (Heparin/Wachstums- und Gerinnungsfaktor, Steroidrezeptor, Endonuklease, Lipoprotein oder Lipase) oder (Cibacron blue®/Enzyme mit NAD-oder NADP-Cofaktoren, Albumin, Gerinnungsfaktor oder Interferon) oder den entsprechenden umgekehrten Paaren, (Antigen/Antikörper), (Hapten/Antikörper), (Antikörper/Antigen), (Antikörper/Hapten), (Nitrilotriessigsäure (NTA)/Übergangsmetall), (EDTA/Übergangsmetall), (Phenylboronsäure (APB)/Salicylhydroxam(in)säure (ASH)) oder (Oligonukleotid/komplementäres Oligonukleotid).

**16.** Kit nach Anspruch 15, **dadurch gekennzeichnet, dass** das amphiphile Molekül ein Polymer der Formel (I) oder (II) ist, welches außerdem einen Prozentsatz zwischen 0 und 4% einschließlich an einem Monomer der Formel

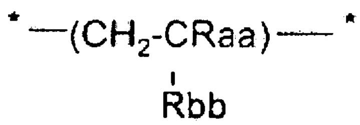

(III)

umfasst, in welcher Raa und Rbb wie in Anspruch 8 definiert sind.

**17.** Kit nach Anspruch 14, **dadurch gekennzeichnet, dass:**

a. das amphiphile Molekül außerdem mindestens eine funktionelle Gruppe umfasst, welche die erste Hälfte eines reaktiven chemischen Paares bildet,
b. der Träger außerdem mindestens eine an seiner Oberfläche angeheftete funktionelle Gruppe umfasst, welche die zweite Hälfte des reaktiven chemischen Paares bildet, und
c. die Anheftung des amphiphilen Moleküls an dem Träger bewirkt wird durch chemische Reaktion zwischen der/den funktionellen Gruppe(n) des amphiphilen Moleküls und der/den an dem Träger angehefteten funktionellen Gruppe(n).

**18.** Verwendung eines amphiphilen Moleküls, wie in einem der Ansprüche 1 bis 4 und 7 bis 9 definiert, um ein Membranprotein zu komplexieren und um dieses an einem Träger anzuheften.

**19.** Amphiphiles Molekül, wie in einem der Ansprüche 1 bis 4 definiert, umfassend außerdem eine funktionelle Gruppe, welche die erste Hälfte eines Paars von Rezeptor-Ligand-Molekülen bildet, wobei die funktionelle Gruppe ausgewählt ist aus Biotin, Avidin, Glutathion, der Glutathion-S-transferase, Calmodulin, einer ATPase, einer Proteinkinase, einer Phosphodiesterase, einem Neurotransmitter, L-Arginin, p-Aminobenzamidin, einer Serinprotease, L-Lysin, Plasminogen (und -aktivator), einer rRNA, AMP, ADP, ATP, einem Enzym mit Cofaktoren, einem Lectin, einem Glucanstrukturen umfassenden Protein, einem Glucolipid, einem Polysaccharid, Heparin, einem Wachstums- und Gerinnungsfaktor, einem Steroidrezeptor, einer Endonuklease, einem Lipoprotein, einer Lipase, Cibacron blue®, einem Enzym mit NAD- oder NADP-Cofaktoren, Albumin, einem Gerinnungsfaktor, einem Interferon, einem Antigen, einem Hapten, einem Antikörper, Nitrilotriessigsäure (NTA), EDTA oder einem Oligonukleotid.

**20.** Amphiphiles Molekül nach Anspruch 19, **dadurch gekennzeichnet, dass** das amphiphile Molekül ein Polymer der Formel (I) oder (II) ist, welches außerdem einen Prozentsatz zwischen 0 und 4% einschließlich an einem Monomer der Formel

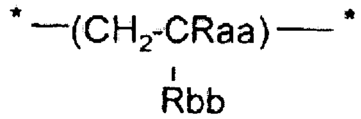 (III)

umfasst, in welcher Raa und Rbb wie in Anspruch 8 definiert sind.

**21.** Amphiphiles Molekül wie in einem der Ansprüche 1 bis 4 definiert, welches außerdem eine funktionelle Gruppe umfasst, welche die erste Hälfte eines reaktiven chemischen Paares bildet, ausgewählt aus Cyclopentadienylgruppen, die auf spontane Weise gemäß einem Diels-Alder-Mechanismus mit an der Oberfläche des festen Trägers angehefteten Chinonderivaten reagieren können, Alkinylgruppen (Dreifachbindung), die mit an der Oberfläche des festen Trägers angehefteten Nitridgruppen reagieren können, oder Alkoxylamin- oder Carbonylgruppen, die sich auf sehr selektive Weise an eine an der Oberfläche des festen Trägers angeheftete Carbonyl- oder Alkoxylaminfunktion ankondensieren können.

**22.** Verfahren zur Analyse von in einer Probe vorhandenen Membranproteinen, welches die Herstellung eines Produkts nach einem der Ansprüche 1 bis 9 umfasst, wobei das Verfahren in vorteilhafter Weise die Schritte umfasst, die daraus bestehen:

a) die Membranproteine in Detergens zu solubilisieren;
b) die Membranproteine mit einem amphiphilen Molekül, wie in den Ansprüchen 1 bis 5 definiert, zu komplexieren und das Detergens zu entfernen.
c) die Membranproteine an einem Träger, wie in den Ansprüchen 1 und 6 bis 8 definiert, durch das Zwischenglied des amphiphilen Moleküls zu immobilisieren,
d) intensiv zu waschen und eine Protease hinzu zu geben, um Proteinfragmente der Membranproteine zu erzeugen, wobei die Transmembrandomäne weiterhin an das an dem Träger angeheftete amphiphile Molekül komplexiert bleibt,
e) den Träger, an welchem die weiterhin an das amphiphile Molekül komplexierte Transmembrandomäne angeheftet bleibt, zu entfernen und die Proteinfragmente durch Massenspektrometrie zu analysieren.

A

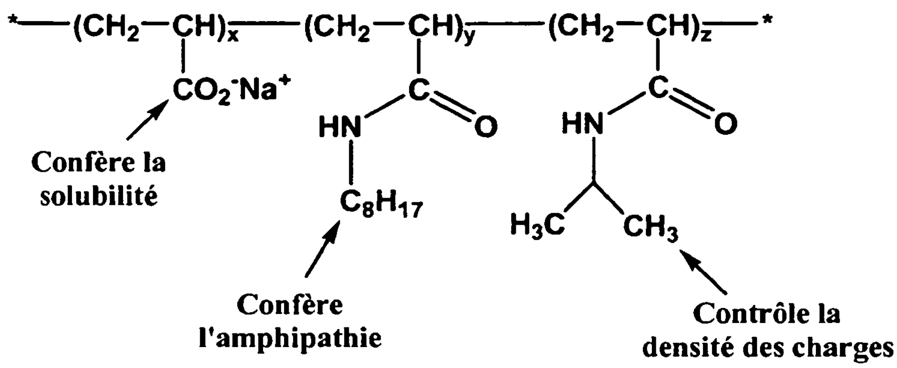
Confère la solubilité
Confère l'amphipathie
Contrôle la densité des charges

B

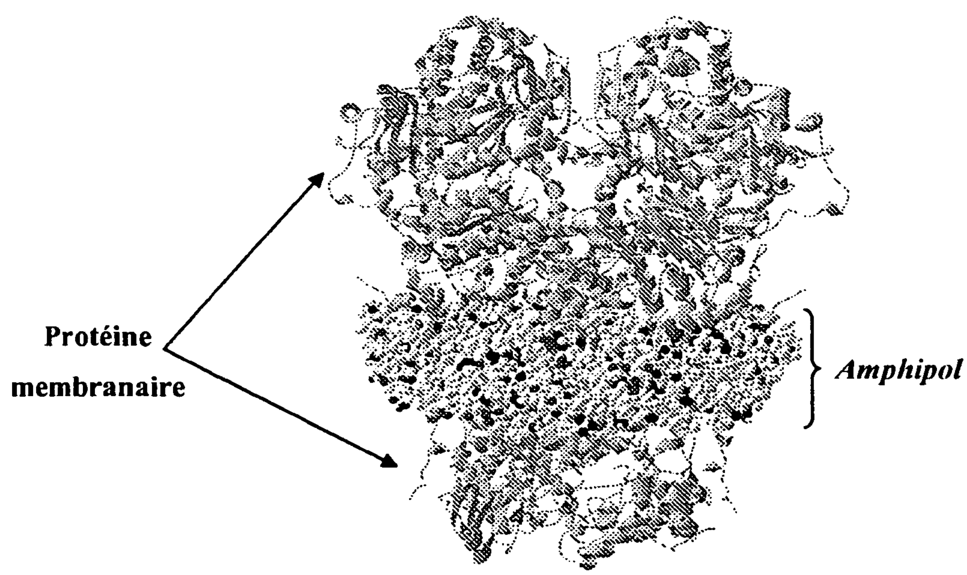
Protéine membranaire
Amphipol

Figure 1

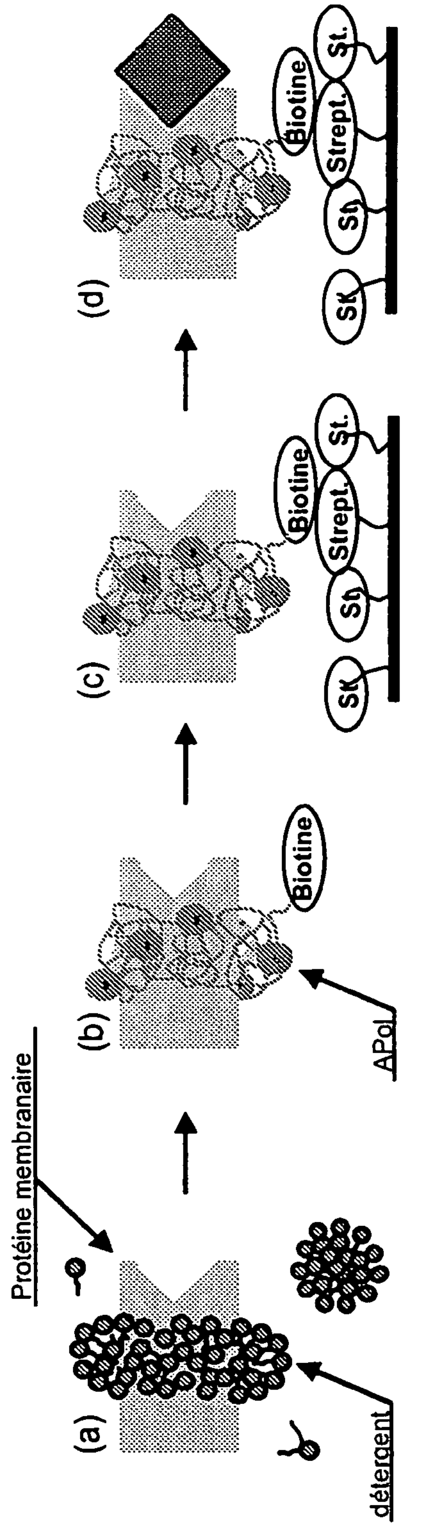
Protéine membranaire
détergent
APol
(a)
(b)
(c)
(d)
Biotine
Strept.
St.

**Figure 2**

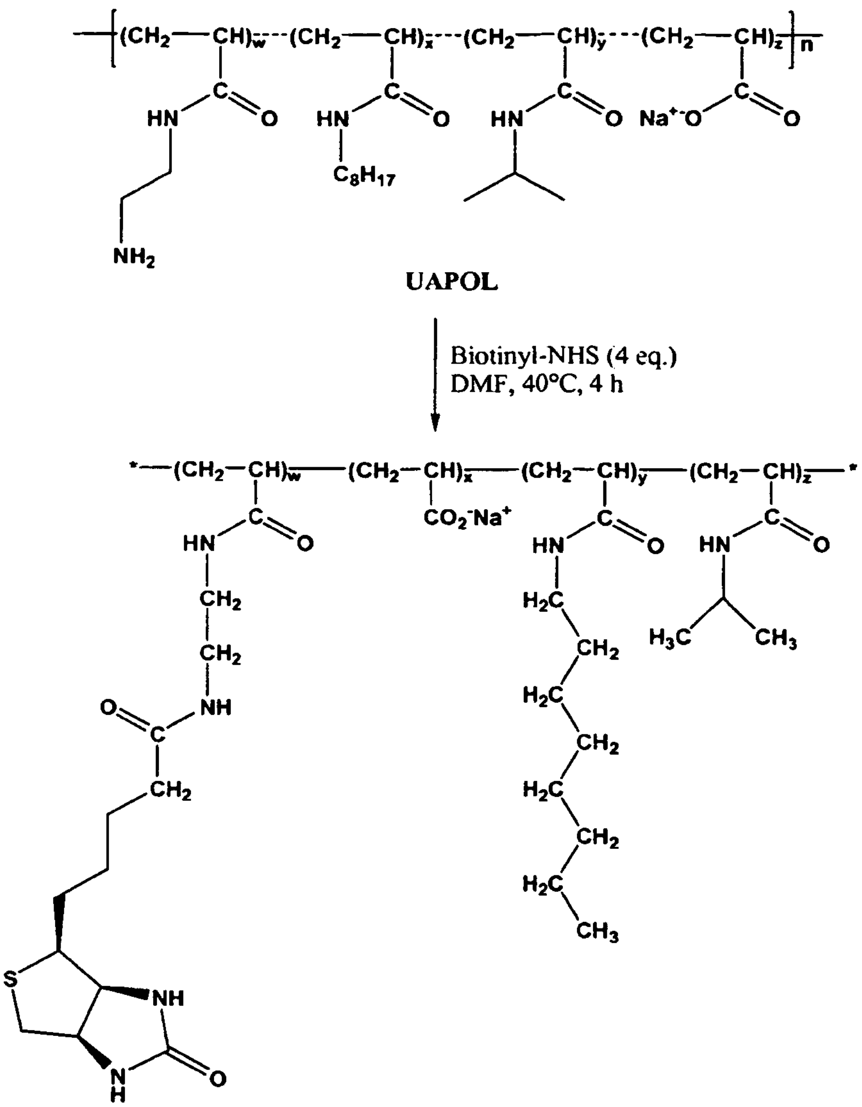


BAPOL

Biotinyl-NHS = 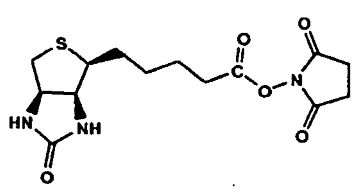

**Figure 3**

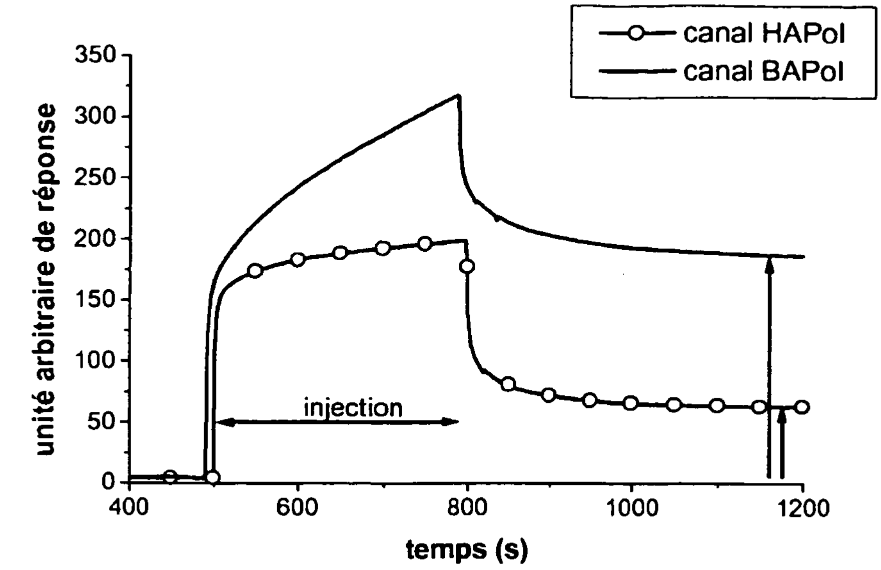


Figure 4

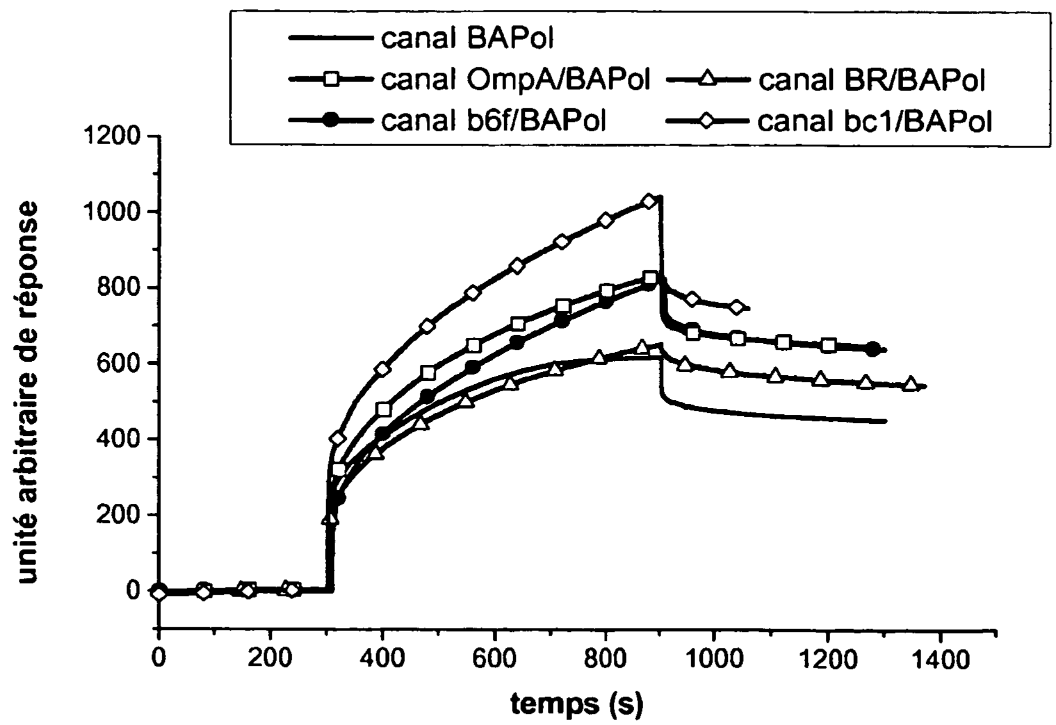


Figure 5

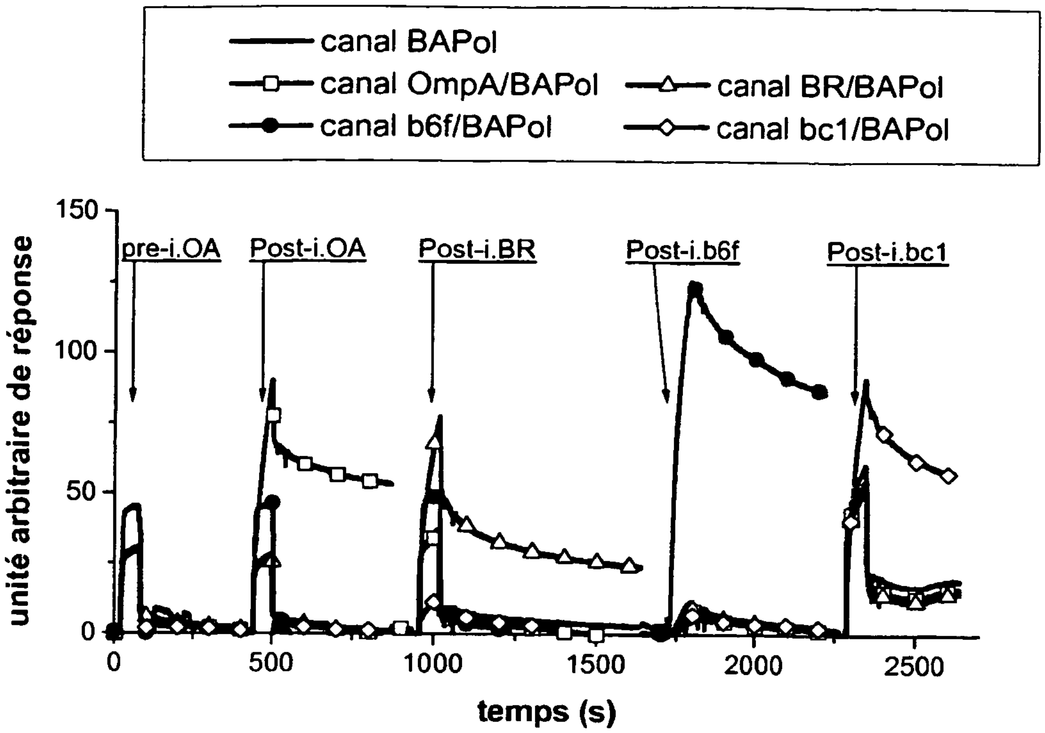


**Figure 6**

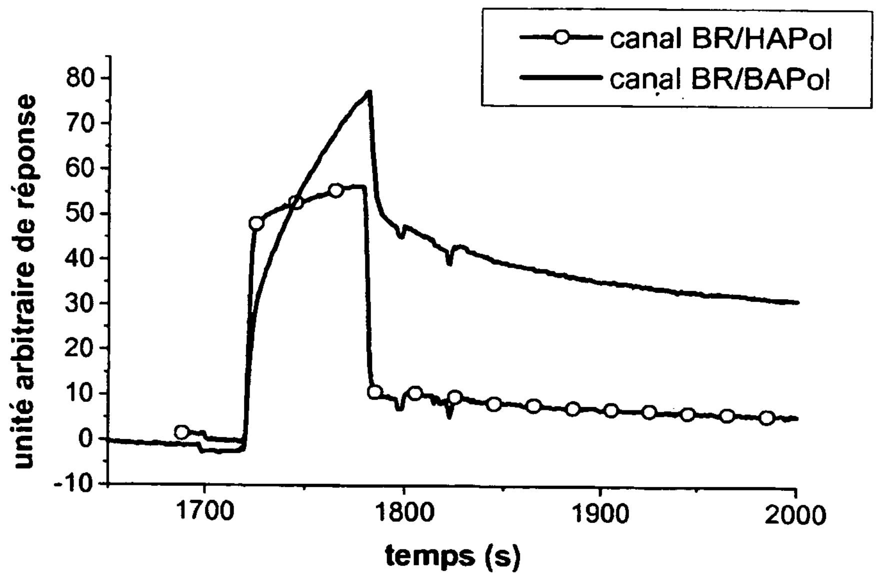


**Figure 7**

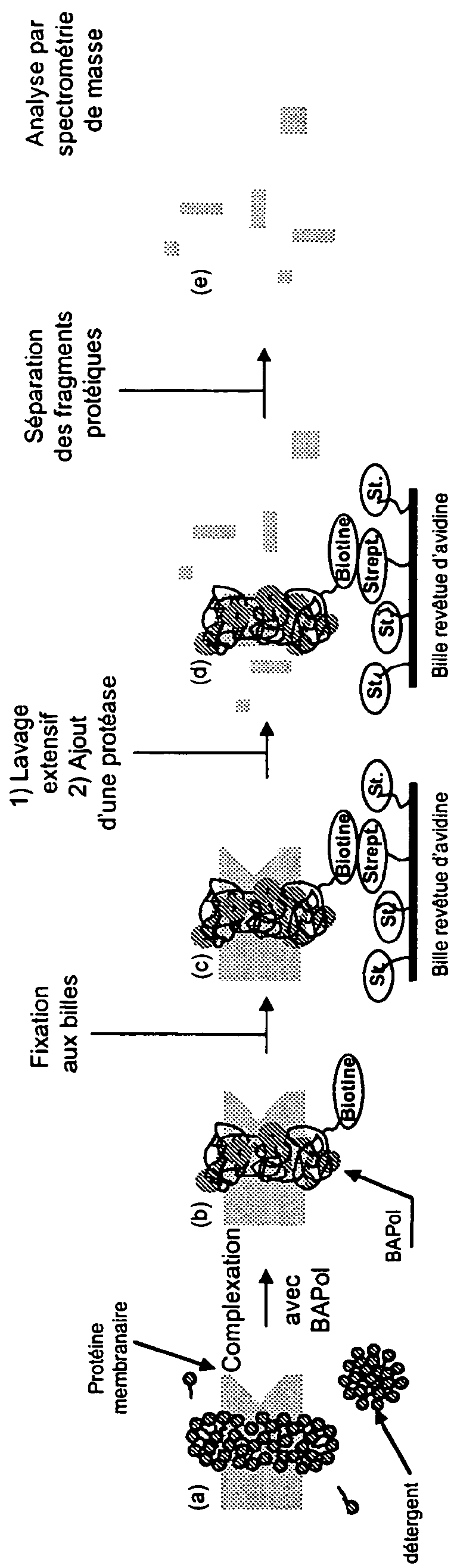
(a)
Protéine membranaire
détergent
Complexation avec BAPol
(b)
Biotine
BAPol
Fixation aux billes
(c)
St.
Strept.
Bille revêtue d'avidine
1) Lavage extensif
2) Ajout d'une protéase
(d)
Séparation des fragments protéiques
(e)
Analyse par spectrométrie de masse

Figure 8

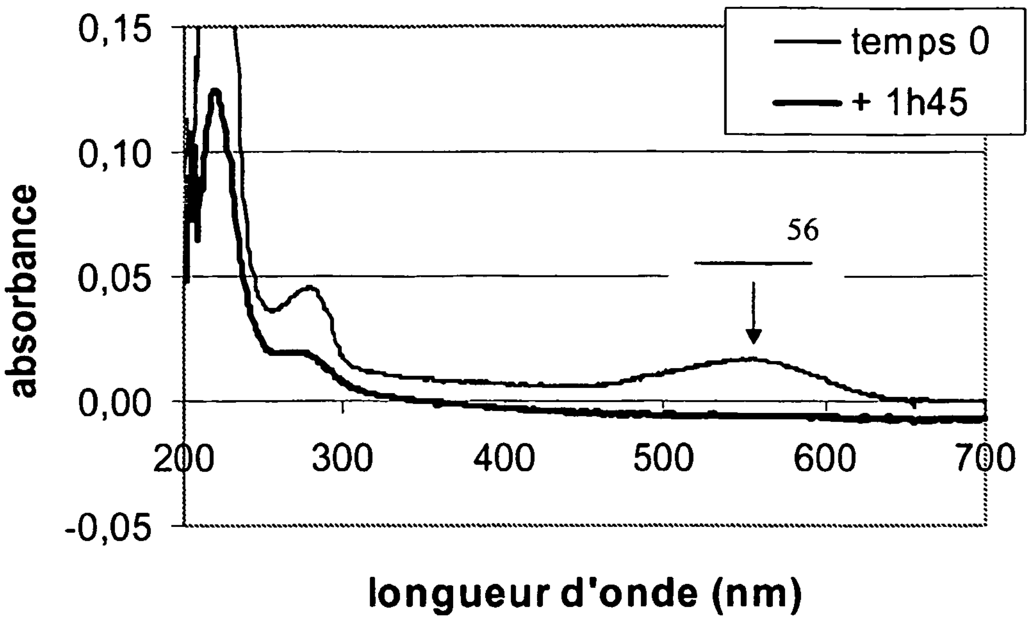
0,15
0,10
0,05
0,00
-0,05
absorbance
200
300
400
500
600
700
longueur d'onde (nm)
temps 0
+ 1h45
56

Figure 9

1)

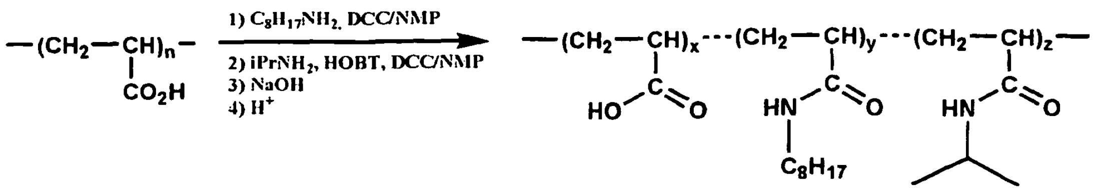


2)

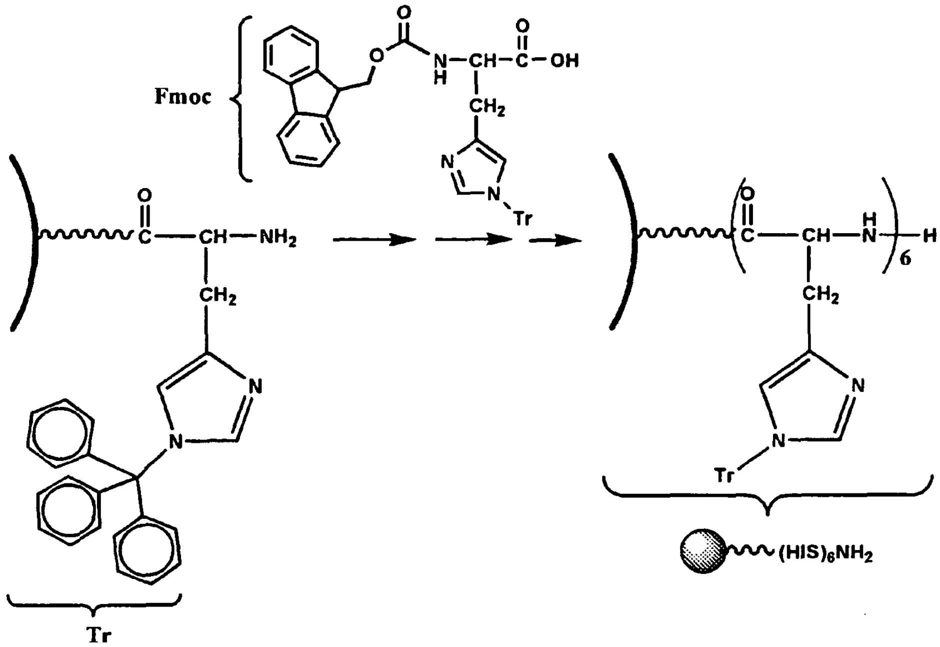

3)

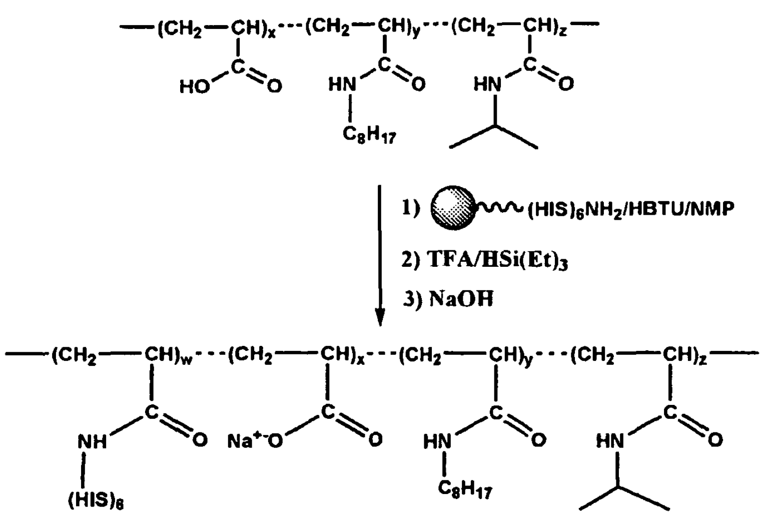


Figure 10

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0946875 A


### Littérature non-brevet citée dans la description


- **Giess F.** The protein-tethered lipid bilayer: a novel mimic of the biological membrane. *Biophys. J.,* 2004, vol. 87 (5), 3213-20
- **Pal P.** A novel immobilisation method for single protein spFRER studies. *Biophys. J.,* 2005, vol. 89 (2), L11-3
- **Hoffman T.L.** A biosensor assay for studying ligand-membrane receptor interactions : binding of antibodies and HIV-1 Env to chemokine receptors. *PNAS,* 2000, vol. 97, 11215-11220
- **Minic J.** Immobilization of native membrane-bound rhodopsin on biosensor surfaces. *Biochim. Biophys. Acta,* 2005, vol. 1724 (3), 324-32
- **Cooper M.A.** Optical biosensor in drug discovery. *Nat. rev. Drug Discov.,* 2000, vol. 1, 515-528
- **Tribet, C. ; Audebert, R. ; Popot, J.-L.** Amphipols: polymers that keep membrane proteins soluble in aqueous solutions. *Proc. Natl. Acad. Sci. USA,* 1996, vol. 93, 15047-15050
- **Popot, J.-L. ; Berry, E. A. ; Charvolin, D. ; Creuzenet, C. ; Ebel, C. ; Engelman, D. M. ; Flötenmeyer, M. ; Giusti, F. ; Gohon, Y. ; Hervé, P.** Amphipols: polymeric surfactants for membrane biology research. *Cell. Mol. Life Sci.,* 2003, vol. 60, 1559-1574
- **Zoonens, M. ; Catoire, L. J. ; Giusti, F. ; Popot, J.-L.** NMR study of a membrane protein in detergent-free aqueous solution. *Proc. Natl. Acad. Sci. USA,* vol. 102, 8893-8898
- **Picard, M. ; Dahmane, T. ; Garrigos, M. ; Gauron, C. ; Giusti, F. ; le Maire, M. ; Popot, J.-L. ; Champeil, P.** Protective and inhibitory effects of various types of amphipols on the Ca2+-ATPase from sarcoplasmic reticulum: a comparative study. *Biochemistry,* 2006, vol. 45, 1861-1869
- **Nagy, J. K. ; Kuhn Hoffmann, A. ; Keyes, M. H. ; Gray, D. N. ; Oxenoid, K. ; Sanders, C. R.** Use of amphipathic polymers to deliver a membrane protein to lipid bilayers. *FEBS Lett.,* 2001, vol. 501, 115-120
- **Q. Xu ; K. S. Lam.** Protein and Chemical Microarrays_Powerful Tools for Proteomics. *J. Biomed. Biotechnol.,* 2003, vol. 257, 2003
- **M. N. Yousaf ; M. Mrksich.** Diels-Aldert Reaction for the Selective Imobilization of Proteine to Electroactive Self-Assembled Monolayers. *J. Am. Chem. Soc.,* 1999, vol. 121, 4286
- **N. K. Devaraj ; G. P. Miller ; W. Ebina ; B. Kakaradov ; J. P. Collman ; E. T. Kool ; C. E. D. Chidsey.** Chemoselective Covalent Coupling of Oligonucleotide Probes to Self-Assembled Monolayers. *J. Am. Chem. Soc.,* 2005, vol. 127, 6800
- **I. Taniguchi ; A. M. Mayes ; E. W. L. Chan ; L. G. Griffith.** *A Chemoselective Approach to Grafting Biodegradable Polyesters, Macromolecules,* vol. 38, 216-219
- **Gohon, Y. ; Pavlov, G. ; Timmins, P. ; Tribet, C. ; Popot, J.-L. ; Ebel, C.** Partial specific volume and solvent interactions of amphipol A8-35. *Anal. Biochem.,* 2004, vol. 334, 318-334
- **Gohon, Y. ; Giusti, F. ; Prata, C. ; Charvolin, D. ; Timmins, P. ; Ebel, C. ; Tribet, C. ; Popot, J.-L.** Well-defined nanoparticles formed by hydrophobic assembly of a short and polydisperse random terpolymer, amphipol A8-35. *Langmuir,* 2006, vol. 22, 1281-1290
- *Chemical Abstract,* 15 Juin 1991, vol. 114, 224839
- *J. Chrom. B.,* 1997, vol. 703, 53-62